# EUROPEAN PATENT APPLICATION

(11) **EP 2 719 706 A1**
(43) Date of publication of application: **16.04.2014**
(21) Application number: 12192465.8
(22) Date of filing: 13.11.2012
(51) Int. Cl.: C07K 14/47, A61K 47/48

(54) **Bispecific HER2 ligands for cancer therapy**

(30) Priority: 15.10.2012 EP 12188598; 07.11.2012 EP 12191673
(71) Applicant: Universität Zürich, 8006 Zürich (CH)
(72) Inventor: Tamaskovic, Ratislav, 4310 Rheinfelden (CH); Jost, Christian, 8050 Zürich (CH); Schwill, Martin, 8006 Zürich (CH); Plückthun, Andreas, 8006 Zürich (CH)
(74) Representative: Junghans, Claas

(57) **Abstract**

The invention relates to a bispecific HER2-targeting agent comprising
a. a first polypeptide ligand that binds to HER2 extracellular domain 1,
b. a second polypeptide ligand that binds to HER2 extracellular domain 4 and
c. a linker covalently attaching said first polypeptide ligand to said second polypeptide ligand.

## Description

The present invention relates to bispecific targeting agents, particularly to antibodies, antibody fragments or other polypeptide ligands targeting HER2, and their use in cancer therapy.

### Background:

The members of the HER family of receptor tyrosine kinases are important mediators of cell growth, differentiation, migration and survival. The receptor family includes four distinct members including epidermal growth factor receptor (EGFR, ErbB1, or HER1), HER2 (ErbB2 or p185<neu>), HER3 (ErbB3) and HER4 (ErbB4). The members of the EGFR family are closely related single-chain modular glycoproteins with an extracellular ligand binding region, a single transmembrane domain and an intracellular tyrosine kinase, followed by specific phosphorylation sites which are important for the docking of downstream signaling proteins.

The extracellular regions of the HER receptor family contain two homologous ligand binding domains (domains 1 and 3) and two cysteine-rich domains (domains 2 and 4), which are important for receptor dimerization. In the absence of a ligand, HER receptors normally exist as inactive monomers, known as the "tethered" structure, which is characterized by close interaction of domain 2 and 4. Ligand binding to the extracellular domain initiates a conformational rearrangement, exposing the dimerization domains 2 and 4. Therefore, binding of growth factors to HER receptors induces conformational changes that allow receptor dimerization. After extracellular receptor dimerization, transmembrane helices switch to an active conformation that enables the intracellular kinase domains to trans-auto-phosphorylate each other. This phosphorylation event allows the recruitment of specific downstream signaling proteins.

Epidermal Growth factor receptor 1, (EGFR), has been causally implicated in human malignancy. In particular, increased expression of EGFR has been observed in breast, bladder, lung, head, neck and stomach cancer as well as glioblastomas.

Human epidermal growth factor receptor 2 (HER2, also known as ErbB2 or Neu; UniProtKB/Swiss-Prot No. P04626) consists of 1233 amino acids and is structurally similar to EGFR, with an extracellular domain consisting of four subdomains 1-4, a transmembrane domain, a juxtamembrane domain, an intracellular cytoplasmic tyrosine kinase and a regulatory C-terminal region. The structure of HER2's extracellular region is different in important aspects from the other EGF receptors, however. In the other EGF receptors, in a non-activated state, domain 2 binds to domain 4. Upon binding to domains 1 and 3, the activating growth factor (ligand) selects and stabilizes a conformation that allows a dimerization arm to extend from domain 2 to interact with an ErbB dimer partner. HER2, on the other hand, has a fixed conformation that resembles the ligand-activated state of the other receptor members: the domain 2-4 interaction is absent and the dimerization loop in domain 2 is continuously exposed. HER2 is activated via formation of heterodimeric complexes with other ErbB family members and thereby indirectly regulated by EGFR and HER3 ligands. HER2 is the preferred heterodimerization partner of the three other ErbB receptors, enhancing the affinity of the other ErbB receptors for their ligands by slowing down the rate of ligand-receptor complex dissociation, whereby HER2 enhances and prolongs signaling.

An excess of HER2 on the cell surface causes transformation of epithelial cells from multiple tissues. Amplification of the human homolog of the *neu* gene (also known as HER2) is observed in breast and ovarian cancers and correlates with a poor prognosis (US 4,968,603). Overexpression of HER2 has also been observed in other carcinomas including carcinomas of the stomach, endometrium, salivary gland, lung, kidney, colon, thyroid, pancreas and bladder.

### Antibodies targeting HER2

Drebin and colleagues have raised antibodies against the rat *neu* gene product, p185<neu>; see US6,733,752 (B1), incorporated herein by reference.

Hudziak et al., Mol. Cell. Biol. 9(3):1165-1172 (1989) describe the generation of a panel of HER2 antibodies which were characterized using the human breast tumor cell line SkBr-3. Using a cell proliferation assay, maximum inhibition was obtained with an antibody called 4D5. The antibody 4D5 was further found to sensitize HER2-overexpressing breast tumor cell lines to the cytotoxic effects of TNF-[alpha]; see also US 5,677,171, incorporated herein by reference. A recombinant humanized version of the murine HER2 antibody 4D5 (huMAb4D5-8, rhuMAb HER2, trastuzumab or HERCEPTIN; US 5,821,337) is clinically active in patients with HER2-overexpressing metastatic breast cancers that have received extensive prior anti-cancer therapy. Herceptin is approved in combination with chemotherapy for use in patients with HER2-positive metastatic stomach (gastric) cancer.

Herceptin is widely used for the treatment of patients with early as well as metastatic breast cancer whose tumors overexpress HER2 protein and/or have HER2 gene amplification. The treatment of breast cancer patients with Herceptin/trastuzumab is, for example, recommended and now routine for patients having HER2-positive disease; see US 2002/0064785, US 2003/0170234A1, US2003/0134344 and US 2003/0147884, all of which are incorporated herein by reference. The prior art thus focuses on the eligibility of breast cancer patients for trastuzumab/Herceptin therapy based on a high HER2 protein expression level (e.g. defined as HER2(3+) by immunohistochemistry (IHC)). HER2-positive disease in breast cancer is defined to be present if a high HER2 (protein) expression level is detected by immunohistochemical methods (e.g. HER2 (+++) or as HER2 gene amplification (e.g. a HER2 gene copy number higher than 4 copies of the HER2 gene per tumor cell) or both, found in samples obtained from the patients such as breast tissue biopsies or breast tissue resections or in tissue derived from metastatic sites. One frequently applied method for detecting HER2 overexpression and amplification at the gene level is fluorescence in situ hybridization (FISH), which is also described in US2003/0152987, Cohen et al.

Pertuzumab, a humanized antibody, is the first of a new class of agents known as HER dimerization inhibitors (HDIs). Pertuzumab binds to HER2 at its dimerization domain, thereby inhibiting its ability to form active heterodimer receptor complexes and probably also HER2 homodimerization, thus blocking the downstream signal cascade that ultimately results in cell growth and division. Pertuzumab is directed against the extracellular domain 2 of HER2. In contrast to trastuzumab, which acts by binding to domain 4 of HER2, pertuzumab is a HER dimerization inhibitor which inhibits dimerization of HER2 with HER3 and the other members of the EGFR receptor family, most pronounced in the presence of the respective activating ligands. By blocking complex formation, pertuzumab prevents the growth-stimulatory effects and cell survival signals activated by ligands of HER1, HER3 and HER4. Pertuzumab has been approved by the FDA under the name Perjeta for treatment in combination with trastuzumab and docetaxel for patients with HER2-positive metastatic breast cancer, who have not received prior anti-HER2 therapy or chemotherapy for metastatic disease. Pertuzumab is a fully humanized recombinant monoclonal antibody based on the human IgG1([kappa]) framework sequences. Patent publications concerning pertuzumab and selection of patients for therapy therewith include: US20060073143 (A1); US2003/0086924; US2004/0013667A1, and US2004/0106161, all of which are incorporated herein by reference.

For trastuzumab, while known to show clinical benefits in terms of e.g. prolonged survival in connection with chemotherapy compared to chemotherapy alone, a majority of HER2 positive breast cancer patients were nevertheless found to be non-responders (45% overall response rate for Herceptin + chemotherapy vs. 29% for chemotherapy alone.

Thus, while monoclonal antibody therapy directed against HER2 has been shown to provide improved treatment in e.g. metastatic breast cancers that overexpress HER2, there is still considerable room for improvement.

### Non-antibody scaffolds targeting HER2

Alternative targeting proteins have been proposed recently, which are more diverse in molecular structure than human immunoglobulin-derived antibody fragments and antibody-derived constructs and formats, and thus allow additional molecular formats by creating heterodimeric and multimeric assemblies, leading to new biological functions. A number of such targeting proteins have been described (reviewed in (Binz et al., Nat. Biotech 2005, Vol 23:1257)). Non-limiting examples of such targeting proteins are camelid antibodies, protein scaffolds derived from protein A domains (termed "Affibodies", Affibody AB), tendamistat (an alpha-amylase inhibitor, a 74 amino acid beta-sheet protein from Streptomyces tendae), fibronectin, lipocalin ("Anticalins", Pieris), T-cell receptors, ankyrins (designed ankyrin repeat proteins termed "DARPins", Univ. Zurich and Molecular Partners; see US20120142611 (A1), incorporated by reference herein), A-domains of several receptors ("Avimers", Avidia) and PDZ domains, fibronectin domains (FN3) ("Adnectins", Adnexus), consensus fibronectin domains ("Centyrins", Centyrex/Johnson&Johnson) and Ubiquitin ("Affilins", SCIL Proteins) and knottins (Moore and Cochrane, Methods in Enzymology 503 (2012), 223-251 and references cited therein).

From these proteins, multimeric and multispecific assemblies can be constructed (Caravella and Lugovskoy, Current Opinions in Chemical Biology 2010, 14:520-528; Vanlandschoot et al. Antiviral Research 2011 92:389-407; Löfblom et al. 2011 Current Opinion in Biotechnology 2011 22:843-848, Boersma et al. 2011 Curr. Opin. Biotechnol. 22:849-857). It is also possible to fuse these and other peptidic domains to antibodies to create so-called Zybodies (Zyngenia Inc., Gaithersburg, MD).

All of these scaffolds, with different inherent properties, have in common that they can be directed to bind specific epitopes, by using selection technologies well known to practitioners in the field (Binz et al., Nat. Biotech 2005, 23:1257-1268).

For example, the different individual domains of HER2 can be individually expressed in insect cells, using a baculovirus expression system, as demonstrated for domain 1 and domain 4 (Frei et al., Nat Biotechnol. 2012 30:997-1001). Thereby, it is guaranteed that binders selected will be directed towards the domain of interest. The HER2 domains can then be biotinylated as previously described (Zahnd et al.. (2006). Selection and characterization of HER2 binding-designed ankyrin repeat proteins. J. Biol. Chem. 281), and thus be immobilized on streptavidin-coated magnetic beads or on microtiter plates coated with streptavidin or neutravidin (Steiner et al. (2008) J. Mol. Biol. 382, 1211-27); (Zahnd et al. (2007) J. Mol. Biol. 369, 1015-28.)). The HER2 domains so immobilized can then serve as targets for diverse protein libraries in either phage display or ribosome display format. A large variety of different antibody libraries has been published (Mondon P. et al., Human antibody libraries: a race to engineer and explore a larger diversity. Frontiers in Bioscience. 13:1117-29, 2008.) and the technology of selecting binding antibodies is well known to the practitioners of the field. Phage display is a suitable format for antibody fragments (Fab fragments, scFv fragments or single domain antibodies s) (Hoogenboom HR.Nature Biotechnology. 23(9):1105-16, 2005 Sep) and any other scaffold that contain disulfide bonds, but it can also be used for scaffolds not containing disulfide bonds (e.g., Steiner et al. (2008) J. Mol. Biol. 382, 1211-27)( Rentero et al. Chimia. 65(11):843-5, 2011., Skerra A. Current Opinion in Biotechnology. 18(4):295-304, 2007 Aug). Similarly, ribosome display can be used for antibody fragments (Hanes et al. (2000), Picomolar affinity antibodies from a fully synthetic naive library selected and evolved by ribosome display. Nat. Biotechnol. 18, 1287-1292) and for other scaffolds (Zahnd et al. (2007). Ribosome display: selecting and evolving proteins in vitro that specifically bind to a target. Nat. Methods 4, 269-279; Zahnd et al. (2007) J. Mol. Biol. 369, 1015-28.). A third powerful technology is yeast display (Pepper et al., Combinatorial Chemistry & High Throughput Screening. 11 (2):127-34, 2008 Feb.). In this case a library of the binding protein of interest is displayed on the surface of yeast, and the respective domain of HER2 is either directly labeled with a fluorescent dye or its his tag is detected with an anti-histag antibody, which is in turn detected with a secondary antibody. Such methods are well known to the practitioners in the field (Boder et al., Yeast surface display for directed evolution of protein expression, affinity, and stability, Methods in Enzymology. 328:430-44, 2000.).

Another possibility of engineering represents the connection of those binders to create bispecific or higher multivalent binding molecules. Such connection can be achieved genetically by fusions of two or more of these binding molecules or chemically by crosslinking separately expressed molecules, or by adding a dimerization domain include separate dependent claims for each or any combination thereof (see, e.g. Stefan et al. (2011) J. Mol. Biol. 413:826; Boersma et al. (2011) J. Biol. Chem. 286: 41273)).

A bispecific anti-HER2 camelidae antibody construct (Bispecific Nanobody) is shown in US20110059090 (A1), incorporated herein by reference. The document relates to a bispecific molecule that simultaneously targets HER2 at the extracellular domain 2, defined by competition with pertuzumab, and domain 4, defined by competition with trastuzumab. This molecule has been described to exhibit stronger anti-proliferative activity than trastuzumab (Herceptin) in a direct comparison in an in vitro cell culture model using the cell line SkBr3.

Due to the absence of any known HER2-specific ligand, current HER2 targeting strategies aim to block the dimerization of the receptor by binding to the interaction interface. Today's knowledge of HER2 receptor dimerization is mostly based on the crystal structure of the ligand-bound form of the EGFR homodimer, which is broadly accepted as the active mode of all EGF receptor family members (Garret et al. (2002) Cell 110, 763-773). The two EGFR show a back-to-back interaction. Extending these findings to HER2 and its possible interaction with other members of the EGFR family, one interaction interface is present on domain 2 of the extracellular part of HER2. Pertuzumab binds to domain 2 and is indeed known to block receptor interaction at this interface. Another known interaction is present on domain 4 of the extracellular part of HER2. This interaction interface is presumably blocked by trastuzumab. Since both antibodies, trastuzumab and pertuzumab, even when simultaneously applied, are not able to block all HER2 interactions to completeness, the interaction of the extracellular part and the kinase domain of HER2 are thought to be loosely linked, allowing some residual interactions even in the trastuzumab- and pertuzumab-blocked state, which is in accordance with crystal structure data (Chafen et al. (2010) Mol. Cel. Biol. vol 5432-43). The bispecific ligand mentioned above that binds both epitopes (pertuzumab and trastuzumab) simultaneously (US20110059090 A1) reduces the cell growth in a cell culture model by approx. 50%, in comparison to a reduction of about 40% effected by trastuzumab. This same effect, however, can also be achieved by treating with the mixture of trastuzumab and pertuzumab.

In view of the above mentioned state of the art, the objective of the present invention is to provide improved means and methods for targeting the HER2 protein for use in therapy of cancer. This objective is attained by the subject-matter of the independent claims.

### Summary of the invention

According to one aspect of the invention, a bispecific agent is provided, comprising
a. a first ligand that binds HER2 extracellular domain 1,
b. a second ligand that binds HER2 extracellular domain 4, and
c. a linker that connects said first ligand to said second ligand.

In some embodiments, the bispecific agent is a polypeptide.

In some embodiments, the bispecific agent is composed of a single sequence of amino acids. In some embodiments, the first ligand is connected to the second ligand covalently through a bridging moiety attached to amino acid side chains on the first and second ligands. In some embodiments, the first ligand is connected to the second ligand through a dimerization domain binding both the first ligand and the second ligand by non-covalent interactions.

According to an alternative to this aspect of the invention, a polypeptide is provided, comprising
a. a first binding site that binds HER2 extracellular domain 1,
b. a second binding site that binds HER2 extracellular domain 4, and
c. a linker that covalently links the first binding site and the second binding site.

The term "binding site" in the context of the present specification refers to the constituent parts, in particular the amino acid residues, of the first or second polypeptide ligand that in binding interact with particular constituent parts, for example a particular epitope, of the extracellular domain 1 or 4 of HER2.

According to another alternative of this aspect of the invention, a bispecific HER2-targeting agent is provided, comprising
a. a first polypeptide ligand that binds to HER2 extracellular domain 1 (Seq. ID 01),
b. a second polypeptide ligand that binds to HER2 extracellular domain 4 (Seq. ID 02) and
c. a linker covalently attaching the first polypeptide ligand to the second polypeptide ligand.

The term "bispecific" in the context of the present specification refers to the ability of the agent to specifically bind to two different epitopes of HER2.

"Binding" or "specifically binding" in the context of the present specification refers to the ability of the first (and respectively, second) polypeptide ligand to specifically and noncovalently attach to domain 1 (or, respectively, domain 4) of HER2 with a dissociation constant of equal or less than 10⁻⁷ M, 10⁻⁸ M or 10⁻⁹ M.

Domain 1 (SEQ ID 01) of HER2 (ErbB-2; Accession no. NP_004439.2) is the amino acid sequence

QVCT GTDMKLRLPA SPETHLDMLR HLYQGCQVVQ GNLELTYLPT NASLSFLQDI
QEVQGYVLIA HNQVRQVPLQ RLRIVRGTQL FEDNYALAVL DNGDPLNNTT
PVTGASPGGL RELQLRSLTE ILKGGVLIQR NPQLCYQDTI LWKDIFHKNN QLALTLIDTN
RSRACHPCSP MCKGSRCWGE SSEDCQSLTR TVA.

Domain 4 (SEQ ID02) of HER2 (ErbB-2; Accession no. NP_004439.2) is the amino acid sequence

VNCS QFLRGQECVE ECRVLQGLPR EYVNARHCLP CHPECQPQNG
SVTCFGPEADQCVACAHYKD PPFCVARCPS GVKPDLSYMP IWKFPDEEGA CQP

Accession numbers and Gene ID numbers refer to entries in the National Center for Biotechnology Information, Bethesda, Maryland, MD.

UniProt. No refer to entries in the UniProt Knowledgebase.

ATCC numbers refer to entries in the American Type Culture Collection.

PDB IDs refer to entries in the protein data bank.

In some embodiments, the first polypeptide ligand or the second polypeptide ligand is an antibody, antibody fragment, an antibody-like molecule or a protein A domains derived polypeptide.

In some embodiments, the antibody is an immunoglobulin consisting of two heavy chains and two light chains. In some embodiments, the antibody is a single domain antibody, consisting of an isolated variable domain from a heavy or light chain. In some embodiments, the antibody is a heavy-chain antibody consisting of only heavy chains such as antibodies found in camelids.

In some embodiments, the antibody fragment is a Fab fragment, i.e. the antigen-binding fragment of an antibody, or a single-chain variable fragment, i.e. a fusion protein of the variable region of heavy and the light chain of an antibody connected by a peptide linker.

An antibody-like molecule in the context of the present specification refers to a molecule showing a specific binding to another molecule or target similar to the specific binding of an antibody. In some embodiments, the antibody-like molecule is a repeat protein, such as a designed ankyrin repeat protein (Molecular Partners, Zürich), a polypeptide derived from armadillo repeat proteins, a polypeptide derived from leucine-rich repeat proteins or a polypeptide derived from tetratricopeptide repeat proteins.

In some embodiments, the antibody-like molecule may also be a polypeptide derived from protein A domains, a polypeptide derived from fibronectin domain FN3, a polypeptide derived from consensus fibronectin domains, a polypeptide derived from lipocalins, a polypeptide derived from Zinc fingers, a polypeptide derived from Src homology domain 2 (SH2), a polypeptide derived from Src homology domain 3 (SH3), a polypeptide derived from PDZ domains, a polypeptide derived from gamma-crystallin, a polypeptide derived from ubiquitin, a polypeptide derived from a cysteine knot polypeptide or a polypeptide derived from a knottin.

A protein A domains derived polypeptide refers to a molecule that is a derivative of protein A and is capable of specifically binding the Fc region and the Fab region of immunoglobulins.

An armadillo repeat protein refers to a polypeptide comprising at least one armadillo repeat, wherein a armadillo repeat is characterized by a pair of alpha helices that form a hairpin structure.

A humanized camelid antibody in the context of the present specification refers to an antibody consisting of only the heavy chain or the variable domain of the heavy chain (VHH domain) and whose amino acid sequence has been modified to increase their similarity to antibodies naturally produced in humans and, thus show a reduced immunogenicity when administered to a human being.

A general strategy to humanize camelid antibodies is shown in Vincke et al. "General strategy to humanize a camelid single-domain antibody and identification of a universal humanized nanobody scaffold", J Biol Chem. 2009 Jan 30;284(5):3273-84, and US2011165621A1, incorporated herein by reference.

In some embodiments, the first polypeptide ligand and/or the second polypeptide ligand is selected from
a. an immunoglobulin Fab fragment,
b. an immunoglobulin scFv fragment,
c. an immunoglobulin variable domain (domain antibody),
d. a humanized camelid antibody,
e. a polypeptide derived from protein A domains,
f. a polypeptide derived from fibronectin domain FN3,
g. a polypeptide derived from consensus fibronectin domains,
h. a polypeptide derived from lipocalins,
i. a polypeptide derived from armadillo repeat proteins,
j. a polypeptide derived from tetratricopeptide repeat proteins,
k. a polypeptide derived from leucine-rich repeat proteins,
l. a polypeptide derived from Zinc fingers,
m. a polypeptide derived from Src homology domain 2 (SH2),
n. a polypeptide derived from Src homology domain 3 (SH3),
o. a polypeptide derived from PDZ domains,
p. a polypeptide derived from gamma-crystallin,
q. a polypeptide derived from ubiquitin,
r. a polypeptide derived from a cysteine knot polypeptide,
s. a polypeptide derived from a knottin and
t. a peptide selected from a random peptide library to bind to domain 1 or domain 4 of HER2.

According to another aspect of the invention, a bispecific antibody is provided, which is selected from
a. a bispecific IgG comprising a first Fab fragment binding to domain 1 of HER2 and a second Fab fragment binding to domain 4 of HER2,
b. an IgG comprising a VH domain binding to domain 1 of HER2 and a VL domain binding to domain 4 of HER2,
c. an IgG comprising a VH domain binding to domain 4 of HER2 and a VL domain binding to domain 1 of HER2,
d. a construct comprising a first scFv fragment binding to domain 1 of HER2, a second scFv fragment binding to domain 4 of HER2 and a linker connecting said first scFv fragment and said second scFv fragment,
e. a diabody comprising a first binding site binding to domain 1 of HER2 and a second binding site binding to domain 4 of HER2,
f. an IgG targeting HER2 domain 4 connected to a polypeptide ligand selected from the list recited in the above embodiment of the invention targeting domain 1 of HER2, or to a peptide ligand of 5 to 35 amino acids selected from a peptide library to bind to domain 1 of HER2, wherein the polypeptide ligand or the peptide ligand is connected to
   i. the N-terminus of a heavy chain of the IgG,
   ii. the C-terminus of a heavy chain of the IgG,
   iii. the N-terminus of a light chain of the IgG or
   iv. the C-terminus of a light chain of the IgG,
g. an IgG targeting HER2 domain 1 connected a polypeptide ligand selected from the list recited in the above embodiment of the invention targeting domain 4 of HER2 or to a peptide ligand of 5 to 35 amino acids selected from a peptide library to bind to domain 1 of HER2, wherein the polypeptide ligand or the peptide ligand is connected to
   i. the N-terminus of a heavy chain of the IgG,
   ii. the C-terminus of a heavy chain of the IgG,
   iii. the N-terminus of a light chain of the IgG or
   iv. the C-terminus of a light chain of the IgG.

The term "diabody" in the context of the present specification refers to a bispecific antibody comprising the VH (variable heavy) domain of a first antibody linked to the VL (variable light) domain of a second antibody and the VL domain of the first antibody fused to the VH domain of the second antibody.

The term "VL domain" in the context of the present specification refers to the variable domain of the light chain of an antibody.

Likewise, the term "VH domain" in the context of the present specification refers to the variable domain of the heavy chain of an antibody.

In some embodiments, a bispecific IgG is provided, consisting exclusively of a VH domain binding to domain 1 of HER2 and a VL domain binding to domain 4 of HER2 or exclusively of a VH domain binding to domain 1 of HER2, a VL domain binding to domain 4 of HER2 and a linker.

In some embodiments, the bispecific HER2-targeting agent of the invention is a bispecific IgG, consisting exclusively of
- a VH domain binding to domain 4 of HER2 and a VL domain binding to domain 1 of HER2 and a linker connecting the two domains, or of
- a VH domain binding to domain 4 of HER2, a VL domain binding to domain 1 of HER2 and a linker connecting the two domains.

In some embodiments, the first polypeptide ligand and/or the second polypeptide ligand is an ankyrin repeat based polypeptide.

An ankyrin repeat based polypeptide in the context of the present specification refers to a polypeptide that comprises repetitive amino acid sequences, each repetitive sequence comprising two α-helices separated by loops.

In one embodiment, the antibody-like molecules are the Designed Ankyrin Repeat Proteins (DARPins) disclosed in US2012142611 (A1), which is incorporated herein by reference.

In some embodiments, the first polypeptide ligand comprises or is a sequence selected from the group composed of SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 30, SEQ ID 31, SEQ ID 32, SEQ ID 33, SEQ ID 34, SEQ ID 35, SEQ ID 36, SEQ ID 37, SEQ ID 38, SEQ ID 39, SEQ ID 40, SEQ ID 41, SEQ ID 42, SEQ ID 43, SEQ ID 44, SEQ ID 45, SEQ ID 46, SEQ ID 47, SEQ ID 48, SEQ ID 49 and SEQ ID 50. Such polypeptide, which comprises or is a sequence described in the preceding paragraph, is an ankyrin repeat based polypeptide that binds the extracellular domain 1 of HER2.

In some embodiments, the second polypeptide ligand comprises or is a sequence from the group composed of SEQ ID 25, SEQ ID 26, SEQ ID 27, SEQ ID 28 and SEQ ID 29.

Such polypeptide, which comprises or is a sequence described in the preceding paragraph, is an ankyrin repeat based polypeptide that binds the extracellular domain 4 of HER2.

Where reference is made herein to a polypeptide characterized by a particular sequence, such reference is meant to also encompass polypeptides having an identical function to the particular sequence, and showing a sequence identity of at least 70%, 80%, 90% or 95% to the certain sequence.

Identity in the context of the present invention is a single quantitative parameter representing the result of a sequence comparison position by position. Methods of sequence comparison are known in the art; the BLAST algorithm available publicly is an example.

In some embodiments, the first polypeptide ligand and the second polypeptide ligand are attached to each other by an oligopeptide linker, the first polypeptide, the second polypeptide ligand and the linker forming a continuous polypeptide chain.

One advantage of a bispecific HER2-targeting agent consisting of a continuous polypeptide chain is that such agent easily can be manufactured by recombinant biotechnology in a suitable host such as *E*. *coli,* yeast or mammal cells by expression of a single nucleotide sequence coding the continuous polypeptide chain.

In some embodiments, the first polypeptide ligand is located at the N-terminus of the continuous polypeptide chain, the second polypeptide ligand is located at the C-terminus of the continuous polypeptide chain, and the linker is located between the first and the second polypeptide ligand. Embodiments wherein the agent of the invention is constituted by one continuous polypeptide chain offers advantages of production of the agent in a single step by methods of recombinant biotechnology, facilitating reproducibility of composition of the agent.

In some embodiments, the first polypeptide ligand and the second polypeptide ligand are attached covalently to each other by a bridging moiety or a crosslinker.

In some embodiments, the crosslinker connects a functionality such as an amino function on the side chain of lysine or a thiol function on a side chain of cysteine or the N-terminal amino group in the first polypeptide ligand to an amino acid side chain functional group in the second polypeptide ligand.

In some embodiments, the crosslinker is selected from glutaraldehyde, succinimide, tris[2-maleimidoethyl]amine, 1,4-bismaleimidobutane, and 1,4 bismaleimidyl-2,3-dihydroxybutane.

In some embodiments, a bispecific HER2-targeting agent according to the above aspects or embodiments of the invention is provided, wherein
a) the first polypeptide ligand partially or fully interacts non-covalently with
   i. a first D1 (domain 1) epitope, wherein the first D1 epitope comprises the amino acid residues E87, N89, Y90, L132, R135, D143, 1145, W147, K148, L157, A158, L159, T160, L161 and I162 comprised within the amino acid sequence of HER2,
   ii. a second D1 epitope, wherein the second D1 epitope comprises the amino acid residues D88, A93, V94, I133, Q134, Q142, T144, L146, F151, H152, K153, N154, Q156 and D163 comprised within the amino acid sequence of HER2,
   iii. a third D1 epitope characterized by Seq. ID 55, or
   iv. a D1 epitope of domain 1 of HER2 (SEQ ID 01), wherein binding to the D1 epitope is competed by a polypeptide selected from SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 30, SEQ ID 31, SEQ ID 32, SEQ ID 33, SEQ ID 34, SEQ ID 35, SEQ ID 36, SEQ ID 37, SEQ ID 38, SEQ ID 39, SEQ ID 40, SEQ ID 41, SEQ ID 42, SEQ ID 43, SEQ ID 44, SEQ ID 45, SEQ ID 46, SEQ ID 47, SEQ ID 48, SEQ ID 49 and SEQ ID 50.
      and/or,
b) the second polypeptide ligand partially or fully interacts non-covalently with
   i. a first D4 (domain 4) epitope, wherein the first D4 epitope comprises the amino acid residues F512, E521, V524, L525, Q526, Y532, V533, N534, A535, R536, D549, G550, S551, V552, C554, F555 and V563 comprised within the amino acid sequence of HER2,
   ii. a second D4 epitope, wherein the second D4 epitope comprises the amino acid residues C522, R523, T553, C562 and A564 comprised within the amino acid sequence of HER2,
   iii. a third D4 epitope characterized by Seq. ID 56,
   iv. a forth D4 epitope characterized by Seq. ID 57, or
   v. a D4 epitope of domain 4 of HER2 (SEQ ID 02), wherein binding to the D4 epitope is competed by a polypeptide having a sequence selected from SEQ ID 25, SEQ ID 26, SEQ ID 27, SEQ ID 28 and SEQ ID 29.

Non-covalent interactions in the context of the present specification include, without being restricted to, electrostatic interaction, hydrophobic interactions and van-der-Waals-interactions.

In some embodiments, the non-covalently interaction mediates the binding of the polypeptide ligand with a dissociation constant of equal or less than 10⁻⁷ M, 10⁻⁸ M or 10⁻⁹ M.

The term "epitope" in the context of the present specification refers to the part of the extracellular domain 1 or 4 of HER2 that is bound by the first or second polypeptide.

A polypeptide ligand interacts partially with an epitope, when 20%, 30%, 40%, 50%, 60%, 70%, 80%, 90% or 95% of the indicated amino acid residues of the epitope were bound by the polypeptide ligand.

Likewise, a polypeptide ligand interacts fully with an epitope, when all amino acids of the epitope are bound by the polypeptide linker.

In some embodiments, a bispecific HER2-targeting agent according to the invention is provided, wherein
a) the first polypeptide ligand is an ankyrin repeat based polypeptide, and the second polypeptide ligand is an antibody, an antibody fragment, an antibody variable domain or a polypeptide ligand selected from the list under point a, b, c, d, e, f, g, h, i, j, k, I, m, n, o, p, q, r, s or t recited in the above embodiment, or
b) the first polypeptide ligand is an antibody, an antibody fragment, an antibody variable domain or a polypeptide ligand selected from the list under point a, b, c, d, e, f, g, h, i, j, k, I, m, n, o, p, q, r, s or t recited in the above embodiment, and the second polypeptide ligand is an ankyrin repeat based polypeptide.

In some embodiments, a bispecific HER2-targeting agent according to the invention is provided, wherein
a) the first polypeptide ligand is a polypeptide ligand selected from the list under point a, b, c, d, e, f, g, h, i, j, k, I, m, n, o, p, q, r, s or t recited in the above embodiment, and the second polypeptide ligand is an antibody, an antibody fragment or an antibody variable domain, or
b) the first polypeptide ligand is an antibody, an antibody fragment or an antibody variable domain, and the second polypeptide ligand is polypeptide ligand selected from the list under point a, b, c, d, e, f, g, h, i, j, k, l, m, n, o, p, q, r, s or t recited in the above embodiment.

In some embodiments, the linker has a length of equal or less than 65 A, 60 A, 55 A, 50 A, 45 Å, 40 Å, 35 A, 30 Å, 25 Å, 20 Å, 15 Å, 10 Å or 5 Å.

In some embodiments, a bispecific HER2-targeting agent according to the above aspects or embodiments is provided, wherein
a) the first polypeptide ligand contacts the HER2 extracellular domain 1 through a D1 binding site,
b) the second polypeptide ligand contacts the HER2 extracellular domain 4 through a D4 binding site, and
c) the linker is selected to allow a direct spatial separation, or in other words a maximal distance between the D1 binding site and the D4 binding site of less than 80 A, 75 A. 70 A, 65 A, 60 A, 55 A, 50 A, 45 A, 40 A, 35 A, 30 A, 25 A, 20 A, 15 A, 10 A or 5 A.

In some embodiments, the linker consists of 1, 2, 3, 4, 5, 6, 7, 8, 9, 10, 11, 12, 13, 14, 15, 16, 17, 18, 19, 20, 21, 22, 23, 24, or 25 amino acids.

In some embodiments, the linker is a polyglycine/serine linker.

The term "polyglycine/serine linker refers to a polypeptide linker that is composed of at least 50%, 60%, 70%, 80%, 90% or 100% of glycine and/or serine residues.

In some embodiments, the linker is characterized by an amino acid sequence (GGGGS)ₙ with n being 1, 2, 3, 4 or 5.

In some embodiments, the linker has the sequence SEQ ID 51, SEQ ID 52, SEQ ID 53 or SEQ ID 54.

In some embodiments, a bispecific HER2-targeting agent according to the invention is provided, which leads to a higher reduction of the viability of a cell selected from the group comprised of AU565 (also AU-565, ATCC number CRL-2351), BT474 (also BT-474, ATCC number HTB-20), HCC149 (ATCC number CRL-2326), HCC2218 (ATCC number CRL-2343), SkBr3 (ATCC number HTB-30) and/or ZR7530 (ATCC number CRL-1504) compared to trastuzumab.

Viability in the context of the present specification refers to the ability of a cell to maintain its homeostasis. The viability of a cell may be determined by spectroscopically measuring the concentration of a Formazan dye, wherein the formazan dye is formed during reduction of tetrazolium salts such as MTT (3-(4, 5-dimethyl-2-thiazolyl)-2, 5-diphenyl-2H-tetrazolium bromide) or XTT (2,3-bis-(2-methoxy-4-nitro-5-sulfophenyl)-2H-tetrazolium-5-carboxanilide) catalyzed by dehydrogenases or reductases of viable cells.

The ability of a bispecific agent of the invention to reduce the viability of the cancer cells described in the preceding paragraphs is useful in a method for treating diseases associated with the occurrence of such cell for example cancer.

A reduction of cell viability may be accompanied by an inhibition of proliferation, a reduction of cell count, a reduced protein content of the cells, a reduced metabolic activity of the cells or an induction of apotosis of the cells, which may be observed.

In some embodiments, a bispecific HER2-targeting agent according to the invention is provided, which leads to a signal reduction of HER2-Y1248, HER3-Y1289, AKT-S473, ERK1/2-T202/Y204 and/or PARP in a Westernblot when incubated with the A565 cell line.

In some embodiments, a bispecific HER2-targeting agent according to the invention is provided, which leads a signal reduction of HER2-Y1248, HER3-Y1289, AKT-S473 and/or ERK1/2-T202/Y204 in a Western blot when incubated with the HCC1419 cell line.

In some embodiments, a bispecific HER2-targeting agent according to the invention is provided, which leads to a signal reduction of HER2-Y1248, HER3-Y1289, AKT-S473 and/or ERK1/2-T202/Y204 in a Western blot when incubated with the HCC2218 cell line.

In some embodiments, a bispecific HER2-targeting agent according to the invention is provided, which leads to a signal reduction of HER2-Y1248, HER3-Y1289, AKT-S473, ERK1/2-T202/Y204 and/or PARP in a Western blot when incubated with the ZR7530 cell line.

The term "HER2-Y1248" in the context of the present specification refers to the human epidermal growth factor receptor 2 (NP_004439.2), wherein the residue Tyr1248 is phosphorylated.

The term "HER3-Y1289" in the context of the present specification refers to the human epidermal growth factor receptor 3 (NP_001005915.1), wherein the residue TYR1289 is phosphorylated.

The term "AKT-S473" in the context of the present specification refers to the protein kinase B (UniProt. No P31749), wherein the serine residue 473 is phosphorylated.

The term "ERK1/2-T202/Y204" in the context of the present specification refers to the mitogen-activated protein kinase 3 (NP_001035145.1), wherein the residue threonine 202 is phosphorylated, and the mitogen-activated kinase 1 (NP_002736.3), wherein the residue tyrosine 204 is phosphorylated.

The term "PARP" in the context of the present specification refers to the Poly ADP ribose polymerase 1 (UniProt. No. P09874).

A signal reduction in a Western blot refers to the decrease of the amount of the indicated protein that is immobilized and stained on the blotting membrane.

An example of signal reduction of the above described signals caused by the use of agents of the invention is shown in example 1.

In some embodiments, a bispecific HER2-targeting agent according to the invention is provided, which leads to an induction of apoptosis in at least 40% of BT474 cells, 8% of AU565 cells, 20% of HCC1419 cells an/or 20% of HCC2218 cells when incubated with the indicated cell line.

According to another aspect of the invention, a bispecific HER2-targeting agent is provided, wherein the bispecific HER2-targeting agent is characterized by a sequence selected from SEQ ID 03, SEQ ID 04, SEQ ID 05, SEQ ID 06, SEQ ID 07, SEQ ID 08 and SEQ ID 09.

According to another aspect of the invention, a bispecific HER2-targeting agent according to any of the above aspect or embodiments of the invention is provided for use in a method for preventing or treating malignant neoplastic diseases.

According to another aspect of the invention, a bispecific HER2-targeting agent according to any of the above aspect or embodiments of the invention is provided for use in a method for preventing or treating malignant neoplastic diseases, wherein the disease is characterized by cells overexpressing HER2.

A disease characterized by cells overexpressing HER2 or a HER2-positive disease is defined in the context of the present specification to be present if a high HER2 (protein) expression level is detected by immunohistochemical methods, by flow-cytometric methods such as FACS, or as HER2 gene amplification, for example a HER2 gene copy number higher than 4 copies of the HER2 gene per tumor cell, or by a combination of these methods, in samples obtained from the patient. One example of such disease is often breast cancer, where cells overexpressing HER2 can be cells obtained from breast tissue biopsies or breast tissue resections or in tissue derived from metastatic sites. One frequently applied method for detecting HER2 overexpression and amplification at the gene level is fluorescence in situ hybridization (FISH), which is also described in US2003/0152987, Cohen et al., which is incorporated herein by reference.

In some embodiments, a cell overexpressing HER2 is characterized by at least 2, 4, 6, 8, 10, 15, 20 or 25 copies of the HER2 gene (ERBB2 gene, Gene ID: 2064) in the nucleus in a FISH (fluorescence in-situ hybridization) assay.

In one embodiment, the copy number of the HER2 gene is measured by fluorescence in situ hybridization.

In one embodiment, a cell overexpressing HER2 is characterized by at least 2, 4, 6, 8, 10, 15, 20 or 25 signals per nucleus in a fluorescence in situ hybridization assay.

According to yet another aspect of the invention, a method is provided for treating a patient suffering from malignant neoplastic disease, comprising the administration of a bispecific agent according to any of the above specified aspects or embodiments of the invention to said patient.

In some embodiments, the malignant neoplasitic disease is a carcinoma of the stomach, endometrium, salivary gland, lung, kidney, colon, thyroid, pancreas or bladder.

### Detailed description of certain embodiments

### The principle of anti-tumor activity of bispecific targeting agents

The minimal setup of a bispecific targeting agent of the invention is composed of 3 units. Firstly, the bispecific binding agent comprises a binding unit targeting domain 1 of the extracellular domain (ECD) of HER2. Secondly, the bispecific binding agent comprises a binding unit targeting domain 4 of the ECD HER2. Thirdly, the bispecific binding agent comprises a linker unit or linker in-between the binding unit targeting domain 1 of HER2 and the binding unit targeting domain 4 of HER2, whose optimal length depends on the nature of both binding units.

In some embodiments, the linker or linker unit is a polypeptide linker.

In some embodiment, the linker is a polyglycine/serine linker. Such linker has the advantage that it is highly soluble in water, has a flexible fold, is resistant against proteolysis and adopts either a random coil or an extended structure.

In some embodiments, the linker is a short linker composed of the amino acids: GGGGS (G₄S). Bispecific constructs comprising 1 to 4 repeats of G₄S show superior anti-tumor activity. Bispecific constructs comprising 5 or more repeats of G₄S show decreasing anti-tumor activity with longer linker length. Other amino acid compositions might be used to connect the binding units.

In some embodiments, the linker or linker unit comprises flexible regions of binding scaffolds described above or is a chemical cross-linker, wherein both binding units are covalently connected by the linker. A chemical cross-linker in the context of the present specification refers to a compound capable of covalently connecting the first and the second polypeptide ligand of the invention. Examples for such chemical crosslinkers include, without being restricted to, glutaraldehyde, bissulfosuccinimidyl suberate, carbodiimide, bis(succinimidyl)penta(ethylene glycol), bis(succinimidyl) nona(ethylene glycol), bis(sulfosuccinimidyl) suberate, dimethyl suberimidate, an ethylene glycol characterized by formula (-CH2OH-CH2OH-)ₙ, wherein n is 2, 3, 4, 5, 6, 7, 8, 9, 10, 11, 12, 13, 14, 15, 16, 17, 18, 19, 20, 21, 22, 23, 24 or 25 and one or both termini of the ethylene glycol are substituted by a succimide or maleimide group, N-(_{K}-Maleimidoundecanoyloxy) sulfosuccinimide ester, sulfosuccinimidyl (4-iodoacetyl) aminobenzoate, 1,8-bismaleimidodiethyleneglycol and 1,11-bismaleimidotriethyleneglycol.

In some embodiments, the linker or linker unit is a dimerization domain or additional functional units inducing the dimerization of both binding units to connect both epitopes on HER2 or, in other words, dimerization domains.

A dimerization domain in the context of the present specification refers to a functional unit consisting of two polypeptides that are capable of specific binding to each other or dimerizing. The two polypolypetides may be part of the same polypeptide chain. Non-limiting examples for such dimerization domains are leucine zipper domains such as in GCN4 (UniProt. No. P03069 ), helix-helix domains, dimerization domains composed of beta-sheets, coiled coil helices such as in c-Jun (Uniprot. No. P05412) or c-Fos (Uniprot. No P01100), helix bundles like in the dimerization domain of the mip protein (Uniprot. No Q70YI1), helix-turn-helix motifs such as in the repressor protein cl (Uniprot. No. P03034) and antibody Fc regions.

Such linker unit may determine the anti-tumor activity of the bispecific targeting agent. The single binding units used in the examples disclosed here have no or only weak anti-tumor activity as single agents.

In some embodiments, linkers of other composition can be used, provided they bring said binding domains into a disposition leading to apoptosis in the targeted cell, as can be assayed by the methods provided herein.

In certain embodiments, DARPin fusions of the composition BinderA-FL-BinderB (FL standing for "flexible linker", for example a linker characterized by formula (G₄S)ₙ, wherein n is 1 to 5) are provided, which have strong anti-tumor activity (reduce cancer cell growth by 80-90%). Here "BinderA" can be a DARPin binding to the described epitope in subdomain 1 of HER2, or any other protein binding to an overlapping epitope, while "BinderB" can be a DARPin binding to HER2 subdomain 4, or any other protein binding to an overlapping epitope. "FL" refers to a flexible linker. The examples have been demonstrated with "BinderA" being either DARPin 9.29 (SEQ ID 18, SEQ ID 19, SEQ ID 20, or SEQ ID 21) or DARPin 9.26 (SEQ ID 14, SEQ ID 15, SEQ ID 16 of SEQ ID 17) binding to subdomain 1 (SEQ ID 01), while "BinderB" being either DARPin G3 (SEQ ID 25) or H14 (SEQ ID 26, SEQ ID 27, SEQ ID 28 or Seq ID 29) binding to subdomain 4 (SEQ ID 02). In the examples, "FL" was a linker of the composition (Gly-Gly-Gly-Gly-Ser)ₙ with n being 1, 2, 3 or 4.

The term "flexible linker" in the context of the present specification refers to a polypeptide connecting the first polypeptide ligand and the second ligand that is characterized by a random coil conformation or extended structure. A flexible linker may further be characterized by the absence of secondary structures such as helices or β-sheets or a maximal secondary structure content of 10%, 20% 30% or 40%.

The term "overlapping epitope" in the context of the present specification refers to an epitope that is partially identical to a certain epitope.

In some embodiments, binders to the most preferred epitopes are generated in using the display methods described above (phage display, ribosome display or yeast display). The DARPins 926, 929 or G3, whose sequences are disclosed in SEQ ID 14, SEQ ID 15, SEQ ID 16, SEQ ID 17, SEQ ID 18 SEQ ID 19, SEQ ID 20, SEQ ID 21 and SEQ ID 25 can be used as competitors. Their genes can be synthesized and they can be expressed and purified as detailed in Zahnd et al. (2007) J. Mol. Biol. 369, 1015-28. When the pool of binders selected in ribosome display or in phage display to the HER2 domains immobilized on magnetic beads or in microtiter plates are exposed to the competing DARPins, the binders will be preferentially eluted which show the same epitope.

In one embodiment, the mode of binding for one bispecific molecule, constructed according to the invention, is intermolecular. The linker unit in the bispecific agents determines the mode of binding. To be more precise, the length of the linker, and the orientation imparted on the binding domains by the attachment points of the linker influence whether the bispecific molecule binds in an intermolecular way, i.e. connecting two HER2 molecules. Hence, upon binding on a cell, the bispecific agents connect domain 1 of one HER2 receptor with the domain 4 of another HER2 receptor.

In some embodiments, the connection between both epitopes bound by the binding units of particularly active bispecific constructs is bridged by a short linker (5 amino acids or approx. 15 A).

In the structure of the whole extracellular domain of HER2 (PDB ID: 1 N8Z) (Cho HS, et al. (2003), Nature 421:756-760), the distance between the epitope on domain 1 and the epitope on domain 4 is at least 80 A long, and it is thus impossible that the bispecific molecule binds in an intramolecular way to this structure of HER2 (i.e., the domain 1 binding moiety and the domain 4 binding moiety cannot bind to domains 1 and 4 of one and the same Her2 molecule).

Domain 4 of the HER2 receptor is close to the transmembrane helix of the HER2 receptor and therefore restricted in its motional freedom. Domains 1, 2 and 3 are connected to domain 4 by flexible hinges. As it is known for other EGFR receptors, domains 1, 2 and 4 can change their relative orientation upon ligand binding. The conformational change in other EGFR receptors occurs from a state where domain 2 and 4 are in direct contact and domain 1 and 3 are separated (tethered conformation) to a state where domain 2 and 4 separate and domain 1 and 3 are connected via the respective ligand (Mark A. Lemmon, Ligand-induced ErbB receptor dimerization, Experimental Cell Research, 315(4), 2009, Pages 638-64). However, even in the tethered conformation, the distance between domain 1 and domain 4 remains too large to be compatible with a 15 A linker. Furthermore, the "tethered" conformation is thought to be absent in HER2, due several findings like e.g. the absence of stabilizing amino acids in the domain 4 contact region (e.g. G563 and H565 of HER3 are replaced with P and F) found in the crystal structure of HER2 (Cho et al., 2003 Nature 421: 756-760).

Hence, without wishing to be bound by theory, a conformation is postulated which is induced or stabilized by the bispecific targeting agents of the invention. This conformation is referred in the following as the stabilized inactive HER2 homodimer conformation. These stabilized inactive homodimers of HER2 may also exist in the context of larger HER2-HER2 interaction units like e.g. trimers, tetramers or up to HER2 clusters. The examples shown herein demonstrate that, in certain embodiments of the present invention, key tyrosine residues on the intracellular part of HER2 at the "phosphorylation tail" and in the kinase domain become dephosphorylated upon treatment with the bispecific targeting agents, while total HER2 levels remain quite constant in cancer cells that have not yet undergone apoptosis.

In certain embodiments, the stabilization of inactive HER2 homodimers by the bispecific targeting agents disclosed in the present invention consequently inhibits other HER2 interactions, e.g. with HER3. HER2 and HER3 receptor form a heterodimer with strong oncogenic, anti-apoptotic signaling. As a consequence of both inhibition of HER2 phosphorylation and HER3 phosphorylation, both downstream pathways PI3K-AKT and MAPK-ERK, and possibly other signaling pathways, become persistently inactivated and or down-regulated. Both pathways are down-regulated to such an extent that the pro-apoptotic protein BIMₛ becomes increasingly expressed in the cancer cells, leading to caspase activation and finally apoptosis.

### Delineation of the invention: design criteria of active bispecific molecules.

While the examples provided relate to the DARPins 9.26 or 9.29 linked to the DARPins G3 or H14 by a short flexible linker, a person skilled in the art can replace, in light of the information provided herein, any or both of said DARPins by other scaffolds or antibody Fab fragments or antibody scFv fragments or antibody domains, binding to an overlapping epitope on domain 1 or domain 4, respectively. If the orientation of the binding protein is not known from structural modeling or experimental structure determination, both linkages (BinderA-FL-BinderB and BinderA-FL-BinderB) can be readily constructed and tested in light of the information provided herein. The modular principle of the bispecific targeting agent makes it thus facile for the person skilled in the art to replace single parts in the construct by other binding or linking units.

### bispecific HER2 targeting

The present invention is based on a binding molecule that functions as a HER2-specific molecular crosslinker, which leads to the formation of inactive HER2 homodimers, instead of inhibiting HER2 dimerization. The mechanism of action of the targeting molecule of the invention is thus, in some embodiments, radically different from the HER2-directed therapies so far described. In these embodiments, the agents of the invention lead to HER2 homodimers being linked in such way that they become signalling-inactivated. The examples shown herein demonstrate the dephosphorylation of key tyrosine residues of the intracellular part of HER2. Hence, the so induced HER2 homodimers show a strongly reduced downstream signalling via the MAPK pathway, which is directly shown by the dephosphorylation of the MAP-kinase extracellular-signal regulated kinase 1 and 2 (Erk1/2).

In addition, these inactive HER2 homodimers fail to interact, in some embodiments, with other members of the EGF receptor family, most importantly with HER3. HER2-HER3 interactions and the corresponding phosphatidylinositol 3-kinase protein kinase B (PI3K-PKB, alternatively called PI3K-AKT) signalling pathway are known to drive cell proliferation and inhibit apoptosis in HER2-overexpressing cancer cells.

In still other embodiments, by preventing HER2-HER3 interactions by the stabilization of inactive HER2 homodimers, the downstream pathway PI3K-AKT becomes also inhibited. Hence, dephosphorylation of AKT was shown to result from application of the molecules of this invention. The simultaneous inhibition of both pathways, to a higher extent than achieved by the application of trastuzumab or pertuzumab or their combined action, stimulates, in yet other embodiments, the expression of Bcl-2-like protein 11 (BIM).

The expression of BIM, mainly the short isoform BIMₛ, finally leads, in certain embodiments, to the induction of the cell's intrinsic apoptotic program. As shown, the mode of action of the bispecific targeting agents is not the sum of actions of known molecular formats, because the building blocks, the single binding units, do not necessarily need to have anti-tumor activity by themselves. However, the connection of both disclosed epitopes in a preferentially intermolecular manner of preferred geometric disposition generates the potent anti-tumor agent.

Disclosed herein are two epitopes that may be bound by the HER2 targeting molecule, at the level of single amino acids of the HER2 extracellular domain, which are derived from multiple crystal structures of HER2 in complex with the respective binding proteins. Furthermore disclosed is the construction plan of such a bispecific molecule, which enables a person having ordinary skill in the art to readily construct such molecules.

In certain embodiments, the molecular structure is thus a bispecific binding molecule, which exhibits superior anti-tumor activity in comparison to trastuzumab and pertuzumab and induces apoptosis in HER2-dependent cancer cells. This bispecific binding molecule can, in certain embodiments, be further modified by fusing moieties like e.g. toxins, half life extending groups and other functionalities.

The invention is exemplarily shown with bispecific binding molecules that are built of designed ankyrin repeat proteins (Binz et al. (2004) Nat. Bio.Tech. 22 575-82; US2012142611 (A1) - 2012-06-07). However, there are no DARPin-specific functions in the molecules according to this disclosure, and thus the DARPins can be substituted by other binding proteins that serve to juxtapose the same epitopes such that they bring to HER2 molecules into a similar inactive orientation on the cell surface.

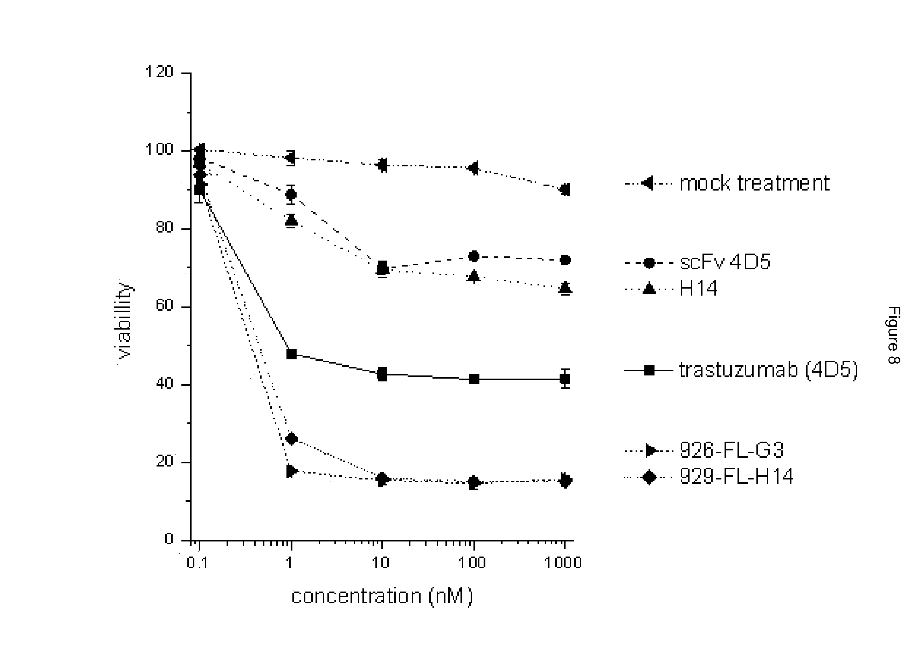

The agents and methods of the present invention are distinct from any method or reagent combination known in the art that binds to the same epitopes as the bispecific agent of the present invention. When converting IgGs into monovalent binding agents (by producing e.g. Fab fragments, or scFv fragments) the anti-tumor activity can vanish mostly or even completely. The results presented herein show that the scFv of 4D5 has only approx. 20% anti-tumor activity of the full length antibody in cell culture (measured in the absence of secondary functions like ADCC, Fig. 8).

Importantly, therefore, a bispecific agent comprising binding units that bind to the domain 1 of ECD HER2 and to domain 4 of ECD HER2 is not the sum of both modes of action that the respective antibody possesses, but is a new molecular entity according to the present invention.

Wherever alternatives for single separable features such as, for example, a first ligand, a second ligand, a bound epitope, a binding scaffold, a linker length or linker chemical constitution are laid out herein as "embodiments", it is to be understood that such alternatives may be combined freely to form discrete embodiments of the invention disclosed herein. Thus, any of the alternative embodiments for a domain 1 epitope may be combined with any of the alternative embodiments of domain 4 epitope, and these combinations may be combined with any linker mentioned herein.

The invention is further illustrated by the following examples and figures, from which further embodiments and advantages can be drawn. These examples are meant to illustrate the invention but not to limit its scope.

### Short description of the figures

- Fig. 1: shows the increased anti-tumor activity of bispecific targeting agents in cell proliferation assays. The Y axis shows cell viability in different cell lines expressing HER2 after treatment with any of the agents identified in the legend.
- Fig. 2: shows the quantification of cellular DNA content by flow cytometry in absence and presence of different anti-tumor agents.
- Fig. 3: shows the induction of apoptosis by bispecific targeting agents quantified by terminal transferase dUTP nick end labeling (TUNEL) assays and flow cytometry.
- Fig. 4: shows the Western blot analysis of HER2/HER3 signaling pathway, PI3K/AKT and MAPK pathway and downstream targets of cell cycle and apoptosis.
- Fig. 5: shows quantitative western blot analyses of the treatment time course measuring HER2/HER3 receptor expression and phosphorylation after treatment with anti-HER2 binding agents.
- Fig. 6: shows the inhibition of ligand-stimulated growth by bispecific targeting agents in cell proliferation assays.

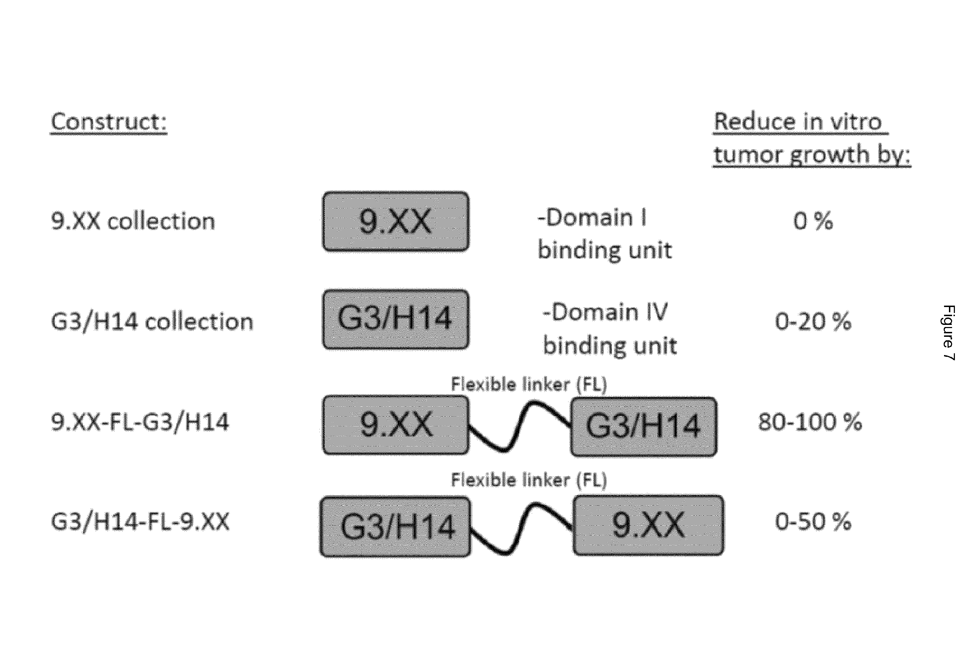

- Fig. 7: shows the pictorial summary of anti-HER2 targeting formats.
- Fig. 8: shows the anti-tumor activity of bispecific binding reagents quantified by cell proliferation assays, shown is the effect of different concentrations of anti-tumor agents on the cell viability.
- Fig. 9: shows the anti-tumor activity of all constructs that share a similar epitope on domain I of ECD HER2 in a cell proliferation assay, the Y-axis showing the viability of BT474 cells after treatment with any of the agents identified in the legend.
- Fig. 10: shows the anti-tumor activity of single binding agents. The Y-axis shows the viability of BT474 cells after treatment with any of the agents identified in the legend.
- Fig. 11: shows the effect of combination treatment of the single anti-HER2 binding agents on the cell viability (Y-axis) of BT474 cells after treatment with any of the agents identified in the legend.
- Fig. 12: shows the effect of different anti-tumor agents on the viability of trastuzumab-resistant cell lines in cell proliferation assays, the Y-axis showing the viability of the cell lines determined by absorbance of reduced XTT after treatment with any of the agents identified in the legend.
- Fig. 13: shows the effect of trastuzumab and pertuzumab on the anti-tumor activity of the bispecific targeting agents in cell proliferation assays. Data presentation as in Fig. 11.
- Fig. 14: shows the anti-tumor activity of different anti-tumor agents in cell proliferation assays. Data presentation as in Fig. 11.
- Fig. 15: shows the results of an ELISA with bispecific targeting agents and pertuzumab. The Y-axis shows the concentration of the agents identified in the legend bound to HER2 in presence of pertuzumab.
- Fig. 16: shows the competitive binding of G3 and H14 with trastuzumab. The Y-axis shows the percent binding of the agents identified in the legend to domain 4 of HER2 in presence of trastuzumab.
- Fig. 17: shows the binding affinity, binding stoichiometry and binding mode of single binding units and bispecific binding agent to HER2 on the surface of cancer cells; A and B, on-rate determination of single binding agents; C, on rate determination of bispecific binding agents; D, off rate determination of single and bispecific binding agents; MFI mean fluorescence intensity.

### Examples

### Example 1: Anti-tumor activity of the bispecific anti-HER2 binding agents in comparison to trastuzumab and pertuzumab

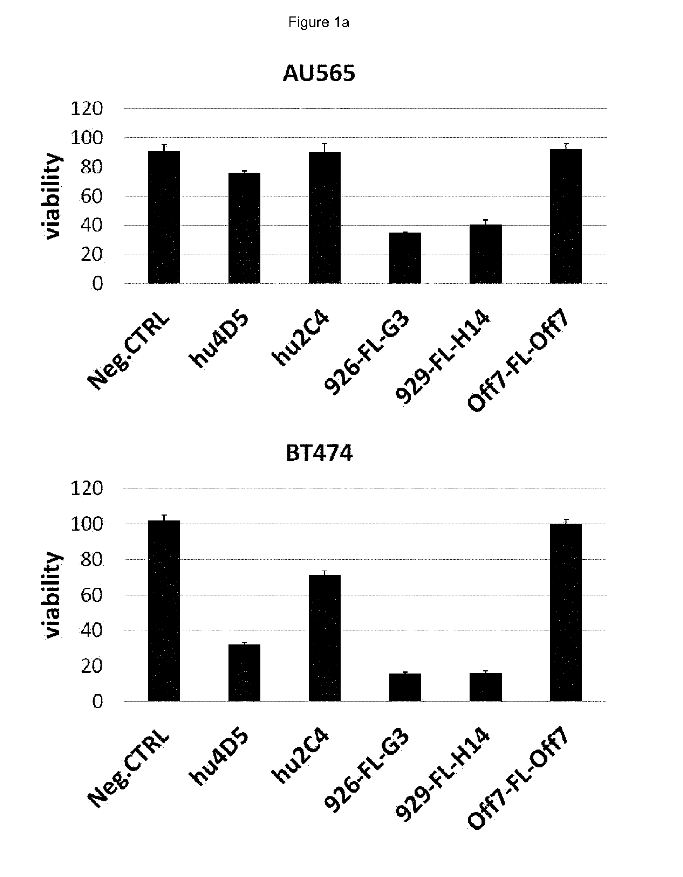

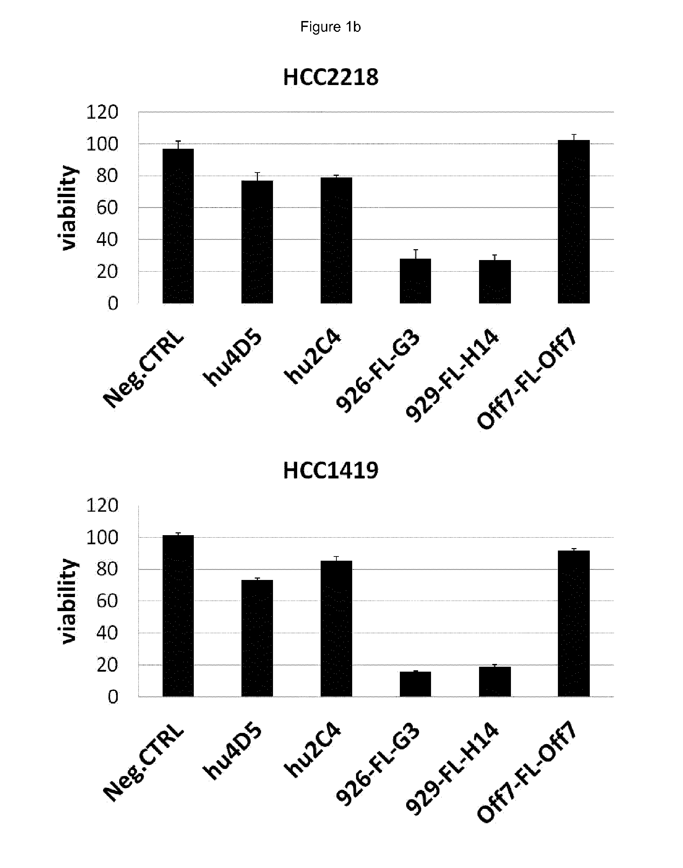

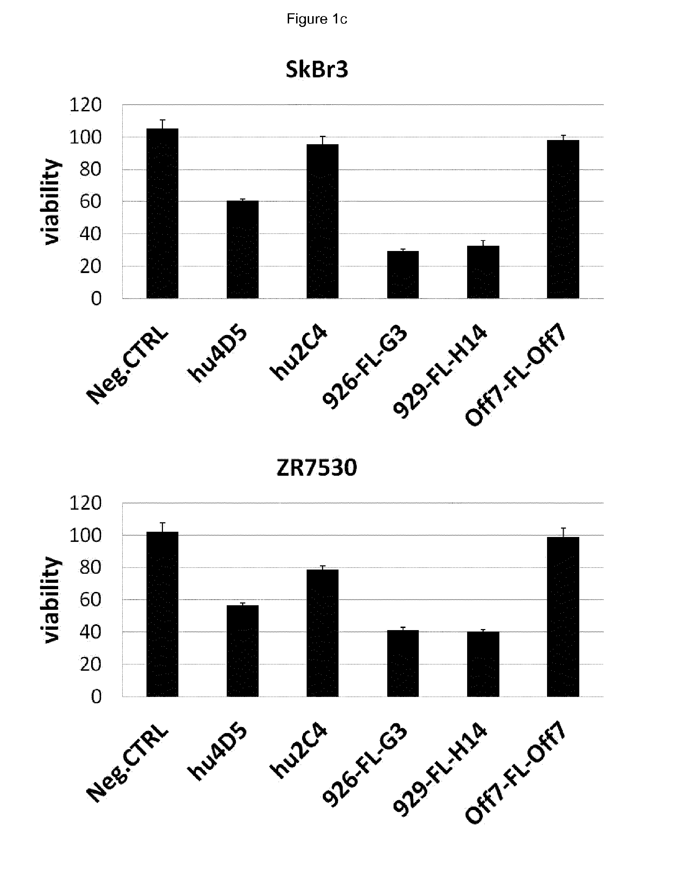

A XTT cell proliferation assay was performed with a panel of HER2 overexpressing cancer cell lines in 96-well 1D or 2D tissue culture plates (Fig. 1). A defined number of cells were seeded in RPMI1640 medium containing 10% fetal calf serum (FCS). Cancer cells were treated for 4 days with 100 nM of anti-HER2 agents and controls. Measuring points were recorded in triplicates. XTT cell viability assays were developed according to the manufacturer's protocol. At a concentration of 100 nM, all anti-HER2 agents show maximal anti-tumor activity (titration not shown). The average of three data points is plotted with standard error. Data were normalized against the negative control on each plate, which corresponds to untreated cells (maximal growth). Bispecific targeting agents reduce cell growth of HER2-dependent cancer cells by 60- 80%, while trastuzumab (hu4D5) reduces cell growth by only 20- 60%. Bispecific targeting agents (926-FL-G3, 929-FL-H14) show consistently strong anti-tumor activity in all cell lines, while some cell lines show resistance against trastuzumab treatment. Sensitive cell lines can be roughly defined as HER2 dependent (e.g. HER2 overexpressing) and absent of any PI3K activating mutation.

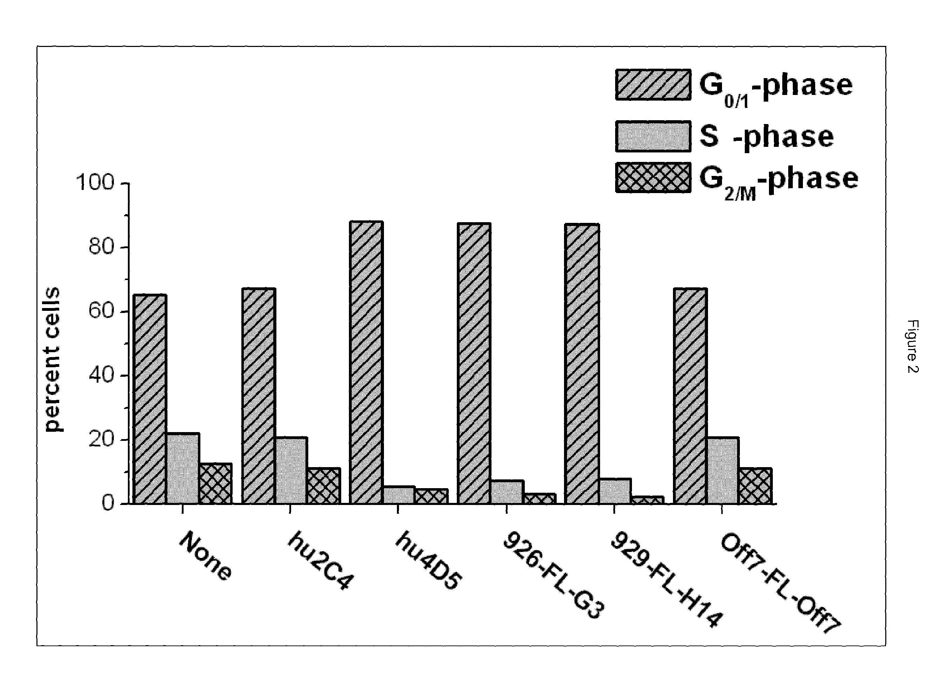

Figure 2 shows that bispecific targeting agents block entrance into S-phase and induce accumulation in G_{0/1}-Phase. BT474 cells were seeded 16 h before treatment in RPMI1640 containing 10% FCS. Anti-HER2 agents were added to a final concentration of 100 nM and cells were treated for 3 days. Afterwards, cells were fixed in 70% EtOH and stained with propidium iodide (PI). FACS measurements were gated to exclude cell debris in a forward vs. side scatter plot and 10⁴ events were recorded. PI fluorescence histograms were analyzed by FlowJo 7.2.5 software, and cell cycle distribution was fitted using the Dean-Jett-Fox algorithm, which excludes the apoptotic SubG1-population of cells. Treatment with bispecific targeting agents (926-FL-G3, 929-FL-H14) reduces S-phase and G_{2/M}-phase content in HER2-dependent cancer cells. It was shown that trastuzumab (hu4D5) treatment induces cell cycle arrest by blocking entrance into S-phase of sensitive HER2 dependent cancer cell lines. Here it is shown that bispecific targeting agents also induce cell cycle arrest in trastuzumab sensitive cell lines.

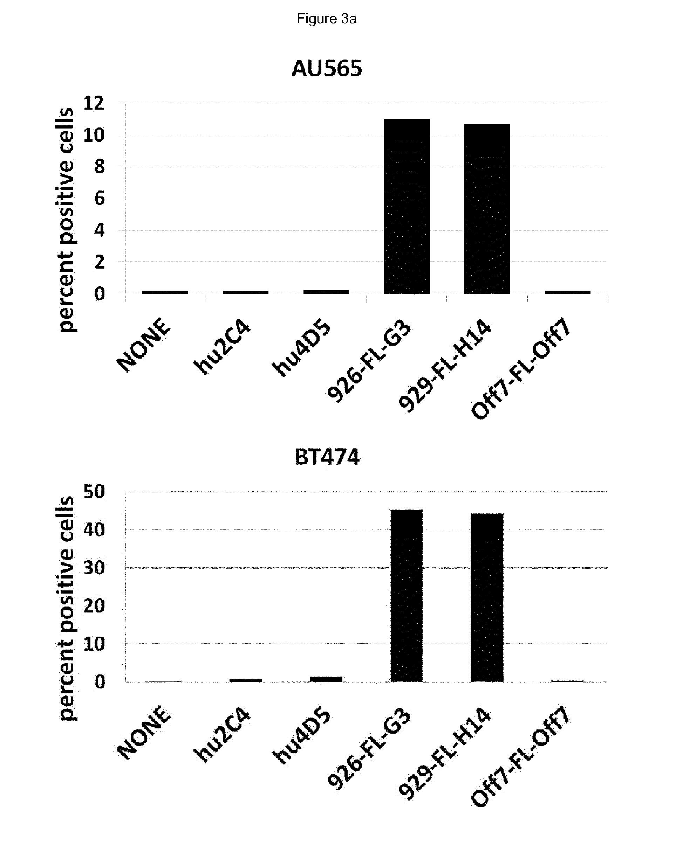

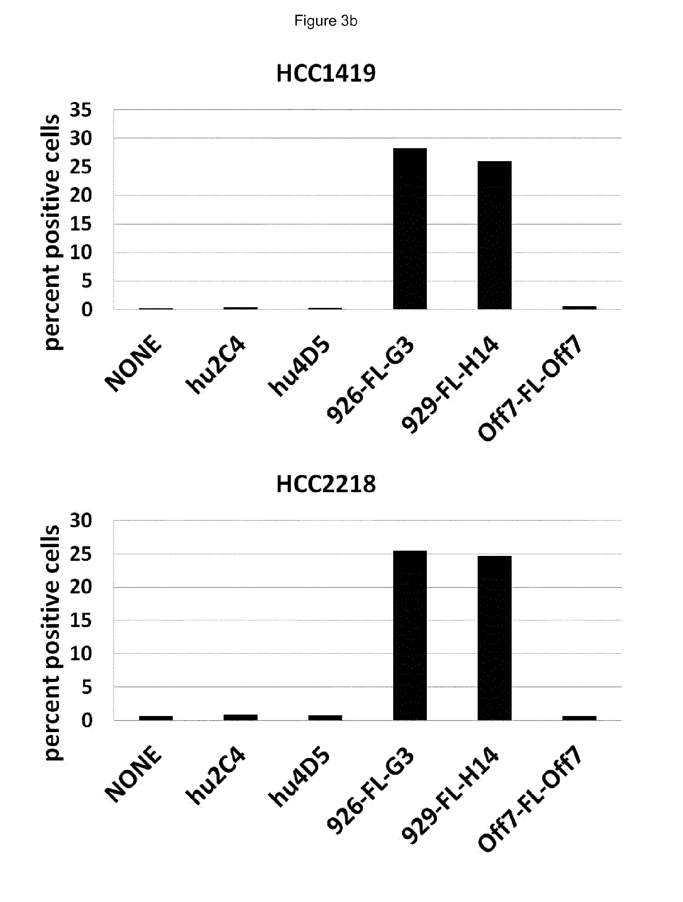

The terminal transferase dUTP nick end labeling (TUNEL) assay and quantification by flow cytometry was used to determine the portion of apoptotic cells upon treatment with anti-HER2 agents (Fig. 3). Cancer cells were seeded 16 h before treatment in RPMI1640 containing 10% FCS. Anti-HER2 agents (pertuzumab: hu2C4; trastuzumab: hu4D5; bispecific targeting agents: 926-FL-G3, 929-FL-H14; mock treatment: Off7-FL-Off7) were added to a final concentration of 100 nM and cells were treated for 3 days. Fractions of adherent and non-adherent cells were pooled. Cells were fixed in cold PBS containing 3% para-formaldehyde, permeabilized in cold PBS containing 1% Triton X-100 for 3 min, washed three times with cold PBS and labeled. FACS measurements were gated to exclude cell debris in a forward vs. side scatter plot and 10⁴ events were recorded. Counts were gated for single cells, measurements were plotted as a histogram in the FL1 channel in a one parameter FL1 histogram plot (FITC fluorescence on x axis and counts on y axis) and positive cells were quantified by a regional gate. Population of TUNEL positive (shift towards higher FL1) cells were quantified by one-dimensional regional gates which exclude TUNEL negative cells (auto fluorescence). Gates were applied according to negative control to exclude auto fluorescent cells. Treatment with bispecific targeting agents induces DNA degradation in HER2-dependent cancer cells, which is a hallmark of apoptosis. The number of TUNEL-positive cells correlates with the formation of a Sub-G₁ population, as determined by cell cycle analysis (data not shown). The quantification shows 30- to 80-fold higher TUNEL signals for the bispecific binding agents than for trastuzumab or pertuzumab in HER2-dependent cancer cells.

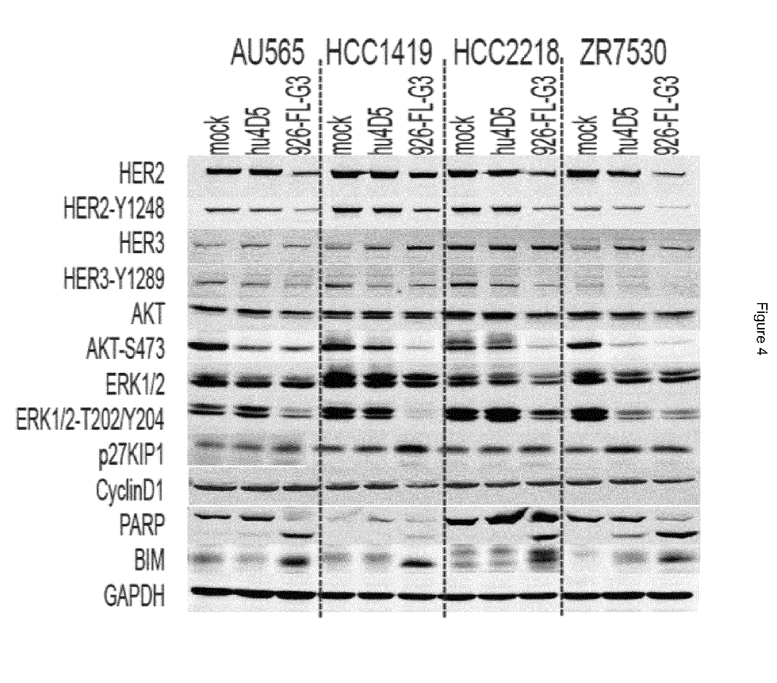

For Western blot analysis of the HER2/HER3 signalling pathway, PI3K/AKT and MAPK pathway and downstream targets of cell cycle and apoptosis, cancer cells were seeded 24 h before treatment in RPMI1640 containing 10% FCS. Anti-HER2 agents were added to a final concentration of 100 nM and cells were treated for 3 days. Afterwards, the fraction of detached apoptotic cells was collected and removed by centrifugation. Remaining attached cells were washed with cold PBS and scraped off into cold PBS_I (PBS containing protease inhibitors (Pefabloc, Leupeptin, Pepstatin, Marimastat) and phosphatase inhibitors (sodium orthovanadate, sodium metavanadate, sodium molybdate, β-glycerol phosphate, sodium fluoride)) on ice. Both cell fractions were pooled and washed in PBS_I. Afterwards, cells were lysed in PBS_I containing 1% Triton X-100 for 30 min at 4°C on a rocker, and cell lysates were centrifuged at 20,000 g for 20 min at 4°C. Protein concentrations of the respective cell lysates were determined by BCA assays and samples were taken up in lithium dodecyl sulfate (LDS) loading buffer containing β-mercaptoethanol for complete reduction. Samples were heated for 5 min at 80°C. Samples were loaded on 10% SDS-PAGE and afterwards blotted on PVDF_FL membrane (Millipore) according to a BioRad protocol. After incubation with primary detection antibodies, western blots (Fig 4.) were stained by secondary antibodies labeled with an infrared dye, and membranes were scanned on an Odyssey IR-fluorescence scanning system (LICOR). The following primary detection antibodies were used: Human Epidermal Growth Factor Receptor 2 (HER2); Phospho-Tyr 1248 Human Epidermal Growth Factor Receptor 2 (HER2-Y1248); Human Epidermal Growth Factor Receptor 3 (HER3); Phospho- Tyr 1289 Human Epidermal Growth Factor Receptor 3 (HER3-Y1289); Protein Kinase B (AKT); Phospho- Ser 473 Protein Kinase B (AKT-S473); p44/42 MAPK (ERK1/2); Phospho- Thr202/Tyr204 p44/42 MAPK (ERK1/2-T202/Y204); Cyclin-depended Kinase Inhibitor 1B (p27KIP1); CyclinD1 (CyclinD1); Poly ADP Ribose Polymerase (PARP); Bcl-2 Interacting Mediator of Cell Death (BIM); Glyceraldehyde 3-Phosphate Dehydrogenase (GAPDH).

For quantitative western blot analysis of the time course treatments, BT474 cells were seeded 24 h before treatment in RPMI1640 containing 10% FCS. Anti-HER2 agents were added to a concentration of 100 nM and cells were treated for 3 days. Afterwards, the fraction of only loosely adherent cells was washed away with cold PBS. Attached cells were scraped off in cold PBS_I (PBS containing protease inhibitors (Pefabloc, Leupeptin, Pepstatin, Marimastat) and phosphatase inhibitors (sodium orthovanadate, sodium metavanadate, sodium molybdate, β-glycerol phosphate, sodium fluoride)) on ice. Afterwards, cells were lysed in PBS_I containing 1% Triton X-100 for 30 min at 4°C on a rocker and cell lysates were centrifuged at 20,000 g for 20 min at 4°C. Protein concentrations of the respective cell lysates were determined by BCA assays. HER2 receptor was immunoprecipitated by 901-FL-zHER2, a DARPin-affibody fusion construct, linked to Biosupport Ultra Link beads. HER2 receptor was depleted from BT474 cell lysate (corresponding to 1 mg protein in the lysate). Beads were washed three times with cold PBS_I. HER2 receptor was eluted from beads by heating to 80 °C for 5 min in LDS loading buffer containing β-mercaptoethanol for complete reduction. HER3 samples were heated for 5 min at 80 °C in LDS loading buffer containing β-mercaptoethanol for complete reduction. Samples were loaded on 10% SDS-PAGE and afterwards blotted on PVDF_FL membrane according to the BioRad protocol. Western blots were stained by secondary antibodies labeled with an infrared dye and membranes were scanned on an Odyssey IR-fluorescence scanning system (LICOR).

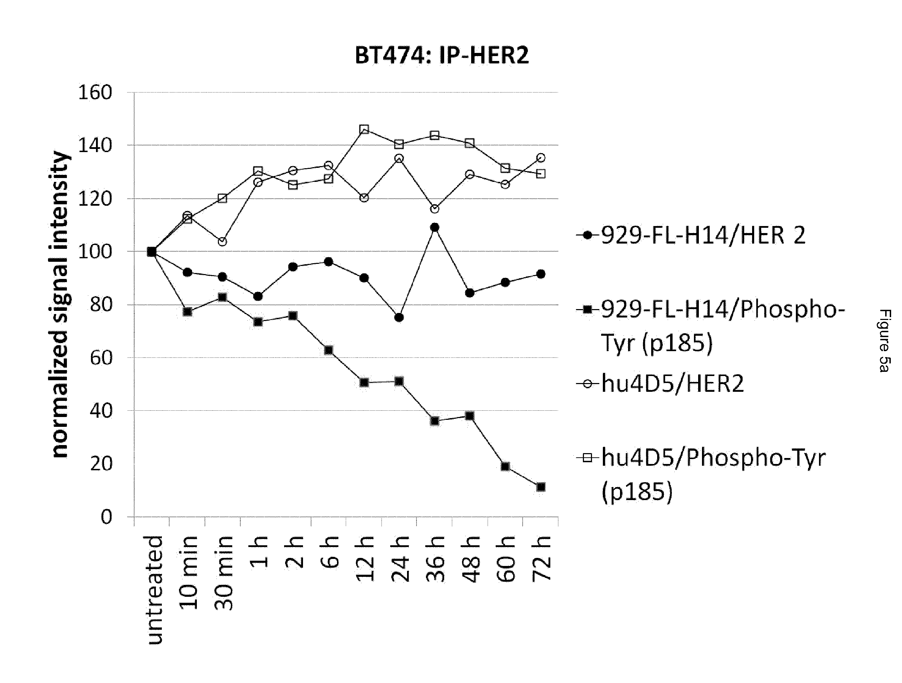

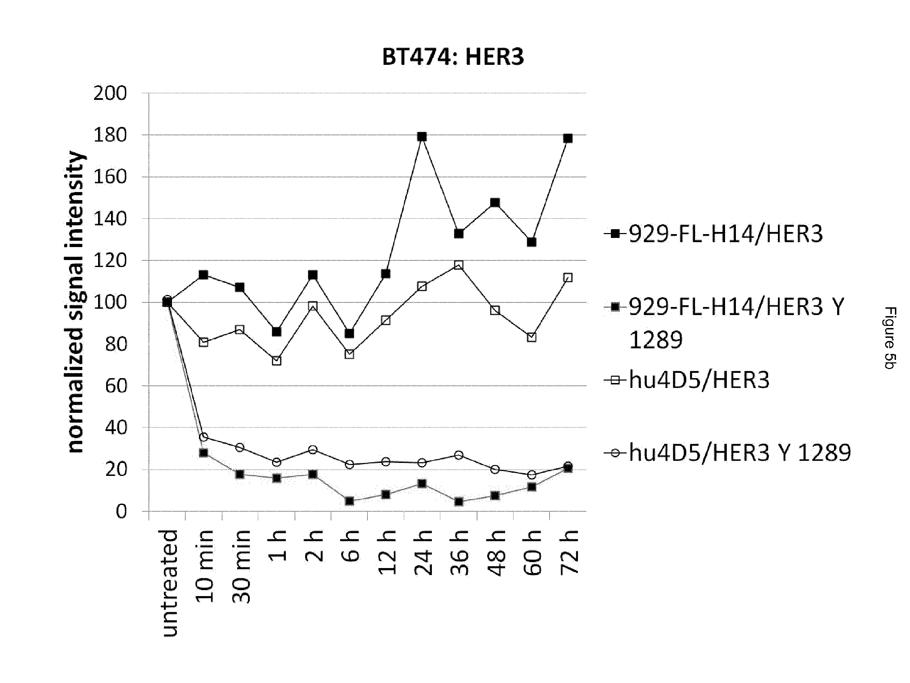

Bispecific agents down-regulate phospho-HER2 levels consistently in all HER2-dependent cancer cells. Down-regulation of phospho-HER2 can correlate with down-regulation of HER2 expression level, which was observed in the fractions of apoptotic cells (Fig. 4). Constant HER2 expression levels were observed in the fraction of attached cells for e.g. BT474 and SkBr3, while phospho-HER2 levels were strongly reduced (Fig. 5). Therefore, down-regulation of HER2 expression can be observed in the apoptotic fraction of HER2-dependent cancer cells but is probably not the cause for induction of apoptosis. Rather, down-regulation of phosho-HER2 together with phosho-HER3 is the cause for induction of apoptosis. Down-regulation of phospho-HER3 can be observed after treatment with bispecific targeting agents and trastuzumab. Bispecific targeting agents show stronger down-regulation of phospho-HER3 than trastuzumab. Up-regulation of HER3 expression can be observed after treatment with bispecific targeting agents. A feedback loop sensing inhibition of phospho-AKT and, consequently, up-regulation of HER3 expression has been proposed. Therefore, up-regulation of HER3 expression can be further evidence for stronger inhibition of the HER3/PI3K/AKT pathway inhibition of bispecific targeting agents. Bispecific targeting agents reduce phospho-AKT (downstream HER3) and phospho-ERK (downstream HER2) signaling simultaneously. Trastuzumab treatment mainly down-regulates phospho-AKT, while in ZR7530 cells, trastuzumab treatment leads to a down regulation of phospho-ERK. Cell cycle regulators p27KIP1 (cyclin-dependent kinase inhibitor) is up-regulated and CyclinD1, which initiated G1/S-phase transition, is down-regulated in several HER2-dependent cancer cell lines. Again, inhibition of the cell cycle is not necessarily observed by bispecific targeting agents, but cell cycle arrest is observed in cell lines which are sensitive to trastuzumab treatment. BIMₛ up-regulation and PARP cleavage (up-regulation of PARP p89) is observed in all HER2-dependent cancer cell lines, which show induction of apoptosis after treatment with bispecific targeting agents. ZR7530 and BT474 cells show also PARP cleavage after treatment with trastuzumab, but bispecific targeting agents show consistently stronger signals.

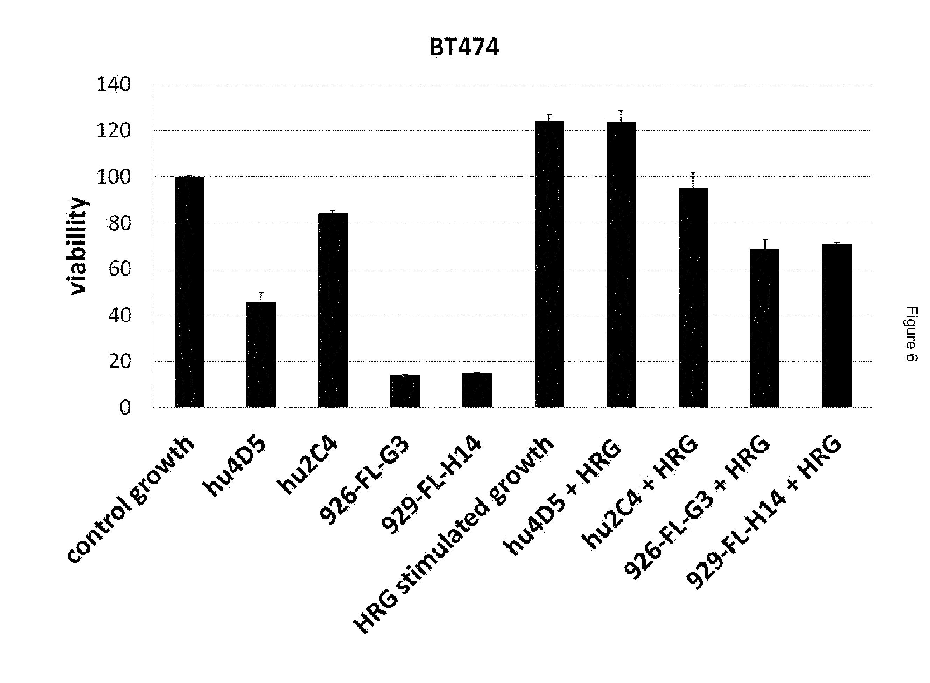

XTT cell proliferation assays were performed with BT474 cells in 96 well 1D tissue culture plates (Fig. 6). Cells were seeded at a density of 10⁴ cells/cm² 16 hours before treatment in RPMI1640 containing 1% FCS (low concentration of additional growth factors). Cells were pre-treated with 100 nM anti-HER2 agents for 2 hours. Afterwards, cells were stimulated by adding heregulin beta-1 (HRG) to a concentration of 1 nM (Recombinant Human NRG1-β1/HRG1-β1: 26.9 kDa). HRG treatment leads to an increase of viable BT474 cells by 20%, compared to the control growth in 1% FCS alone (Fig. 6). The single treatments with anti-HER2 agents are thus compared to the corresponding controls in the absence (100% viability) or presence (120% viability) of HRG. Trastuzumab (hu4D5) treatment reduced viability by 50-60% in the absence of HRG, but did not show anti-tumor activity in ligand-stimulated cancer cells. Trastuzumab completely looses anti-tumor activity in presence of 1 nM HRG. Pertuzumab (hu2C4) treatment reduced viability by 20-30% in the presence or absence of HRG. Bispecific targeting agents reduced the viability by 80-90% in the absence of HRG and also showed 40-50% reduction in the presence of HRG. Therefore, the bispecific targeting agents show strongest anti-tumor activity both in the presence and in the absence of HRG. The additive effect of trastuzumab and pertuzumab resembles the individual maximal anti-tumor activity of the single agents (data not shown), but has no significant mechanistically synergism in in-vitro models. Therefore, the mechanism of action of bispecific targeting agents is superior to the treatment with trastuzumab combined with pertuzumab in in-vitro models. The treatment with the bispecific reagents exceeds the effect of the sum of effects from both antibodies.

### Example 2: Construction plan of bispecific anti-HER2 targeting agents that induce apoptosis in HER2 dependent cancer cells

### Generation of binding agents that form the components of the active molecule

Binding molecules were obtained by ribosome display selection of ankyrin repeat protein libraries for specific binding to the full length extracellular domain of HER2 (ECD HER2) by methods previously disclosed (Zahnd et al. (2006) J. Biol. Chem. 279, 18870-18877).

### Preparation of the biotinylated HER2 target

In order to obtain binders to the individual domains, the different individual domains of HER2 were individually expressed in insect cells, using a baculovirus expression system. Thereby, it is guaranteed that binders selected will be directed towards the domain of interest. Briefly, recombinant ErbB2-ectodomains carrying an N-terminal melittin signal sequence (MKFLVNVALVFMVVYISYIYA) and an N-terminal His6 tag were expressed in Spodoptera frugiperda (Sf9) cells using baculoviral vectors. Sf9 cells were grown to a density of 4 × 10⁶ cells/mL and co-infected with the respective virus at a MOI of 1. 72 h post-infection, cells were harvested by centrifugation (30 min, 5,000g, 4 °C) and the cleared medium was subjected to immobilized metal ion affinity chromatography (IMAC) purification with Ni-NTA Superflow purification resin.

To generate binders against any domain of the extracellular region, the extracellular domain (residues 1-621) of HER2 was used as target for the selection with ribosome display (Zahnd et al., J. Biol. Chem. (2006) 281: 35167-35175) or, to generate binders against the first three domains, HER2 residues 1-509 was used.

For immobilization, aliquots of these target proteins (200-600 µg) were chemically biotinylated using EZ-Link Sulfo- NHS-SS-Biotin. Due to the size difference of the target proteins, a variable molar excess of the biotinylating reagent relative to the target protein was used (6-fold for HER2 1-621 or 1-509, 3-fold for the single domains). Reaction conditions were used according to the supplier's manual. Successful biotinylation was confirmed by ELISA and Western blot experiments. The biotinylated HER2 constructs were dialyzed extensively against PBS150.

Target proteins had to be immobilized for selection. To avoid partial protein denaturation of the target proteins that may result from direct immobilization on solid plastic (i.e. polystyrene) surfaces, biotinylated target proteins were bound to neutravidin or streptavidin, which had been immobilized directly on a solid plastic surface, as follows: neutravidin (66 nM, 100 µl/well) or streptavidin (66 nM, 100 µl/ well) in PBS was immobilized on MaxiSorp plates (Nunc, Denmark) by incubation at 4 °C overnight. The wells were blocked with 300 µl of PBSTB (PBS containing 0.1% Tween-20, 0.2% BSA) for 1 h at room temperature. Binding of the biotinylated target proteins (100 µl, 100 nM for selection) in PBSTB was allowed to occur for 1 h at 4 °C. For the first selection round on immobilized target protein, requiring larger volumes, neutravidin (66 nM, 4 ml/tube) in PBS was immobilized on MaxiSorp Immunotubes by incubation at 4 °C overnight. The tubes were blocked with 4 ml of PBSTB for 1 h at room temperature. Binding of the biotinylated target proteins (4 ml, 100 nM) in PBSTB was allowed to occur for 1 h at 4 °C. For selection on immobilized target protein, neutravidin and streptavidin were used alternately in selection rounds to avoid selection of binders against these proteins.

### Ribosome Display

Ribosome display followed the published protocols (Dreier et al. (2012) Methods Mol. Biol. 805, 261-286; Zahnd et al. (2007) Nat. Methods 4, 269-279.) Typically 3 or 4 rounds were carried out. The first round was always carried out on plates, the later rounds in some of the selection on plates, in others in solution, where the biotinylated HER2 target is then bound to streptavidin-coated magnetic beads, as described in the protocols in detail (Dreier et al. (2012) Methods Mol. Biol. 805, 261-286; Zahnd et al. (2007) Nat. Methods 4, 269-279.).

In the forth round, the selection pressure was increased by applying off-rate selection. For this purpose, after the in vitro translation was stopped by 5-fold dilution into ice-cold WBT buffer (50 mM Tris acetate, pH 7.5, 150 mM NaCl, 50 mM Mg(CH3COO⁻)₂, 0.05% Tween 20), biotinylated HER2 construct was added to a final concentration of 10 nM, and the translation was allowed to equilibrate for 2 h at 4 °C. The translation reaction was split into two aliquots, and non-biotinylated HER2 construct was added to a final concentration of 1 µM to each aliquot, corresponding to a 100-fold excess over biotinylated antigen. The aliquots were incubated for 2 and 20 h, respectively, to increase the selection stringency for slower off rates. Ribosomal complexes were recovered using 30 µl of streptavidin-coated magnetic beads. In a subsequent round, 175 nM biotinylated HER2 construct was immobilized on a NeutrAvidin-coated Maxisorp plate, i.e. rather non-stringent conditions to collect the binder ("collection round") (Dreier et al. (2012) Methods Mol. Biol. 805, 261-286; Zahnd et al. (2007) Nat. Methods 4, 269-279.)

In all selection rounds on solid-phase immobilized HER2 construct, a prepanning step of 30 min on a neutravidin-coated Maxisorp plate was performed as described (Dreier et al. (2012) Methods Mol. Biol. 805, 261-286; Zahnd et al. (2007) Nat. Methods 4, 269-279.). After prepanning, the translation extracts were allowed to bind for 45 min to HER2 construct-coated Maxisorp plates. Retained complexes were extensively washed with WBT buffer.

### Phage display

Phage display of the DARPin library followed the published protocol (Steiner et al. (2008) J. Mol. Biol. 382, 1211-27). The immobilization of the various biotinylated HER2 constructs has been described above.

Unless stated otherwise, all steps of the phage display selection were carried out at room temperature. Selection rounds were performed either on biotinylated target protein in solution with subsequent capturing on streptavidin-coated magnetic beads (referred to as: "target protein in solution") or on biotinylated target protein bound to neutravidin or streptavidin, which had been directly immobilized on a solid plastic surface (referred to as: "immobilized target protein"), as described below. Very good results were obtained when performing the first selection round of selection on immobilized target protein, presumably because of the greater efficiency of capturing binders (especially important in the first round), followed by further rounds on target protein in solution, presumably because of the lower enrichment of background binders

### Selection on target proteins in solution

When the first selection cycle was done in solution, about 2.5 x 10¹³ phage particles of the phage DARPin library were incubated for 1 hour with 100 nM biotinylated target protein in 2 ml PBSTB for the first round of selection. In subsequent selection rounds, about 10¹² phage particles were used (see below). The phage-antigen complexes were then captured on 100 µl streptavidin-coated paramagnetic beads (10 mg/ml) for 20 min. After washing the beads eight times with PBST (PBS, 0.1% Tween-20) the phage particles were eluted with 200 µl of 100 mM triethylamine (Et3N, pH not adjusted) for 6 min, followed by 200 µl of 100 mM glycine-HCl, pH 2, for 10 min. Eluates were neutralized with 100 µl of 1 M Tris-HCl, pH 7, or 18 µl of 2 M Tris-base, respectively, combined and used to infect 5 ml of exponentially growing E. *coli* XL1-Blue cells. After shaking for 1 hour at 37 °C, cells were expanded into 50 ml of fresh 2YT medium (5 g NaCl, 10 g yeast extract, 16 g tryptone per liter) containing 10 µg/ml cam and incubated at 37 °C with shaking. After a maximum of 5 h (shorter times if OD₆₀₀ = 0.5 was reached earlier), isopropyl-β-D-thiogalactoside (IPTG) was added to a final concentration of 0.2 mM and 15 minutes later the phage library was rescued by infection with VCS M13 helper phage at 10¹⁰ pfu (plaque forming units) per ml (multiplicity of infection 20). Cells were grown overnight at 37 °C without the addition of kanamycin. Cells were removed by centrifugation (5600 g, 4 °C, 10 min) and 40 ml of the culture supernatant was incubated on ice for 1 hour with one-fourth volume of ice-cold PEG/NaCl solution (20 % polyethylene glycol (PEG) 6000, 2.5 M NaCl). The precipitated phage particles were then collected by centrifugation (5600 g, 4 °C, 15 min) and redissolved in 2 ml of PBS and used for the second round of selection.

For the subsequent selection rounds, about 10¹² of the amplified phage particles were used as input and incubated with 100 µl of streptavidin-coated paramagnetic beads for 1 h to remove unspecific and streptavidin binding phage particles. After removing the beads, phage particles were incubated for 1 hour with 100 nM biotinylated target protein, complexes were captured on fresh beads, beads were washed 12 times with PBST, phages eluted with 400 µl of 100 mM glycine-HCl, pH 2, for 10 min, the eluate neutralized with 36 µl of 2 M Tris-base and phage particles amplified and purified as described above.

After three rounds, enrichment of phage particles displaying DARPins binding specifically to the HER2 target construct was monitored by phage ELISA. About 5 x 10¹⁰ phage particles (estimated spectrophotometrically) of the initial library and the amplified pools of each selection round were pipetted to wells with and without immobilized target protein and incubated at RT for 2 h. After washing the wells four times with 300 µl of PBST, bound phage particles were detected with mouse anti-M13 antibody horseradish peroxidase conjugate and soluble BM Blue peroxidase (POD) substrate.

### Selection on immobilized target proteins

For the first selection cycle about 3.5 x 10¹³ phage particles of the phage DARPin library were added to an immunotube containing the immobilized target protein (biotinylated target protein bound to neutravidin, which had been directly immobilized on the solid plastic surface) and incubated with rotation for 2 h. After rinsing the tube ten times with PBST, the phage particles were eluted with 500 µl of 100 mM Et₃N (pH not adjusted) for 6 min, followed by 500 µl of 100 mM glycine-HCl, pH 2, for 10 min. Eluates were neutralized with 250 µl of 1 M Tris-HCl, pH 7, or 45 µl of 2 M Tris-base, respectively, combined and used to infect 13 ml of exponentially growing *E*. *coli* XL1-Blue cells. After shaking for 1 hour at 37 °C cells were expanded into 130 ml of fresh 2YT medium containing 10 µg/ml chloramphenicol (cam) and incubated at 37 °C with shaking. Phage amplification and precipitation was done as described above.

In the subsequent selection rounds about 10¹² of the amplified phage particles were first incubated in a blocked immunotube (coated either with neutravidin or streptavidin used for immobilization of the target protein in the previous round of selection and BSA) one hour to remove neutravidin, streptavidin or unspecific binding phage particles. For the binding selection the phage particles were incubated for one hour in four wells containing the immobilized biotinylated target protein (directly coated neutravidin or streptavidin were alternately used in subsequent selection rounds). The wells were washed 12 times with PBST, phages eluted from each well with 100 µl of 100 mM glycine-HCl, pH 2, for 10 min, the combined eluates neutralized with 36 µl of 2 M Tris-base and phage particles amplified and purified as described above. After three rounds, enrichment was determined by phage ELISA as described above.

### Phage display from antibody library

Single-chain antibody fragments (scFv) were selected for binding to HER2, which have a molecular weight of 30 kDa, from HuCAL-1 (Knappik et al., 2000), a library of synthetic human antibody fragments. The library has a diversity of about 2 × 10⁹ members (Knappik et al., JMB, 2000, 296(1), 57-86). M13 phages presenting the HuCAL-1 scFv library as a fusion to the CT domain of g3p coat protein were selected for binding to soluble biotinylated HER2 domain 1 or domain 4, which was immobilized on neutravidin or streptavidin on microtiter wells as described above.

Phage selections were performed by incubating 50 pmol of biotinylated antigen with 1 pmol of phages in 100 µl PBS 0.5% BSA for 1 h at 4°C. The complexes were captured with 1 mg of BSA-blocked streptavidin magnetic particles and washed 10 times with PBS 0.5% BSA. Bound phages were eluted with 100 mM glycine, pH 2.2, and neutralized with the same volume of 1 M Tris, pH 8. *E. coli* TG1 cells were infected with eluted phages and plated on LB agar plates containing 1% glucose and 34 mg/l chloramphenicol. The plates were incubated overnight at 30°C, and bacteria were scraped off to inoculate 2xYT medium containing 1% glucose and 34 mg/l chloramphenicol. The culture was incubated at 37°C and at OD₆₀₀ = 0.5 the phage library was rescued by infection with VCS M13 helper phage (Stratagene). The bacteria were harvested by centrifugation and resuspended in 2xYT medium containing 30 mg/l kanamycin, 34 mg/l chloramphenicol, 0.1 mM IPTG and grown overnight at 30°C. Phages were precipitated from the culture supernatant by addition of polyethylene glycol PEG-6000 (3.3% final concentration), NaCl (0.4 M final concentration). Phages were resuspended in H₂O, precipitated by addition of polyethylene glycol PEG-6000 (3.3% final concentration), NaCl (0.4 M final concentration) and resuspended in PBS.

After the fourth and fifth round of phage display, pools of selected scFv-encoding sequences were subcloned via restriction sites Xbal and EcoRI into the expression plasmid pMX7 (Knappik et al., JMB, 2000, 296(1), 57-86). E. *coli* SB536 cells were transformed with the constructed vector. Bacteria were grown at 37°C in 2xYT medium containing 0.1% glucose and 34 mg/l chloramphenicol. At OD₆₀₀ = 0.5 cultures were induced with 1 mM IPTG. ScFv fragments are secreted to the periplasm of *E. coli.* For small-scale expressions, cultures were incubated for 5 h after induction at 30°C. For periplasmic extracts, cells were collected by centrifugation and incubated overnight in 300 mM boric acid, 150 mM NaCl, 2 mM EDTA, pH 8, at 4°C. After centrifugation, the supernatant was used for enzyme linked immunosorbent assay (ELISA) screening.

For large-scale expression of scFv fragments, cultures were incubated for 20 h at 22°C. Bacteria were collected by centrifugation and resuspended in 50 mM NaH₂PO₄, 300 mM NaCl, pH 8. After addition of a spatula tip of DNAsel and 2 mM MgCl₂, bacteria were lysed in a French pressure cell. The lysate was filtered and purified on Ni-NTA agarose, washing with 16 column volumes of 50 mM NaH₂PO₄, 300 mM NaCl, pH 8; 12 column volumes of 50 mM NaH₂PO₄, 900 mM NaCl, pH 8; 16 column volumes of 50 mM NaH₂PO₄, 300 mM NaCl, 0.1% Triton X-100, pH 8; and 8 column volumes of 50 mM NaH₂PO₄, 300 mM NaCl, pH 8. Eluates were concentrated by ultra-centrifugation and buffer-exchanged to PBS using Micro BioSpin P-6 columns. For proliferation assays, samples were additionally purified on Detoxi-Gel endotoxin removal columns and eluted with PBS. When stored at 4°C under sterile conditions, purified scFv fragments maintained unchanged binding activity for more than 3 months.

### Bispecific scFv1-linker-scFv2 constructs

Antibody scFv fragments binding to either HER2 domain 1 or HER2 domain 4 were identified by ELISA as described above. From these scFv fragments, a series of bispecific scFv1-linker-scFv2 constructs (bispecific tandem scFv), where always a HER2 domain 1 binder was connected to a HER2 domain 4 binder (in either orientation), was constructed as follows: Since all HuCAL scFv fragments have common internal restriction sites, a vector could be constructed, pHu202, in which the upstream scFv fragment is connected via a flexible linker to the downstream fragment, which does not have a signal sequence, resulting in the arrangement phoA-scFv1-linker-scFv2, where phoA is the secretion signal. The linker segment can be exchanged via unique restriction sites that have been engineered into this fragment at its flanks, NotI and Sfil. Thus, all combinations of potential active bispecific antibodies were conveniently constructed by ligating the linker-scFv2 unit into the secretion vector containing phoA-scFv1, downstream of scFv1. After the active combinations had been identified, the linker was systematically varied in these constructs, by exchanging it into a series of linkers with different length, ligating it via NotI and SfiI.

For large-scale expression of the scFv1-linker-scFv2 fragments, cultures were incubated for 20 h at 22°C. Bacteria were collected by centrifugation and resuspended in 50 mM NaH₂PO₄, 300 mM NaCl, pH 8. After addition of a spatula tip of DNAsel and 2 mM MgCl₂, bacteria were lysed in a French pressure cell. The lysate was filtered and purified on Ni-NTA agarose, washing with 16 column volumes of 50 mM NaH₂PO₄, 300 mM NaCl, pH 8; 12 column volumes of 50 mM NaH₂PO₄, 900 mM NaCl, pH 8; 16 column volumes of 50 mM NaH₂PO₄, 300 mM NaCl, 0.1% Triton X-100, pH 8; and 8 column volumes of 50 mM NaH₂PO₄, 300 mM NaCl, pH 8. Eluates were concentrated by ultra-centrifugation and buffer-exchanged to PBS using Micro BioSpin P-6 columns. For proliferation assays, samples were additionally purified on Detoxi-Gel endotoxin removal columns and eluted with PBS.

### Bispecific diabodies

The cloning of the bispecific diabodies is similar to that of tandem scFvs, but with some important differences. We need to create two genes phoA-VH1-VL2, followed by phoA-VH2-VL1. For simplicity, we opted for two promoters, each driving one of the genes. VH1 and VL1 are the heavy and light chain variable regions of svFv1, and VH2 and VL2 correspondingly of svFv2, but in the diabody arrangement they are now connected to the partner chain of the other scFv. The modularity of the synthetic HuCAL library with its conserved restriction sites within the synthetic genes makes this cloning very convenient. As can be seen, it was only neccessary to exchange VH (or VL) between to scFv fragments, using the unique restriction sites by which VH and VL are flanked in the scFv fragment (Knappik et al., 2000). The whole cassette, promoter-phoA-VH1-linker-VH2 had been flanked by Not1 and Sfil sites in the newly created vectors pDia202, while in pDia203, the same sites had been engineered downstream of the scFv expression cassette. Thus, the complete unit promoter-phoA-VH1-linker-VH2 could be cloned into a vector already containing promoter-phoA-VH2-linker-VH1. Thus, both chains of the diabody were encoded on the same plasmid. Both are secreted to the periplasm where they assemble.

For large-scale expression of the diabodies, cultures were incubated for 20 h at 22°C. Bacteria were collected by centrifugation and resuspended in 50 mM NaH₂PO₄, 300 mM NaCl, pH 8. After addition of a spatula tip of DNAsel and 2 mM MgCl₂, bacteria were lysed in a French pressure cell. The lysate was filtered and purified on Ni-NTA agarose, washing with 16 column volumes of 50 mM NaH₂PO₄, 300 mM NaCl, pH 8; 12 column volumes of 50 mM NaH₂PO₄, 900 mM NaCl, pH 8; 16 column volumes of 50 mM NaH₂PO₄, 300 mM NaCl, 0.1% Triton X-100, pH 8; and 8 column volumes of 50 mM NaH₂PO₄, 300 mM NaCl, pH 8. Eluates were concentrated by ultra-centrifugation and buffer-exchanged to PBS using Micro BioSpin P-6 columns. For proliferation assays, samples were additionally purified on Detoxi-Gel endotoxin removal columns and eluted with PBS.

### Analysis of single binding agents

Binding agents were characterized by means of enzyme-linked immunosorbent assay (ELISA). ELISAs, using the full length extracellular domain of HER2 (ECD HER2) for coating, were carried out to show binding of all individual binding agents. ELISA, using a truncated form of ECD HER2 (domain 1 - 3) as target, were performed to show specific binding of the DARPins to this part of HER2 ECD. This was originally applied to the collection of the 9XX binders (molecules derived from ErbB2_509 selection). Domain 4 binders G3 and H14 were identified by binding to full length ECD HER2 but an absence of binding to the truncated ECD HER2 comprising only domains 1 to 3.

Specific binding experiments were carried out on the surface of viable HER2 overexpressing cancer cells e.g. BT474, SkBr3, SkOv3, using standard flow cytometry methods. Multiple fluorescent detection systems, like e.g. detection of the His-Tag by an anti His-tag antibody, followed by a secondary antibody labeled with Alexa488, or alternatively, genetic superfolder GFP (sfGFP) fusions with the binding molecules or using directly Alexa488-labeled binding reagents, were used to confirm specific binding of all single binding reagents to the surface of HER2 overexpressing cancer cells. The binding to a single epitope was confirmed by the analysis of mean fluorescence intensities, resulting in similar values for all binders at saturation, and more importantly, by complete inhibition of the signal when competed to an unlabeled control binding to said epitope. The single binding reagents also passed different quality control measurements like e.g. size exclusion chromatography, multi-angle light scattering and polyacrylamide gel electrophoresis (PAGE).

### Competitive binding analysis of binding reagents

Competitive binding analysis was performed to characterize the epitopes of the binding agents of the 9XX collection. All binding agents of the 9XX collection compete for binding to a similar epitope on domain 1 of HER2, except binder 901. Competitive binding FACS analysis was also performed with domain 4 binding agents versus trastuzumab. Groups of competing and non-competing binding agents were identified. Importantly, binding to the trastuzumab epitope is not a prerequisite for the anti-tumor activity of the bispecific molecules (G3 does not compete with trastuzumab for binding). Binder H14 does compete with G3 and does show competition with trastuzumab.

Competitive binding FACS analysis performed with the 9XX binding molecules versus pertuzumab binding did not show competition. None of the single binding agent binds to the pertuzumab epitope. ELISA, using the domain 1 of the ECD HER2 as target, was performed to show specific binding of the 9XX collection.

Table 1 summarizes properties of preferential binding units (that can be components bispecific molecules with bioactivity) and control binding units (which do not contribute bioactivity) for the construction of bispecific binding agents with superior anti-tumor activity. Listed are the single domains of the extracellular part of HER2 that are bound by the single agents. The epitope is characterized by inhibition of a binding assay performed in ELISA or on the surface of HER2 overexpressing cancer cells by means of FACS. Crystal structure data are available for the indicated binding agents, which characterize the specific epitopes in detail on the single amino acid level. For the construction of potent bispecific anti-tumor agents, a binding agent which targets domain 1 of HER2 is preferentially fused to a binding agent that targets domain 4 of HER2 from the list of indicated binding agents.

**Table1: Summary of single binding agents**

| | Binds to HER2 domain: | Competitive Binding to HER2 known with: | Crystal Structure available: | Strong anti-tumor activity in bispecific setup: |
|---|---|---|---|---|
| G3 | IV | H14 | YES | YES |
| H14 | IV | G3; 4D5 | - | YES |
| 902 | I | 929;926 | - | YES |
| 903 | I | 929;926 | - | YES |
| 910 | I | 929;926 | - | YES |
| 916 | I | 929;926 | - | YES |
| 926 | I | 929;926 | YES | YES |
| 929 | I | 929;926 | YES | YES |
| 930 | I | 929;926 | - | YES |
| H01 | I | 929;926 | - | YES |
| H03 | I | 929;926 | - | YES |
| Off7 | none | none | YES | - |
| 4D5, trastuzumab | IV | H14,Nanobody,Zybody | YES | YES |
| 2C4, pertuzumab | II | Nanobody,Zybody | YES | - |
| zHER2 | III | none | YES | - |

### Expression of bispecific binding agents

The genes or coding sequences of the bispecific molecules were constructed in a vector pQiBi-01- (or -11-; -12-; -22-; -23-; -33-); using conventional restriction digest and ligation techniques with a BamHI/HindIII restriction site for the N-terminal binding molecules and BglII/BsaI restriction sites for the C-terminal binding molecules. This vector is derived from pQE30 , but encodes the lacl^{q} gene and unique restriction sites (BamHI/HindIII and BglII/BsaI, respectively) to clone one binder upstream, the other downstream of a linker via BamHI/HindIII. The numbers indicate the different linker lengths, where each unit is a (Gly₄Ser) unit. E.g., the pQiBi-22- vector encodes 4 (Gly₄Ser) units between the binders.

Bispecific constructs were expressed in *E*. *coli* strains XL1blue or E. *coli* BL21 using the *lac-*operon induction system by isopropyl-β-D-thiogalactopyranoside (IPTG). Bacteria were lysed by the French press method or by sonification. Filtered bacterial lysates were loaded on NiNTA-agarose bench top columns, washed with TBS_W (50 mM Tris, 400 mM NaCl, 20 mM imidazole, pH 7.5) and in addition washed with 70 CV PBS containing 0.1% Triton X-114 for endotoxin removal. Proteins were eluted in PBS containing 250 mM imidazole. Proteins were further purified by size exclusion chromatography using PBS buffer. Limulus amebocyte lysate (LAL)-assays were performed to assess endotoxin content. Protein concentrations were determined by absorbance spectroscopy at 280 nm and or by a BCA-assay.

### Analysis of bispecific binding reagents

Bispecific binding reagents passed quality control measurements for molecular weight, monomeric status and binding to ECD HER2. Bispecific binding agents comprising trastuzumab-competing binders (in the example, DARPin H14) also compete with trastuzumab in the bispecific setup, as expected. Bispecific binders that do not contain a trastuzumab-competing unit did not show competition in the bispecific setup, also as expected. Competitive binding ELISA, using full length HER2 ECD as target, was performed with all bispecific binding agents also versus pertuzumab. None of the bispecific binding agents competes with pertuzumab for binding to full length ECD HER2 in ELISA. Binding to the surface of viable HER2 overexpressing cancer cells was shown by flow cytometry.

For determination of the anti-tumor activity of the bispecific agents (Fig. 8), BT474 cells were seeded into 96 well plates 16 h before treatment at a density of 10x10⁴ per cm² in RPMI1640 containing 10% FCS. Titrations from 100 pM to 1 µM of each agent (final concentrations) were added and cells were treated for 4 days in a cell culture incubator. XTT viability assays were used according to the manufacturer's protocol to assess the remaining viability of the cancer cells. The targeting agents can be grouped according to their anti-tumor activity. The single binding agents scFv 4D5 and DARPin H14 reduced the cell growth by a similar extent, by 20-30%. Trastuzumab reduced the cell growth by an extent of approx. 50%. The flexible bispecific agents 926-FL-G3 and 929-FL-H14 reduced the cell growth by a similar extent of 80-90%.

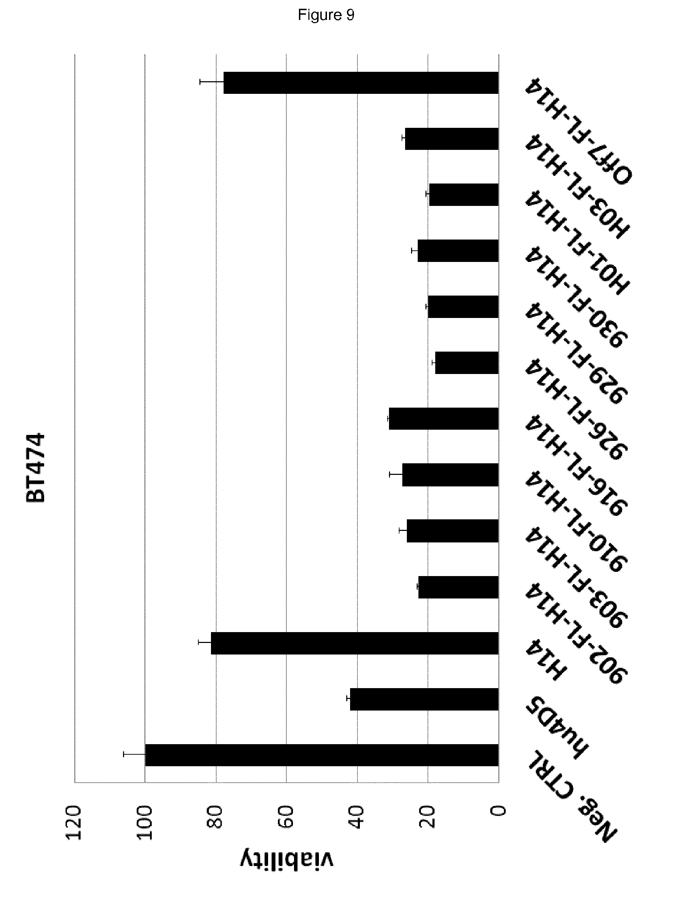

All bispecific constructs that share a similar epitope with e.g. monovalent DARPin 929 on domain 1 of HER2 ECD show strong anti-tumor activity in cell proliferation assays (Fig. 9). BT474 cells were seeded into 96 well plates 16 h before treatment at a density of 10⁴ per cm² in RPMI1640 containing 10% FCS. Anti-HER2 binding agents were added to a concentration of 100 nM (final concentration), and cells were treated for 4 days. XTT cell proliferation assays were developed according to the manufacturer's protocol. All bispecfic agents containing 9XX at the N-terminus, which showed competitive binding with 926 and 929 in ELISA to ECD HER2, reduced the viability of the cancer cells by 70-80%, i.e. to a higher extent than trastuzumab.

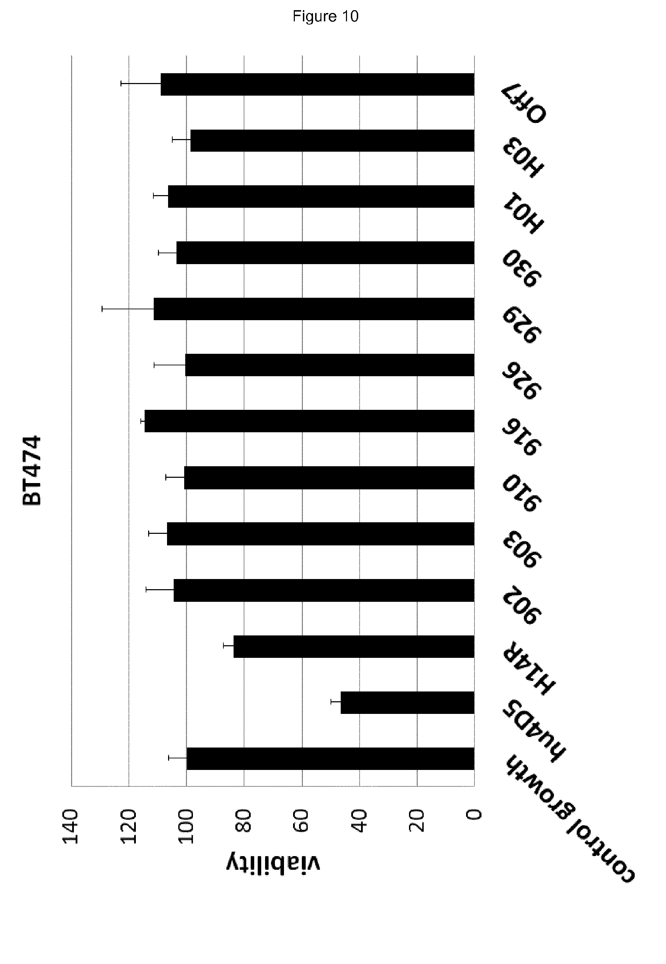

For determination of the anti-tumor activity of single binding agents, BT474 cells were seeded into 96 well plates 16 h before treatment at a density of 10⁴ per cm² in RPMI1640 containing 10% FCS. Anti-HER2 binding agents were added to a concentration of 100 nM, and cells were treated for 4 days. XTT cell proliferation assays were developed according to the manufacturer's protocol. H14, the HER2 domain 4 binding agents which competes for binding with trastuzumab (hu4D5), reduces tumor growth by 20%. The 9XX domain 1 binding agents do not show any anti-tumor activity as single binding agents (Fig. 10).

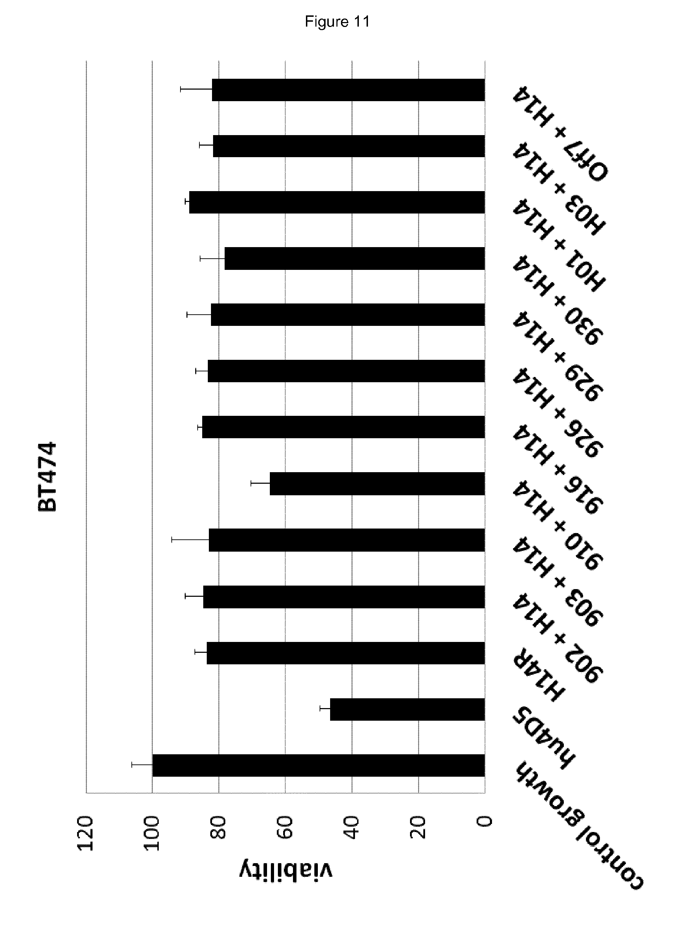

The combination treatment of the single anti-HER2 binding agents is shown in Fig. 11. BT474 cells were seeded into 96 well plates 16 h before treatment at a density of 10⁴ per cm² in RPMI1640 containing 10% FCS. Anti-HER2 binding agents were added to a concentration of 100 nM, and cells were treated for 4 days. XTT cell proliferation assays were developed according to the manufacturer's protocol. The 9XX domain 4 binding agents do not show an additive effect to anti-tumor activity of H14. Thus, the strong anti-tumor activity requires that the binding agents are connected into a bispecific molecule.

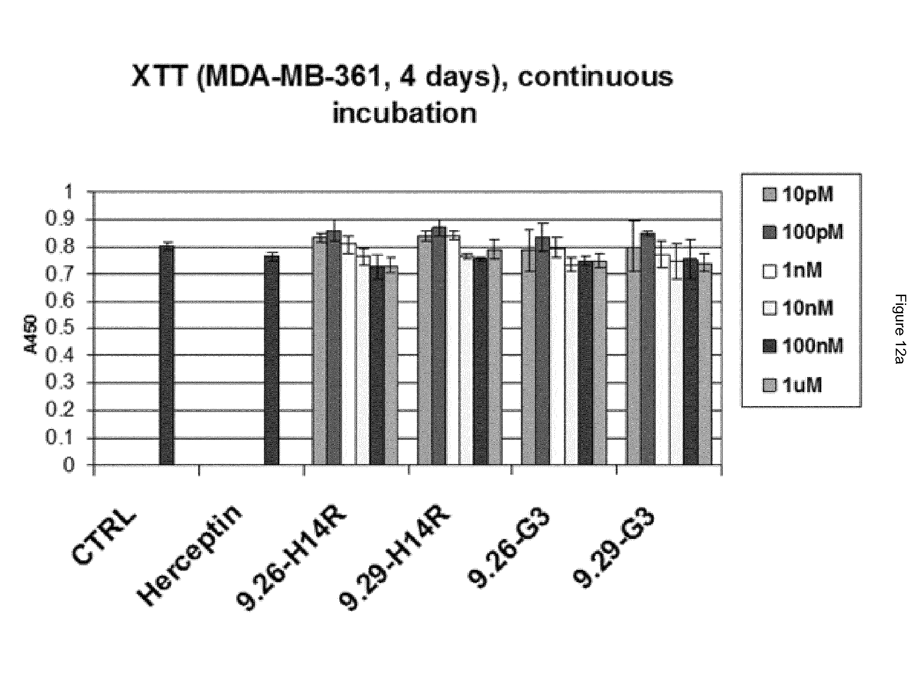

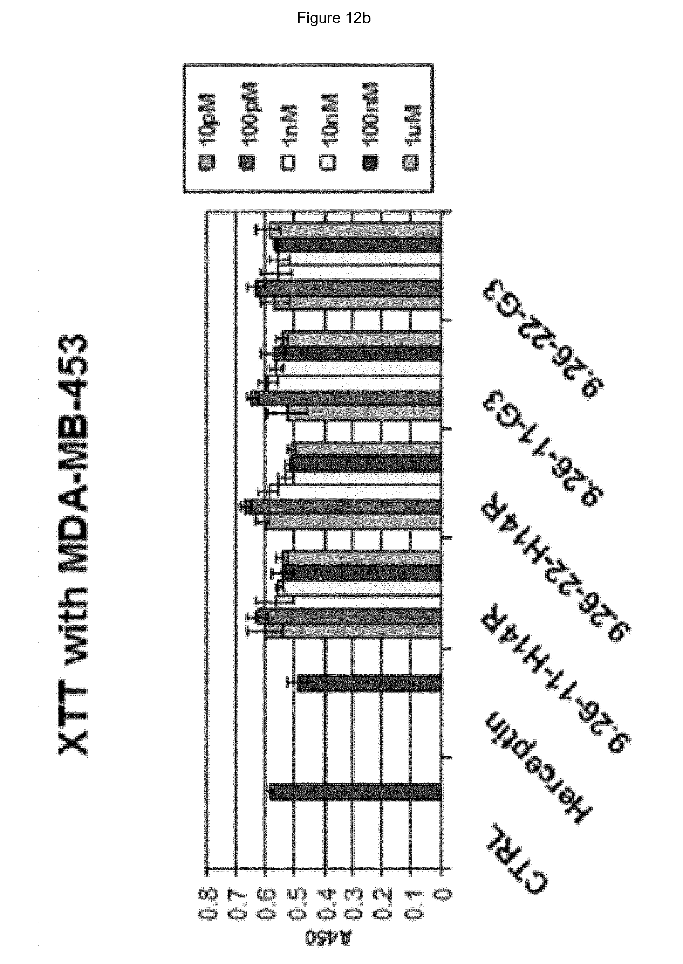

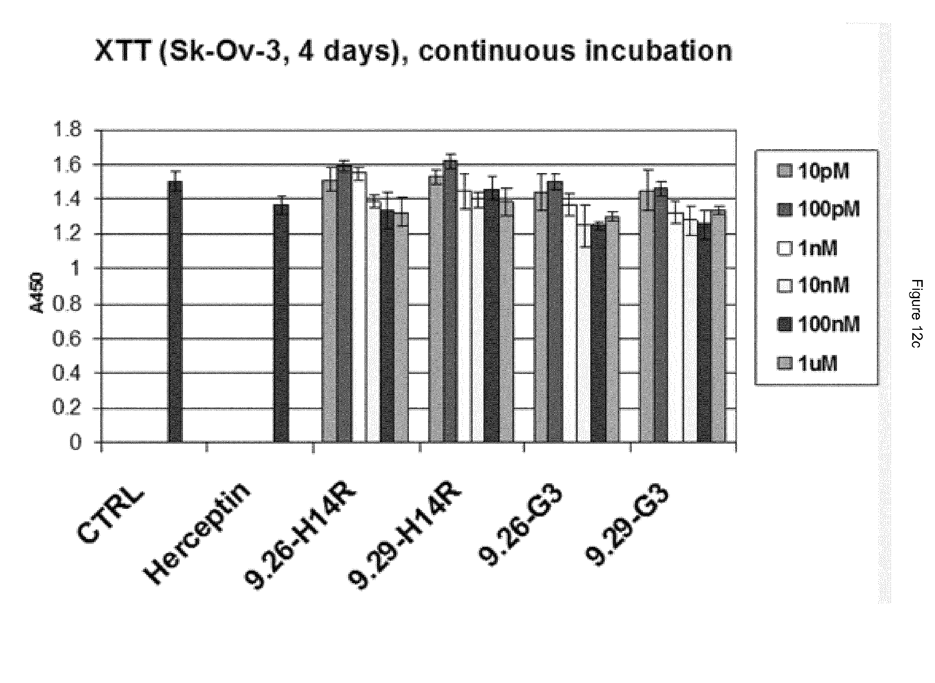

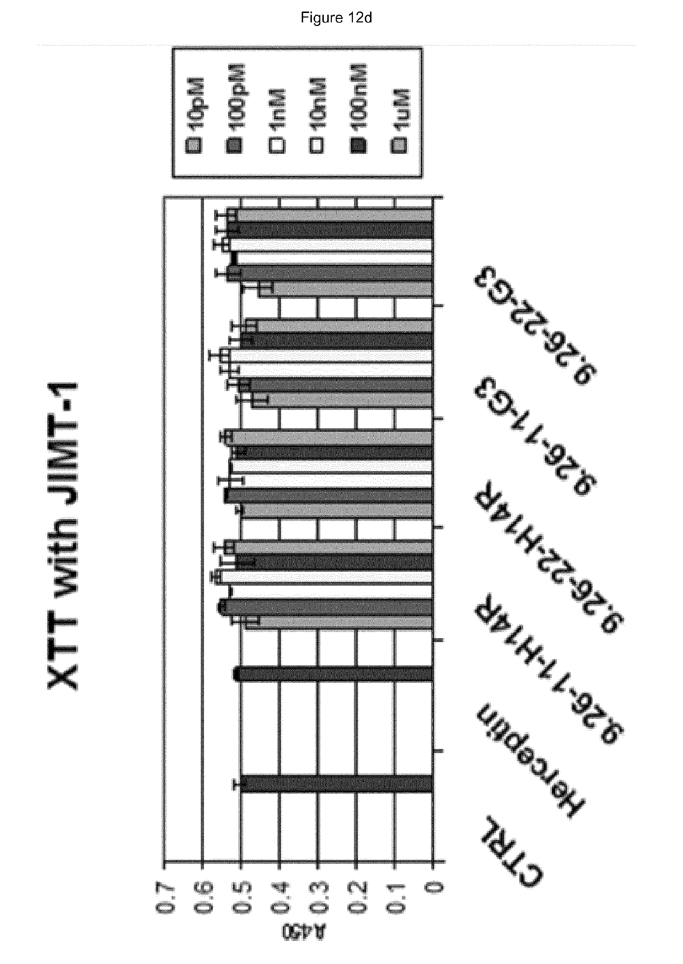

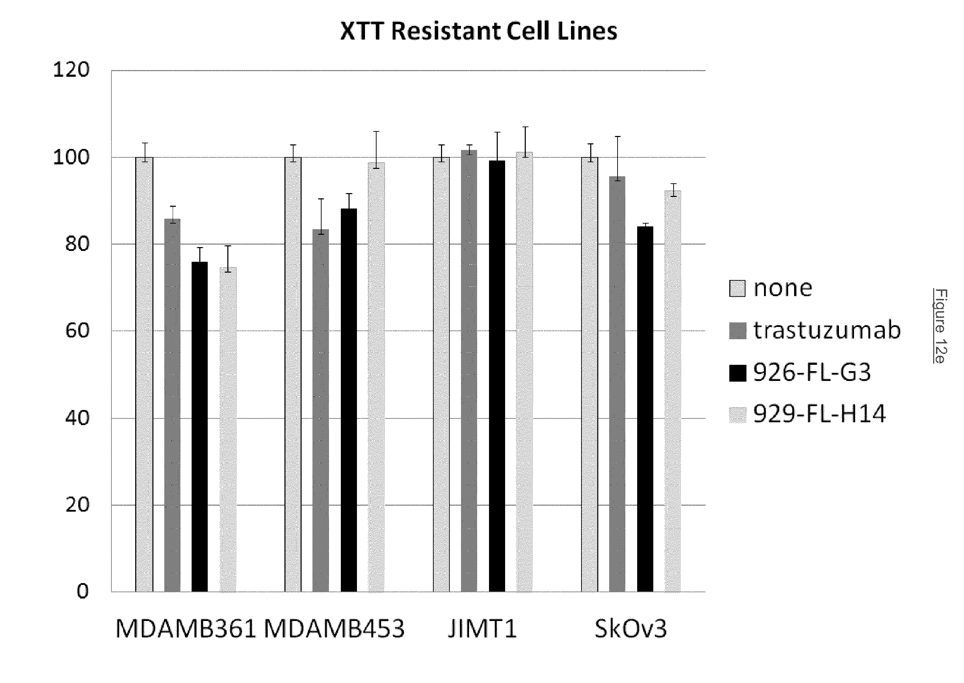

Cell proliferation assays with trastuzumab-resistant cell lines are shown in Fig. 12. Cancer cells were seeded into 96 well plates 16 h before treatment. A serial dilution of anti-HER2 binding agents was added and cells were treated for 4 days. XTT cell proliferation assays were developed according to the manufacturer's protocol. The anti-tumor activity of bispecific targeting agents is similarly modest to trastuzumab in trastuzumab-resistant cell lines.

### Example 3: Differentiation from prior art constructs: Comparison of apoptosis induced by 7C2 in combination with 4D5 versus bispecific targeting agents

As was demonstrated in the patent (US 7,371,376 B1; US20110033460 (A1) ANTI-ErbB2 ANTIBODIES), the antibody 7C2 is competent as a single agent to induce apoptosis in the following cell lines BT474, SkBr3, SkOv3 or Calu-3. The epitopes on domain 1 of the ECD HER2 bound by 7C2 and 7F3 are different from the epitopes bound by the 9XX collection (see below). The bispecific targeting agents disclosed here induce apoptosis in BT474 and SkBr3 cells, but not in SkOv3 cells. The absence of anti-tumor activity in SkOv3 cells can be explained by the activating mutation H1047R of the PI3-Kinase. The induction of apoptosis by the bispecific targeting agents is thus correlated with a non-mutated, wild-type downstream signaling pathway of HER2 and HER3.

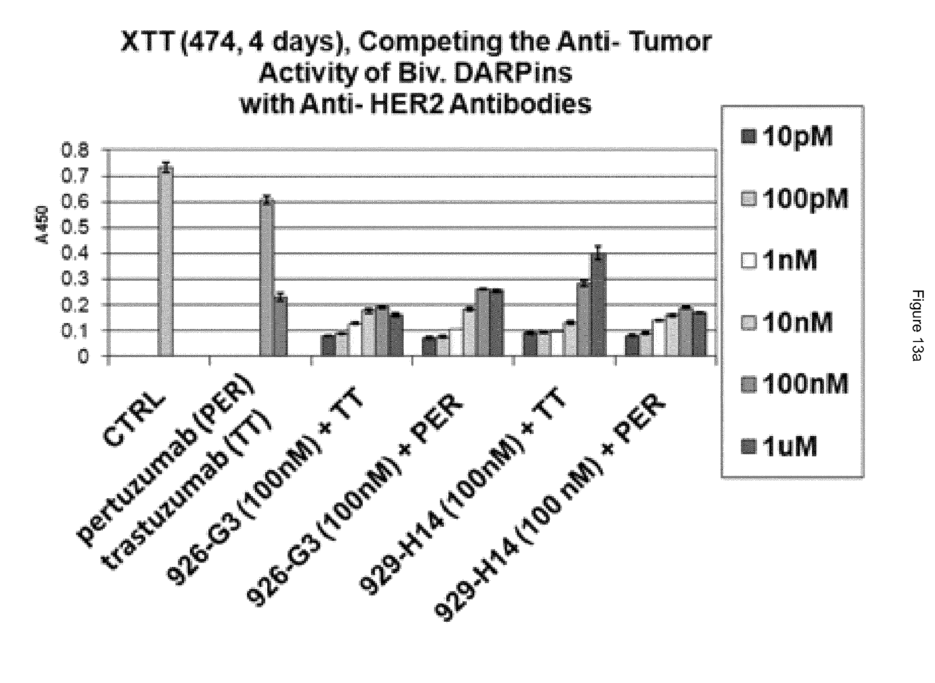

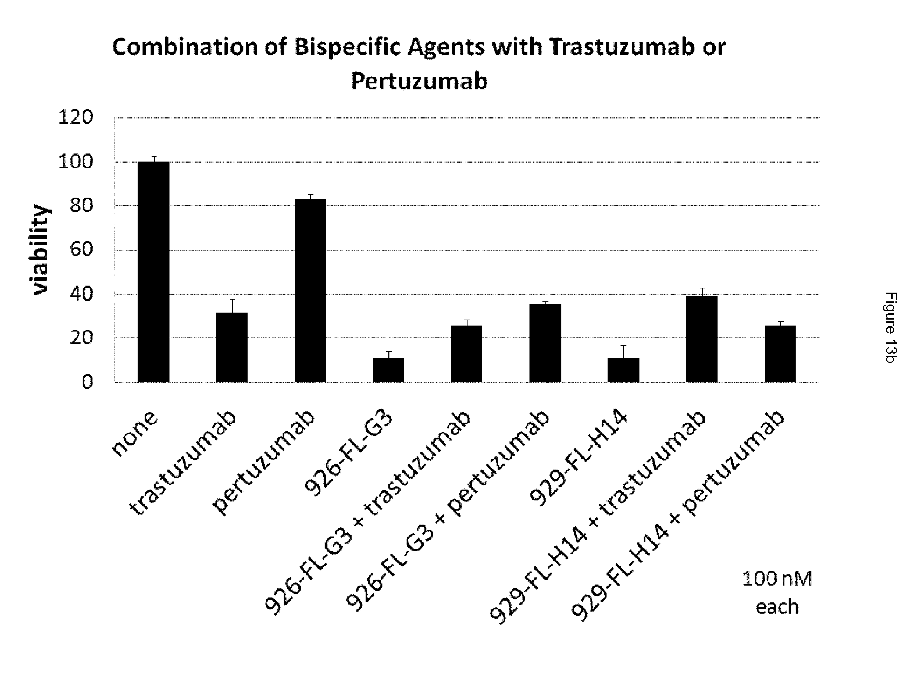

The absence of anti-tumor activity is another difference to the antibodies 7C2 and 7F3, which show anti-tumor activity as single agents. In the patent US20110033460A1 an additive effect of 7C2 and 4D5 (trastuzumab) to anti-tumor activity was shown. In contrast, the anti-tumor activities of bispecific targeting agents disclosed here are significantly reduced in combination with trastuzumab (Fig. 13).

In the case of H14 fusions this can be explained by simple competition for binding to the same epitope, while in the case of G3 fusions, trastuzumab and G3 do not compete for binding to domain 4. Hence, trastuzumab blocks the formation of inactive HER2 homodimers that are induced by the bispecific molecules according to the invention. Therefore, the modes of action of 7C2 in combination with 4D5, in comparison to the bispecific targeting agents according to our invention, are different. Furthermore, the concept for induction of apoptosis in HER2 overexpressing cancer cells is completely different. Here it is shown that through the strong inhibition of the internal cell signalling in these HER2-dependent cancer cells, apoptosis is induced by the bispecific binding molecules. In contrast, 7C2 is shown to induce apoptosis but not inhibition of cell growth. This mode of action uncouples signalling from apoptosis and is therefore more similar to e.g. death receptor signaling (FAS or TNF receptor).

The antibodies trastuzumab (TT, 4D5) and pertuzumab (PER, 2C4) disrupt the inactive HER2 homodimers formed by bispecific targeting agents (Fig. 13). BT474 cells were seeded into 96 well plates more than 16 h before treatment at a density of 10⁴ per cm² in RPMI1640 containing 10% FCS. The bispecific targeting agents 926-G3 and 929-H14 were added at a concentration of 100 nM. Subsequently, titration from 10 pM to 1 µM of an anti-HER2 antibody, either trastuzumab (TT, 4D5) or pertuzumab (PER, 2C4), was added. BT474 cells were treated for 4 days in a cell culture incubator at 37°C and 5% CO₂. XTT cell viability assays were performed according to manufacturer's protocol. The absorbance at 450 nm correlates with the number of viable cells. By increasing concentrations of trastuzumab or pertuzumab in the presence of the bispecific agents 926-G3 and 929-H14, the antitumor activity of the bispecific targeting agents is significantly reduced. This indicates that the anti-tumor effect of the bispecific molecules according to this invention is greater than that of trastuzumab or pertuzumab.

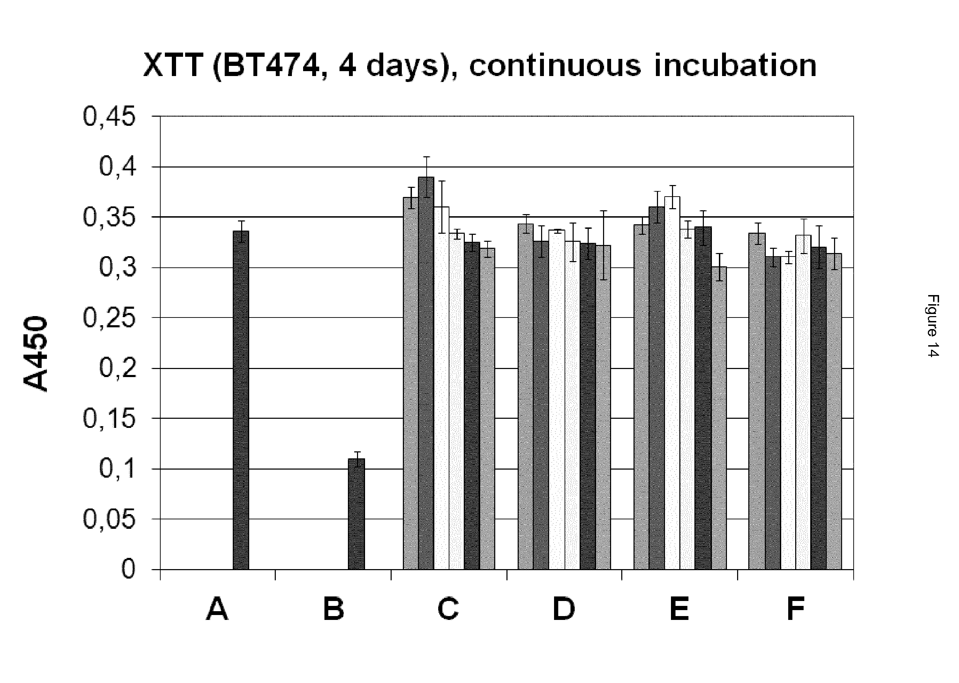

The anti-tumor activity of bispecific targeting agents is not caused by random cross-linking of receptors (Fig. 14; A-control; C - 926-22-926/H14R-22-H14R; D - 926AvantE-22-926AventE/H14R-22-H14R; E - 926AvantE-22-926AventE/H14AvantE-22-H14AventE; F - 926-22-926/G3-22-G3; first column=10 pM, second column=100 pM, third column=1 nM, forth column=10 nM, fifth column=100 nM and sixth column=1 µM of C, D, E and F, respectively). BT474 cells were seeded into 96 well plates more than 16 h before treatment at a density of 10⁴ per cm² in RPMI1640 containing 10% FCS. Combinations of homo-bivalent targeting agents were titrated from 10 pM to 1 µM. The combination of both homo-bivalent targeting agents did not show any signification reduction in the viability of the cancer cells.

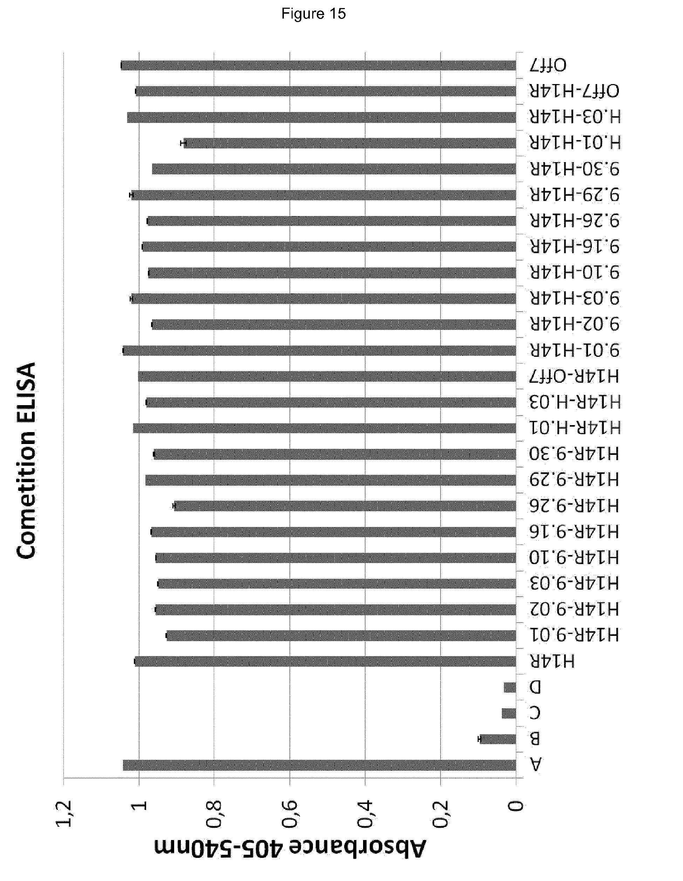

Bispecific targeting agents do not compete for binding with pertuzumab in ELISA (Fig. 15, A-pertuzumab, 2ndAb (no competitor), B-2nd Ab, C-pertuzumab, 2nd Ab (no ErbB2), D-2nd Ab (no ErbB2)). Wells of the MaxiSorp plate were coated with 100 µl PBS containing 66 nM streptavidin for 12 hours at 4°C. Liquids were removed completely after each step. The plastic surface was blocked by PBS_TB (PBS containing 0.1% Tween20, 0.2% BSA) for 1 hour at room temperature with continuous shaking. Afterwards, 20 nM of truncated ErbB2-avidin conjugate was added in 100 µl PBS_TB and incubated for 1 hour. The plate was washed four times with PBS_TB. Then, bivalent DARPins were added to 1 µM in PBS_TB, and binding took place for 3 hours on a shaker. Next, 1 nM of pertuzumab was added and incubated for 30 min. The plate was washed four times in PBS_TB. The secondary antihuman antibody coupled to alkaline phosphatase was incubated in 100 µl PBS_TB for 1 hour. The plate was washed four times with PBS_TB. Finally, 100 µl of freshly prepared and filtered pNPP buffer (3 mM pNPP, 50 mM NaHCO₃, 50 mM MgCl₂) was added and the color reaction was developed for 5 min at room temperature. Absorbance was detected on an ELISA plate reader at the wavelength of 405 nm.

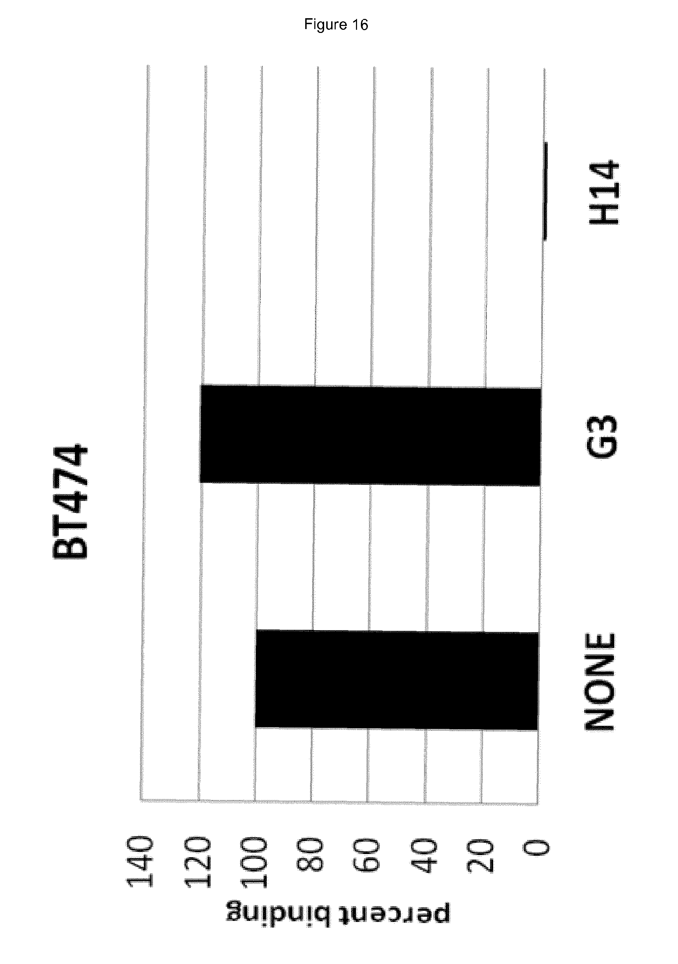

Analysis of competitive binding to domain 4 of HER2 was measured by flow cytometry (Fig. 16). 10⁵ BT474 cells were incubated with either 1 µM of G3 or H14 for 30 min at room in 100 µl PBS_BA (PBS, 0.2% NaN₃, 1% BSA). Subsequently, Alexa₄₈₈-trastuzumab, which had been labeled with Alexa₄₈₈-succinimidyl ester, was added to a concentration of 100 nM and incubated for 30 min at room temperature. Afterwards, cells were washed twice using PBS_BA. Flow cytometry measurements were performed on a Cyflow space system. 10⁴ events were recorded in a FSC/SSC gate to measure cells with proper size. Mean fluorescence intensities were calculated by FlowJo software and data were normalized to the MFI of Alexa₄₈₈-trastuzumab binding. G3 does not compete with the binding of trastuzumab, while H14 and trastuzumab bind to a very similar epitope and therefore show 100 % competition for binding.

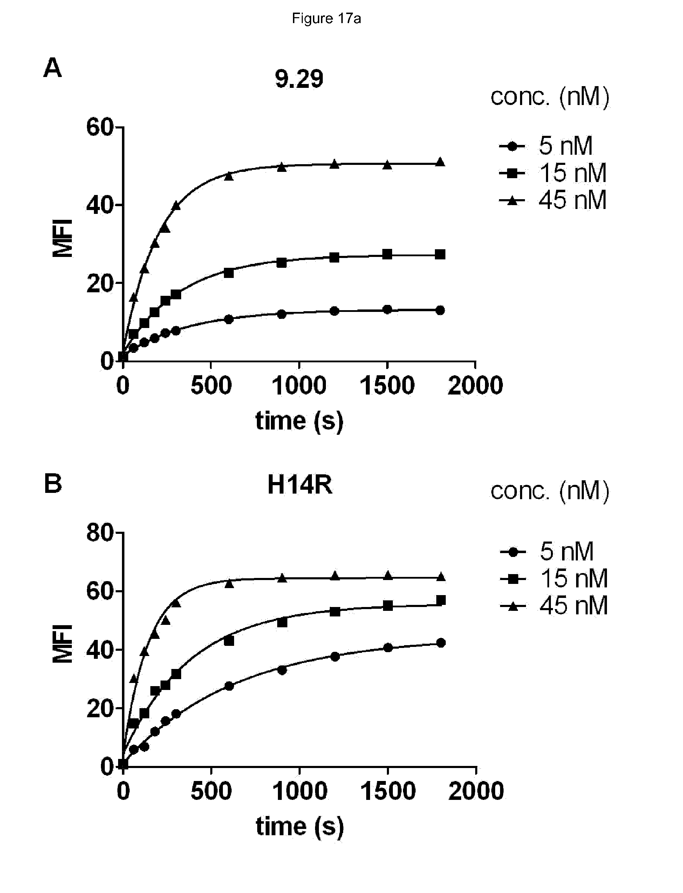

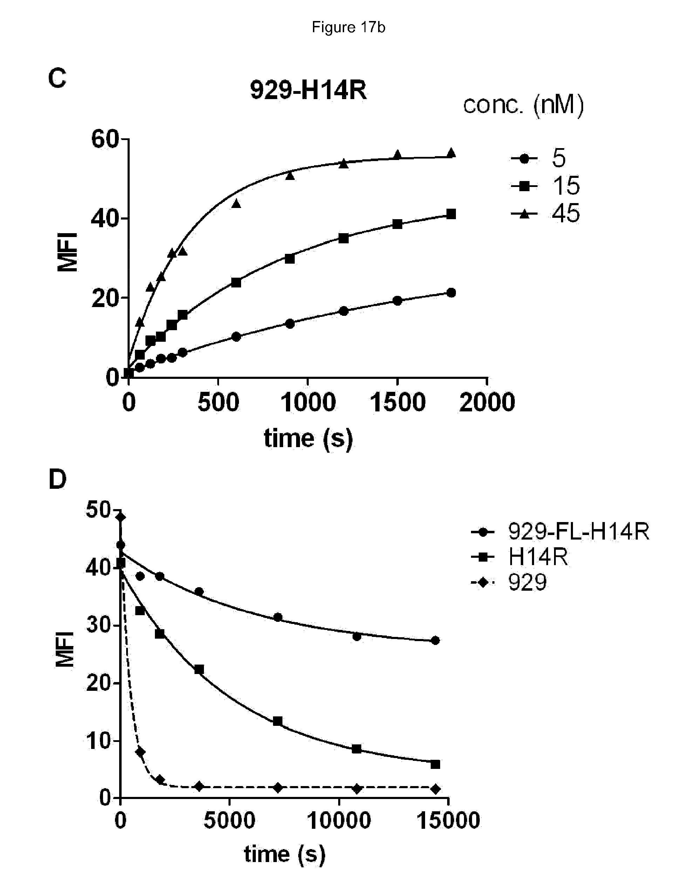

Bivalent binding of the bispecific targeting agent to HER2 at the surface of cancer cells is a prerequisite for strong anti-tumor activity. To confirm the binding of bispecific agents, the association rate constant kₒₙ and dissociation rate constant k_{off} on intact cells can be measured by flow cytometry (Fig. 17) (Tamaskovic et al. (2012) Methods Enzymol. 503, 101-134).

The following tables show the determined binding affinities of single and bispecific binding agents and certain DARPins.

| | average | average | average | average |
|---|---|---|---|---|
| | Kₒₙ(M⁻¹s⁻¹) | Kobs (S⁻¹) | K_{off}(s⁻¹) | K_{d} (M) |
| 929 | 68977 | 0.0035 | 2.21 x 10⁻³ | 33.47 x 10⁻⁹ |
| H14 | 196244 | 0.0037 | 1.79 x 10⁻⁴ | 0.97 x 10⁻⁹ |
| 929-FL-H14 | 77959 | 0.0015 | 3.99 x 10⁻⁵ | 0.52 x 10⁻⁹ |

| **DARPin** | **K_{D}(nM)** | **Kₒₙ(10⁵M⁻¹s⁻¹)** | **K_{off}(10⁻³s⁻¹)** |
|---|---|---|---|
| 916 (domain 1 binder) | 6.9 | 1.2 | 0.9 |
| 926(domain 1 binder) | 1.4 | 0.7 | 0.1 |
| 929 (domain 1 binder) | 3.8 | 2.0 | 0.8 |
| H14 (domain 4 binder) | 0.2 | 4.1 | 0.1 |

### Preparation of cancer cells for flow cytometry measurements

Cells were detached by collagenase and EDTA for 5 min at 37° C. The solution was quenched by addition of medium and centrifuged at 300 g for 3 min. Cells were washed twice in warm PBS. Cell densities were determined with a CASY cell analyzer and adjusted to 10⁶ cells per sample. Internalization was blocked by incubation in PBS containing 0.2% NaN₃ and 1 % BSA for 30 min at 37°C.

### Flow cytometry measurements

Samples were resuspended in 1 ml cold PBS and measured on flow cytometer. 10,000 cells per sample were recorded. Results were gated for FSC vs SSC of the cells. Green fluorescence was detected with the FL1 detector. Data were processed by the FlowJo 7.2.5 software.

### Measuring association of binding agents on the surface of cancer cells

For on-rate determinations, BT474 cells are incubated at a concentration of 1x10⁶cells/ml with 2.5, 7.5, and 22.5nM DARPin-Alexa Fluor-488 conjugates in PBSBA at room temperature for defined time intervals, ranging from 1 to 60min. For each time point, a 1 ml aliquot of cells is withdrawn and subjected to FACS._Since the applied concentrations of the labeled ligand conjugates are very low, and since the time resolution of the measurement is to be maintained to ensure the accuracy of the on-rate determination, the samples are processed without further washing. For each time point, at least 10⁴ intact cells (gated as a uniform population on a FSC/SSC scatter plot) are counted, and the MFI (mean fluorescence intensity) is recorded.

### Measuring dissociation of binding agents on the surface of cancer cells

10⁶ cells per time point were incubated with 1 µM Alexa488 labeled binding agents in 100 µl PBS (0.2% NaN₃, 1% BSA) for 1 hour at 4°C on the shaker. Corresponding to 100 µl cell suspension, samples were washed twice in 1 ml PBS (0.2% NaN₃, 1% BSA) and centrifuged at 600 g for 30 sec at room temperature. Cells were resuspended in 1 ml PBS (0.2% NaN₃, 1% BSA) containing 100 nM of equivalent unlabeled binding agent. The dissociation reaction was incubated for the indicated times (15, 30, 60, 120, 180 and 240 min) at room temperature while continuously stirring in the dark. Dissociation was stopped by placing the cell pellets on ice. Each sample was washed once with 1 ml cold PBS.

## Claims

1. A bispecific HER2-targeting agent comprising
a. a first polypeptide ligand that binds to HER2 extracellular domain 1,
b. a second polypeptide ligand that binds to HER2 extracellular domain 4 and
c. a linker covalently attaching said first polypeptide ligand to said second polypeptide ligand.

2. A bispecific HER2-targeting agent according to claim 1, wherein said first polypeptide ligand and/or said second polypeptide ligand is selected from
a. an immunoglobulin Fab fragment,
b. an immunoglobulin scFv fragment,
c. an immunoglobulin variable domain,
d. a humanized camelid antibody,
e. a polypeptide derived from protein A domains,
f. a polypeptide derived from fibronectin domain FN3,
g. a polypeptide derived from consensus fibronectin domains,
h. a polypeptide derived from lipocalins,
i. a polypeptide derived from armadillo repeat proteins,
j. a polypeptide derived from tetratricopeptide repeat proteins,
k. a polypeptide derived from leucine-rich repeat proteins,
l. a polypeptide derived from Zinc fingers,
m. a polypeptide derived from Src homology domain 2 (SH2),
n. a polypeptide derived from Src homology domain 3 (SH3),
o. a polypeptide derived from PDZ domains,
p. a polypeptide derived from gamma-crystallin,
q. a polypeptide derived from ubiquitin,
r. a polypeptide derived from a cysteine knot polypeptide,
s. a polypeptide derived from a knottin and
t. a peptide selected from a random peptide library to bind to domain 1 or domain 4 of HER2.

3. A bispecific HER2-targeting agent according to claim 1, wherein said agent is a bispecific antibody selected from
a. an IgG comprising a first Fab fragment binding to domain 1 of HER2 and a second Fab fragment binding to domain 4 of HER2,
b. an IgG comprising a VH domain binding to domain 1 of HER2 and VL domain binding to domain 4 of HER2,
c. an IgG comprising a VH domain binding to domain 4 of HER2 and VL domain binding to domain 1 of HER2,
d. a construct comprising a first scFv fragment binding to domain 1 of HER2, a second scFv fragment binding to domain 4 of HER2 and a linker connecting said first scFv fragment and said second scFV fragment,
e. a diabody comprising a first binding site binding to domain 1 of HER2 and a second binding site binding to domain 4 of HER2,
f. an IgG targeting HER2 domain 4 connected to a polypeptide ligand selected from the list of ligands recited in claim 2 targeting domain 1 of HER2 or to a peptide ligand of 5 to 35 amino acids selected from a peptide library to bind to domain 1 of HER2, wherein said polypeptide ligand or said peptide ligand is connected to
i. the N-terminus of a heavy chain of said IgG,
ii. the C-terminus of a heavy chain of said IgG,
iii. the N-terminus of a light chain of said IgG or
iv. the C-terminus of a light chain of said IgG,
g. an IgG targeting HER2 domain 1 connected a polypeptide ligand selected from the list recited in claim 2 targeting domain 4 of HER2 or to a peptide ligand of 5 to 35 amino acids selected from a peptide library to bind to domain 1 of HER2, wherein said polypeptide ligand or said peptide ligand is connected to
i. the N-terminus of a heavy chain of said IgG,
ii. the C-terminus of a heavy chain of said IgG,
iii. the N-terminus of a light chain of said IgG or
iv. the C-terminus of a light chain of said IgG.

4. A bispecific HER2-targeting agent according to claim 1, wherein said first polypeptide ligand and/or said second polypeptide ligand is an ankyrin repeat based polypeptide.

5. A bispecific HER2-targeting agent according to claim 4, wherein said first polypeptide ligand comprises or is a sequence selected from the group composed of SEQ ID 10, SEQ ID 11, SEQ ID 12, SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 30, SEQ ID 31, SEQ ID 32, SEQ ID 33, SEQ ID 34, SEQ ID 35, SEQ ID 36, SEQ ID 37, SEQ ID 38, SEQ ID 39, SEQ ID 40, SEQ ID 41, SEQ ID 42, SEQ ID 43, SEQ ID 44, SEQ ID 45, SEQ ID 46, SEQ ID 47, SEQ ID 48, SEQ ID 49 and SEQ ID 50.
and/or
said second polypeptide ligand comprises or is a sequence selected from the group composed of SEQ ID 25, SEQ ID 26, SEQ ID 27, SEQ ID 28 and SEQ ID 29.

6. A bispecific HER2-targeting agent according to any of the previous claims, wherein said first polypeptide ligand and said second polypeptide ligand are attached to each other by an oligopeptide linker, said first polypeptide ligand, said second polypeptide ligand and said linker forming one continuous polypeptide chain.

7. A bispecific HER2-targeting agent according to claim 6, wherein said first polypeptide sequence is located at the N-terminus of said continuous polypeptide chain, said second polypeptide sequence is located at the C-terminus of said continuous polypeptide chain, and said linker is located between said first and said second polypeptide ligand.

8. A bispecific HER2-targeting agent according to any of claims 1 to 5, wherein said first polypeptide ligand and said second polypeptide ligand are covalently attached to each other by a crosslinker.

9. A bispecific HER2-targeting agent according to any of the previous claims, wherein
a. said first polypeptide ligand partially or fully interacts non-covalently with
i. a first D1 epitope, wherein said first D1 epitope comprises the amino acid residues E87, N89, Y90, L132, R135, D143, I145, W147, K148, L157, A158, L159, T160, L161 and I162 comprised within the amino acid sequence of HER2,
ii. a second D1 epitope, wherein said second D1 epitope comprises the amino acid residues D88, A93, V94, 1133, Q134, Q142, T144, L146, F151, H152, K153, N154, Q156 and D163 comprised within the amino acid sequence of HER2,
iii. a third D1 epitope **characterized by** Seq. ID 55, or
iv. a D1 epitope of domain 1 of HER22 (SEQ ID 01), wherein binding to said D1 epitope is competed by a polypeptide selected from SEQ ID 10, SEQ ID 11, SEQ ID 12, SEQ ID 13, SEQ ID 14, SEQ ID 15, SEQ ID 16, SEQ ID 17 SEQ ID 18, SEQ ID 19, SEQ ID 20, SEQ ID 21, SEQ ID 22, SEQ ID 23, SEQ ID 24, SEQ ID 30, SEQ ID 31, SEQ ID 32, SEQ ID 33, SEQ ID 34, SEQ ID 35, SEQ ID 36, SEQ ID 37, SEQ ID 38, SEQ ID 39, SEQ ID 40, SEQ ID 41, SEQ ID 42, SEQ ID 43, SEQ ID 44, SEQ ID 45, SEQ ID 46, SEQ ID 47, SEQ ID 48, SEQ ID 49 and SEQ ID 50
and/ or
b. said second polypeptide ligand partially or fully interacts non-covalently with
i. a first D4 epitope, wherein said first D4 epitope comprises the amino acid residues F512, E521, V524, L525, Q526, Y532, V533, N534, A535, R536, D549, G550, S551, V552, C554, F555 and V563 comprised within the amino acid sequence of HER2,
ii. a second D4 epitope, wherein said second D4 epitope comprises the amino acid residues C522, R523, T553, C562 and A564 comprised within the amino acid sequence of HER2,
iii. a third D4 epitope **characterized by** Seq. ID 56,
iv. a forth D4 epitope **characterized by** Seq. ID 57, or
v. a D4 epitope of domain 4 of HER2 (SEQ ID 02), wherein binding to said D4 epitope is competed by a polypeptide having a sequence selected from SEQ ID 25, SEQ ID 26, SEQ ID 27, SEQ ID 28 and SEQ ID 29.

10. A bispecific HER2-targeting agent according to any of claims 4 to 9, wherein
a) said first polypeptide ligand is an ankyrin repeat based polypeptide and said second polypeptide ligand is an antibody, an antibody fragment, an antibody variable domain or a polypeptide ligand selected from the list recited in claim 2,
b) said first polypeptide ligand is an antibody, antibody fragment, an antibody variable domain or a polypeptide ligand selected from the list recited in claim 2 and said second polypeptide ligand is an ankyrin repeat based polypeptide,
c) said first polypeptide ligand is an antibody, an antibody fragment or an antibody variable domain and said second polypeptide ligand is a polypeptide ligand selected from the list recited in claim 2, or
d) said second polypeptide ligand is an antibody, an antibody fragment, an antibody variable domain and said first polypeptide ligand is a polypeptide ligand selected from the list recited in claim 2.

11. A bispecific HER2-targeting agent according to any of the previous claims, wherein said linker has a length of equal or less than 65 Å, 60 Å, 55 Å, 50 Å, 45 Å, 40 Å, 35 Å, 30 Å, 25 Å, 20 Å, 15 Å, 10 Å or 5 Å and/or
said linker consists of 1, 2, 3, 4, 5, 6, 7, 8, 9, 10, 11, 12, 13, 14, 15, 16, 17, 18, 19, 20, 21, 22, 23, 24, or 25 amino acids.

12. A bispecific HER2-targeting agent according to any of the previous claims, wherein
a. said first polypeptide ligand contacts HER2 extracellular domain 1 through a D1 binding site;
b. said second polypeptide ligand contacts HER2 extracellular domain 4 through a D4 binding site;
c. and said linker is selected to allow a spatial separation between said D1 binding site and said D4 binding site of less than 75 Å, 70 Å, 65 Å, 60 Å, 55 Å, 50 Å, 45 Å, 40 Å, 35 Å, 30 Å, 25 Å, 20 Å, 15 Å, 10 Å or 5 Å.

13. A bispecific HER2 agent according to any of the previous claims, wherein said linker is a polyglycine/serine linker, particularly a linker **characterized by** an amino acid sequence (GGGGS)ₙ, with n being 1, 2, 3, 4 or 5.

14. A bispecific HER2-targeting agent according to any of the previous claims, which
a. leads to a reduction of viability of a cells selected from AU565, BT474 HCC1419, HCC2218, SkBr3 and/or ZR7530, said reduction being higher than the reduction achieved by a similar treatment with trastuzumab,
b. leads to a signal reduction of HER2-Y1248, HER3-Y1289, AKT-S473, ERK1/2-T202/Y204 and/or PARP in a Western blot when incubated with cells of the A565 cell line,
c. leads to a signal reduction of HER2-Y1248, HER3-Y1289, AKT-S473 and/or ERK1/2-T202/Y204 in a Western blot when incubated with the HCC1419 cell line,
d. leads to a signal reduction of HER2-Y1248, HER3-Y1289, AKT-S473 and/or ERK1/2-T202/Y204 in a Western blot when incubated with the HCC2218 cell line,
e. leads to a signal reduction of HER2-Y1248, HER3-Y1289, AKT-S473, ERK1/2-T202/Y204 and/or PARP in a Western blot when incubated with the ZR7530 cell line, or
f. leads to an induction of apoptosis in at least 40% of BT474 cells, in at least 8% of AU565 cells, in at least 20% of HCC1419 cells an/or in at least 20% of HCC2218 cells when incubated with the indicated cell line.

15. A bispecific HER2 agent, wherein said bispecific agent is **characterized by** an amino acid sequence selected from SEQ ID 03, SEQ ID 04, SEQ ID 05, SEQ ID 06, SEQ ID 07, SEQ ID 08 and SEQ ID 09.


SEQUENCE LISTING

<110>  Universität Zürich

<120>  Bispecific dimerization ligands for cancer therapy

<130>  uz167ep-2

<160>  57

<170>  PatentIn version 3.5

<210>  1
<211>  197
<212>  PRT
<213>  Homo sapiens

<400>  1

Gln Val Cys Thr Gly Thr Asp Met Lys Leu Arg Leu Pro Ala Ser Pro
1               5                   10                  15

Glu Thr His Leu Asp Met Leu Arg His Leu Tyr Gln Gly Cys Gln Val
            20                  25                  30

Val Gln Gly Asn Leu Glu Leu Thr Tyr Leu Pro Thr Asn Ala Ser Leu
        35                  40                  45

Ser Phe Leu Gln Asp Ile Gln Glu Val Gln Gly Tyr Val Leu Ile Ala
    50                  55                  60

His Asn Gln Val Arg Gln Val Pro Leu Gln Arg Leu Arg Ile Val Arg
65                  70                  75                  80

Gly Thr Gln Leu Phe Glu Asp Asn Tyr Ala Leu Ala Val Leu Asp Asn
                85                  90                  95

Gly Asp Pro Leu Asn Asn Thr Thr Pro Val Thr Gly Ala Ser Pro Gly
            100                 105                 110

Gly Leu Arg Glu Leu Gln Leu Arg Ser Leu Thr Glu Ile Leu Lys Gly
        115                 120                 125

Gly Val Leu Ile Gln Arg Asn Pro Gln Leu Cys Tyr Gln Asp Thr Ile
    130                 135                 140

Leu Trp Lys Asp Ile Phe His Lys Asn Asn Gln Leu Ala Leu Thr Leu
145                 150                 155                 160

Ile Asp Thr Asn Arg Ser Arg Ala Cys His Pro Cys Ser Pro Met Cys
                165                 170                 175

Lys Gly Ser Arg Cys Trp Gly Glu Ser Ser Glu Asp Cys Gln Ser Leu
            180                 185                 190

Thr Arg Thr Val Ala
        195

<210>  2
<211>  97
<212>  PRT
<213>  Homo sapiens

<400>  2

Val Asn Cys Ser Gln Phe Leu Arg Gly Gln Glu Cys Val Glu Glu Cys
1               5                   10                  15

Arg Val Leu Gln Gly Leu Pro Arg Glu Tyr Val Asn Ala Arg His Cys
            20                  25                  30

Leu Pro Cys His Pro Glu Cys Gln Pro Gln Asn Gly Ser Val Thr Cys
        35                  40                  45

Phe Gly Pro Glu Ala Asp Gln Cys Val Ala Cys Ala His Tyr Lys Asp
    50                  55                  60

Pro Pro Phe Cys Val Ala Arg Cys Pro Ser Gly Val Lys Pro Asp Leu
65                  70                  75                  80

Ser Tyr Met Pro Ile Trp Lys Phe Pro Asp Glu Glu Gly Ala Cys Gln
                85                  90                  95

Pro

<210>  3
<211>  302
<212>  PRT
<213>  artifical

<400>  3

Met Arg Gly Ser His His His His His His Gly Ser Asp Leu Gly Lys
1               5                   10                  15

Lys Leu Leu Glu Ala Ala Arg Ala Gly Gln Asp Asp Glu Val Arg Ile
            20                  25                  30

Leu Met Ala Asn Gly Ala Asp Val Asn Ala Lys Asp Phe Tyr Gly Ile
        35                  40                  45

Thr Pro Leu His Leu Ala Ala Ala Tyr Gly His Leu Glu Ile Val Glu
    50                  55                  60

Val Leu Leu Lys His Gly Ala Asp Val Asn Ala His Asp Trp Asn Gly

65                  70                  75                  80

Trp Thr Pro Leu His Leu Ala Ala Lys Tyr Gly His Leu Glu Ile Val
                85                  90                  95

Glu Val Leu Leu Lys His Gly Ala Asp Val Asn Ala Ile Asp Asn Ala
            100                 105                 110

Gly Lys Thr Pro Leu His Leu Ala Ala Ala His Gly His Leu Glu Ile
        115                 120                 125

Val Glu Val Leu Leu Lys Tyr Gly Ala Asp Val Asn Ala Gln Asp Lys
    130                 135                 140

Phe Gly Glu Thr Ala Glu Asp Leu Ala Lys Asp Asn Gly Asn Gln Asp
145                 150                 155                 160

Ile Ala Asp Leu Leu Glu Lys Ala Leu Lys Leu Gly Gly Gly Gly Ser
                165                 170                 175

Arg Ser Asp Leu Gly Lys Lys Leu Leu Glu Ala Ala Arg Ala Gly Gln
            180                 185                 190

Asp Asp Glu Val Arg Ile Leu Met Ala Asn Gly Ala Asp Val Asn Ala
        195                 200                 205

Lys Asp Glu Tyr Gly Leu Thr Pro Leu Tyr Leu Ala Thr Ala His Gly
    210                 215                 220

His Leu Glu Ile Val Glu Val Leu Leu Lys Asn Gly Ala Asp Val Asn
225                 230                 235                 240

Ala Val Asp Ala Ile Gly Phe Thr Pro Leu His Leu Ala Ala Phe Ile
                245                 250                 255

Gly His Leu Glu Ile Ala Glu Val Leu Leu Lys His Gly Ala Asp Val
            260                 265                 270

Asn Ala Gln Asp Lys Phe Gly Lys Thr Ala Phe Asp Ile Ser Ile Gly
        275                 280                 285

Asn Gly Asn Glu Asp Leu Ala Glu Ile Leu Gln Lys Leu Asn
    290                 295                 300

<210>  4
<211>  335
<212>  PRT
<213>  artifical

<400>  4

Met Arg Gly Ser His His His His His His Gly Ser Asp Leu Gly Lys
1               5                   10                  15

Lys Leu Leu Glu Ala Ala Arg Ala Gly Gln Asp Asp Glu Val Arg Ile
            20                  25                  30

Leu Met Ala Asn Gly Ala Asp Val Asn Ala Lys Asp Phe Tyr Gly Ile
        35                  40                  45

Thr Pro Leu His Leu Ala Ala Ala Tyr Gly His Leu Glu Ile Val Glu
    50                  55                  60

Val Leu Leu Lys His Gly Ala Asp Val Asn Ala His Asp Trp Asn Gly
65                  70                  75                  80

Trp Thr Pro Leu His Leu Ala Ala Lys Tyr Gly His Leu Glu Ile Val
                85                  90                  95

Glu Val Leu Leu Lys His Gly Ala Asp Val Asn Ala Ile Asp Asn Ala
            100                 105                 110

Gly Lys Thr Pro Leu His Leu Ala Ala Ala His Gly His Leu Glu Ile
        115                 120                 125

Val Glu Val Leu Leu Lys Tyr Gly Ala Asp Val Asn Ala Gln Asp Lys
    130                 135                 140

Phe Gly Glu Thr Ala Glu Asp Leu Ala Lys Asp Asn Gly Asn Gln Asp
145                 150                 155                 160

Ile Ala Asp Leu Leu Glu Lys Ala Leu Lys Leu Gly Gly Gly Gly Ser
                165                 170                 175

Arg Ser Asp Leu Gly Lys Lys Leu Leu Glu Ala Ala Arg Ala Gly Gln
            180                 185                 190

Asp Asp Glu Val Arg Ile Leu Met Ala Asn Gly Ala Asp Val Asn Ala
        195                 200                 205

Thr Asp Ile His Gly His Thr Pro Leu His Leu Ala Ala Ala Met Gly
    210                 215                 220

His Leu Glu Ile Val Glu Val Leu Leu Lys Asn Gly Ala Asp Val Asn
225                 230                 235                 240

Ala Asn Asp Trp Arg Gly Phe Thr Pro Leu His Leu Ala Ala Leu Asn
                245                 250                 255

Gly His Leu Glu Ile Val Glu Val Leu Leu Lys Asn Gly Ala Asp Val
        260                 265                 270

Asn Ala Thr Asp Thr Ala Gly Asn Thr Pro Leu His Leu Ala Ala Trp
    275                 280                 285

Phe Gly His Leu Glu Ile Val Glu Val Leu Leu Lys Asn Gly Ala Asp
    290                 295                 300

Val Asn Ala Gln Asp Lys Phe Gly Lys Thr Ala Phe Asp Ile Ser Ile
305                 310                 315                 320

Asp Asn Gly Asn Glu Asp Leu Ala Glu Ile Leu Gln Lys Leu Asn
                325                 330                 335

<210>  5
<211>  298
<212>  PRT
<213>  artifical

<400>  5

Met Arg Gly Ser His His His His His His Gly Ser Asp Leu Gly Lys
1               5                   10                  15

Lys Leu Leu Glu Ala Ala Arg Ala Gly Gln Asp Asp Glu Val Arg Ile
            20                  25                  30

Leu Met Ala Asn Gly Ala Asp Val Asn Ala His Asp Phe Tyr Gly Ile
        35                  40                  45

Thr Pro Leu His Leu Ala Ala Asn Phe Gly His Leu Glu Ile Val Glu
    50                  55                  60

Val Leu Leu Lys His Gly Ala Asp Val Asn Ala Phe Asp Tyr Asp Asn
65                  70                  75                  80

Thr Pro Leu His Leu Ala Ala Asp Ala Gly His Leu Glu Ile Val Glu
                85                  90                  95

Val Leu Leu Lys Tyr Gly Ala Asp Val Asn Ala Ser Asp Arg Asp Gly
            100                 105                 110

His Thr Pro Leu His Leu Ala Ala Arg Glu Gly His Leu Glu Ile Val
        115                 120                 125

Glu Val Leu Leu Lys Asn Gly Ala Asp Val Asn Ala Gln Asp Lys Phe
    130                 135                 140

Gly Lys Thr Ala Phe Asp Ile Ser Ile Asp Asn Gly Asn Glu Asp Leu
145                 150                 155                 160

Ala Glu Ile Leu Gln Lys Leu Gly Gly Gly Gly Ser Arg Ser Asp Leu
                165                 170                 175

Gly Lys Lys Leu Leu Glu Ala Ala Arg Ala Gly Gln Asp Asp Glu Val
            180                 185                 190

Arg Ile Leu Met Ala Asn Gly Ala Asp Val Asn Ala Lys Asp Glu Tyr
        195                 200                 205

Gly Leu Thr Pro Leu Tyr Leu Ala Thr Ala His Gly His Leu Glu Ile
    210                 215                 220

Val Glu Val Leu Leu Lys Asn Gly Ala Asp Val Asn Ala Val Asp Ala
225                 230                 235                 240

Ile Gly Phe Thr Pro Leu His Leu Ala Ala Phe Ile Gly His Leu Glu
                245                 250                 255

Ile Ala Glu Val Leu Leu Lys His Gly Ala Asp Val Asn Ala Gln Asp
            260                 265                 270

Lys Phe Gly Lys Thr Ala Phe Asp Ile Ser Ile Gly Asn Gly Asn Glu
        275                 280                 285

Asp Leu Ala Glu Ile Leu Gln Lys Leu Asn
    290                 295

<210>  6
<211>  331
<212>  PRT
<213>  artifical

<400>  6

Met Arg Gly Ser His His His His His His Gly Ser Asp Leu Gly Lys
1               5                   10                  15

Lys Leu Leu Glu Ala Ala Arg Ala Gly Gln Asp Asp Glu Val Arg Ile
            20                  25                  30

Leu Met Ala Asn Gly Ala Asp Val Asn Ala His Asp Phe Tyr Gly Ile
        35                  40                  45

Thr Pro Leu His Leu Ala Ala Asn Phe Gly His Leu Glu Ile Val Glu
    50                  55                  60

Val Leu Leu Lys His Gly Ala Asp Val Asn Ala Phe Asp Tyr Asp Asn
65                  70                  75                  80

Thr Pro Leu His Leu Ala Ala Asp Ala Gly His Leu Glu Ile Val Glu
                85                  90                  95

Val Leu Leu Lys Tyr Gly Ala Asp Val Asn Ala Ser Asp Arg Asp Gly
            100                 105                 110

His Thr Pro Leu His Leu Ala Ala Arg Glu Gly His Leu Glu Ile Val
        115                 120                 125

Glu Val Leu Leu Lys Asn Gly Ala Asp Val Asn Ala Gln Asp Lys Phe
    130                 135                 140

Gly Lys Thr Ala Phe Asp Ile Ser Ile Asp Asn Gly Asn Glu Asp Leu
145                 150                 155                 160

Ala Glu Ile Leu Gln Lys Leu Gly Gly Gly Gly Ser Arg Ser Asp Leu
                165                 170                 175

Gly Lys Lys Leu Leu Glu Ala Ala Arg Ala Gly Gln Asp Asp Glu Val
            180                 185                 190

Arg Ile Leu Met Ala Asn Gly Ala Asp Val Asn Ala Thr Asp Ile His
        195                 200                 205

Gly His Thr Pro Leu His Leu Ala Ala Ala Met Gly His Leu Glu Ile
    210                 215                 220

Val Glu Val Leu Leu Lys Asn Gly Ala Asp Val Asn Ala Asn Asp Trp
225                 230                 235                 240

Arg Gly Phe Thr Pro Leu His Leu Ala Ala Leu Asn Gly His Leu Glu
                245                 250                 255

Ile Val Glu Val Leu Leu Lys Asn Gly Ala Asp Val Asn Ala Thr Asp
            260                 265                 270

Thr Ala Gly Asn Thr Pro Leu His Leu Ala Ala Trp Phe Gly His Leu
        275                 280                 285

Glu Ile Val Glu Val Leu Leu Lys Asn Gly Ala Asp Val Asn Ala Gln
    290                 295                 300

Asp Lys Phe Gly Lys Thr Ala Phe Asp Ile Ser Ile Asp Asn Gly Asn
305                 310                 315                 320

Glu Asp Leu Ala Glu Ile Leu Gln Lys Leu Asn
                325                 330

<210>  7

<211>  336
<212>  PRT
<213>  artifical

<400>  7
   Met Arg Gly Ser His His His His His His Gly Ser Asp Leu Gly Lys
   1               5                   10                  15
   Lys Leu Leu Glu Ala Ala Arg Ala Gly Gln Asp Asp Glu Val Arg Ile
               20                  25                  30
   Leu Met Ala Asn Gly Ala Asp Val Asn Ala His Asp Phe Tyr Gly Ile
           35                  40                  45
   Thr Pro Leu His Leu Ala Ala Asn Phe Gly His Leu Glu Ile Val Glu
       50                  55                  60
   Val Leu Leu Lys His Gly Ala Asp Val Asn Ala Phe Asp Tyr Asp Asn
   65                  70                  75                  80
   Thr Pro Leu His Leu Ala Ala Asp Ala Gly His Leu Glu Ile Val Glu
                   85                  90                  95
   Val Leu Leu Lys Tyr Gly Ala Asp Val Asn Ala Ser Asp Arg Asp Gly
               100                 105                 110
   His Thr Pro Leu His Leu Ala Ala Arg Glu Gly His Leu Glu Ile Val
           115                 120                 125
   Glu Val Leu Leu Lys Asn Gly Ala Asp Val Asn Ala Gln Asp Lys Phe
       130                 135                 140
   Gly Lys Thr Ala Phe Asp Ile Ser Ile Asp Asn Gly Asn Glu Asp Leu
   145                 150                 155                 160
   Ala Glu Ile Leu Gln Lys Leu Gly Gly Gly Gly Ser Gly Gly Gly Gly
                   165                 170                 175
   Ser Arg Ser Asp Leu Gly Lys Lys Leu Leu Glu Ala Ala Arg Ala Gly
               180                 185                 190
   Gln Asp Asp Glu Val Arg Ile Leu Met Ala Asn Gly Ala Asp Val Asn
           195                 200                 205
   Ala Thr Asp Ile His Gly His Thr Pro Leu His Leu Ala Ala Ala Met
       210                 215                 220
   Gly His Leu Glu Ile Val Glu Val Leu Leu Lys Asn Gly Ala Asp Val
   225                 230                 235                 240
   Asn Ala Asn Asp Trp Arg Gly Phe Thr Pro Leu His Leu Ala Ala Leu
                   245                 250                 255
   Asn Gly His Leu Glu Ile Val Glu Val Leu Leu Lys Asn Gly Ala Asp
               260                 265                 270
   Val Asn Ala Thr Asp Thr Ala Gly Asn Thr Pro Leu His Leu Ala Ala
           275                 280                 285
   Trp Phe Gly His Leu Glu Ile Val Glu Val Leu Leu Lys Asn Gly Ala
       290                 295                 300
   Asp Val Asn Ala Gln Asp Lys Phe Gly Lys Thr Ala Phe Asp Ile Ser
   305                 310                 315                 320
   Ile Asp Asn Gly Asn Glu Asp Leu Ala Glu Ile Leu Gln Lys Leu Asn
                   325                 330                 335

<210> 8
<211> 341
<212> PRT
<213> artifical

<400> 8
   Met Arg Gly Ser His His His His His His Gly Ser Asp Leu Gly Lys
   1               5                   10                  15
   Lys Leu Leu Glu Ala Ala Arg Ala Gly Gln Asp Asp Glu Val Arg Ile
               20                  25                  30
   Leu Met Ala Asn Gly Ala Asp Val Asn Ala His Asp Phe Tyr Gly Ile
           35                  40                  45
   Thr Pro Leu His Leu Ala Ala Asn Phe Gly His Leu Glu Ile Val Glu
       50                  55                  60
   Val Leu Leu Lys His Gly Ala Asp Val Asn Ala Phe Asp Tyr Asp Asn
   65                  70                  75                  80
   Thr Pro Leu His Leu Ala Ala Asp Ala Gly His Leu Glu Ile Val Glu
                   85                  90                  95
   Val Leu Leu Lys Tyr Gly Ala Asp Val Asn Ala Ser Asp Arg Asp Gly
               100                 105                 110
   His Thr Pro Leu His Leu Ala Ala Arg Glu Gly His Leu Glu Ile Val
           115                 120                 125
   Glu Val Leu Leu Lys Asn Gly Ala Asp Val Asn Ala Gln Asp Lys Phe
   130                 135                 140
   Gly Lys Thr Ala Phe Asp Ile Ser Ile Asp Asn Gly Asn Glu Asp Leu
   145                 150                 155                 160
   Ala Glu Ile Leu Gln Lys Leu Gly Gly Gly Gly Ser Gly Gly Gly Gly
                   165                 170                 175
   Ser Gly Gly Gly Gly Ser Arg Ser Asp Leu Gly Lys Lys Leu Leu Glu
               180                 185                 190
   Ala Ala Arg Ala Gly Gln Asp Asp Glu Val Arg Ile Leu Met Ala Asn
           195                 200                 205
   Gly Ala Asp Val Asn Ala Thr Asp Ile His Gly His Thr Pro Leu His
       210                 215                 220
   Leu Ala Ala Ala Met Gly His Leu Glu Ile Val Glu Val Leu Leu Lys
   225                 230                 235                 240
   Asn Gly Ala Asp Val Asn Ala Asn Asp Trp Arg Gly Phe Thr Pro Leu
                   245                 250                 255
   His Leu Ala Ala Leu Asn Gly His Leu Glu Ile Val Glu Val Leu Leu
               260                 265                 270
   Lys Asn Gly Ala Asp Val Asn Ala Thr Asp Thr Ala Gly Asn Thr Pro
           275                 280                 285
   Leu His Leu Ala Ala Trp Phe Gly His Leu Glu Ile Val Glu Val Leu
       290                 295                 300
   Leu Lys Asn Gly Ala Asp Val Asn Ala Gln Asp Lys Phe Gly Lys Thr
   305                 310                 315                 320
   Ala Phe Asp Ile Ser Ile Asp Asn Gly Asn Glu Asp Leu Ala Glu Ile
                   325                 330                 335
   Leu Gln Lys Leu Asn
               340

<210>  9
<211>  346
<212>  PRT
<213>  artifical

<400>  9

Met Arg Gly Ser His His His His His His Gly Ser Asp Leu Gly Lys
1               5                   10                  15

Lys Leu Leu Glu Ala Ala Arg Ala Gly Gln Asp Asp Glu Val Arg Ile
        20                  25                  30

Leu Met Ala Asn Gly Ala Asp Val Asn Ala His Asp Phe Tyr Gly Ile
    35                  40                  45

Thr Pro Leu His Leu Ala Ala Asn Phe Gly His Leu Glu Ile Val Glu
    50                  55                  60

Val Leu Leu Lys His Gly Ala Asp Val Asn Ala Phe Asp Tyr Asp Asn
65                  70                  75                  80

Thr Pro Leu His Leu Ala Ala Asp Ala Gly His Leu Glu Ile Val Glu
                85                  90                  95

Val Leu Leu Lys Tyr Gly Ala Asp Val Asn Ala Ser Asp Arg Asp Gly
            100                 105                 110

His Thr Pro Leu His Leu Ala Ala Arg Glu Gly His Leu Glu Ile Val
        115                 120                 125

Glu Val Leu Leu Lys Asn Gly Ala Asp Val Asn Ala Gln Asp Lys Phe
    130                 135                 140

Gly Lys Thr Ala Phe Asp Ile Ser Ile Asp Asn Gly Asn Glu Asp Leu
145                 150                 155                 160

Ala Glu Ile Leu Gln Lys Leu Gly Gly Gly Gly Ser Gly Gly Gly Gly
                165                 170                 175

Ser Gly Gly Gly Gly Ser Gly Gly Gly Gly Ser Arg Ser Asp Leu Gly
            180                 185                 190

Lys Lys Leu Leu Glu Ala Ala Arg Ala Gly Gln Asp Asp Glu Val Arg
        195                 200                 205

Ile Leu Met Ala Asn Gly Ala Asp Val Asn Ala Thr Asp Ile His Gly
    210                 215                 220

His Thr Pro Leu His Leu Ala Ala Ala Met Gly His Leu Glu Ile Val
225                 230                 235                 240

Glu Val Leu Leu Lys Asn Gly Ala Asp Val Asn Ala Asn Asp Trp Arg
                245                 250                 255

Gly Phe Thr Pro Leu His Leu Ala Ala Leu Asn Gly His Leu Glu Ile
            260                 265                 270

Val Glu Val Leu Leu Lys Asn Gly Ala Asp Val Asn Ala Thr Asp Thr
        275                 280                 285

Ala Gly Asn Thr Pro Leu His Leu Ala Ala Trp Phe Gly His Leu Glu
    290                 295                 300

Ile Val Glu Val Leu Leu Lys Asn Gly Ala Asp Val Asn Ala Gln Asp
305                 310                 315                 320

Lys Phe Gly Lys Thr Ala Phe Asp Ile Ser Ile Asp Asn Gly Asn Glu
                325                 330                 335

Asp Leu Ala Glu Ile Leu Gln Lys Leu Asn
            340                 345

<210>  10
<211>  158
<212>  PRT
<213>  artifical

<400>  10

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Tyr Tyr Gly Ile Thr Pro Leu His Leu Ala Ala His Thr Gly
        35                  40                  45

His Leu Glu Ile Val Glu Val Leu Leu Lys His Gly Ala Asp Val Asn
    50                  55                  60

Ala Arg Asn Trp Gly Trp Thr Pro Leu His Leu Ala Ala Met Thr Gly
65                  70                  75                  80

His Leu Glu Ile Val Glu Val Leu Leu Lys Tyr Gly Ala Asp Val Asn
                85                  90                  95

Ala Val Asp Glu Asp Gly Asp Thr Pro Leu His Leu Ala Ala Thr His
            100                 105                 110

Gly His Leu Glu Ile Val Glu Val Leu Leu Lys Tyr Gly Ala Asp Val
        115                 120                 125

Asn Ala Gln Asp Lys Phe Gly Lys Thr Ala Phe Asp Ile Ser Ile Asp
    130                 135                 140

Asn Gly Asn Glu Asp Leu Ala Glu Ile Leu Gln Lys Leu Asn
145                 150                 155

<210>  11
<211>  160
<212>  PRT
<213>  artifical

<400>  11

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Trp Tyr Gly Ile Thr Pro Leu His Leu Ala Ala Asp Thr Gly
        35                  40                  45

His Leu Glu Ile Val Glu Val Leu Leu Lys His Gly Ala Asp Val Asn
    50                  55                  60

Ala Phe Asp Ser Tyr Thr Gly His Thr Pro Leu His Leu Ala Ala Gln
65                  70                  75                  80

Lys Gly Gln Leu Glu Ile Val Glu Val Leu Leu Lys Tyr Gly Ala Asp
                85                  90                  95

Val Asn Ala Ile Asp Arg His Gly Lys Thr Pro Leu His Leu Ala Ala
            100                 105                 110

Leu Met Gly His Leu Glu Ile Val Glu Val Leu Leu Lys Tyr Gly Ala
        115                 120                 125

Asp Val Asn Ala Gln Asp Lys Phe Gly Lys Thr Ala Phe Asp Ile Ser
    130                 135                 140

Ile Asp Asn Gly Asn Glu Asp Leu Ala Glu Ile Leu Gln Lys Leu Asn
145                 150                 155                 160


<210>  12
<211>  125
<212>  PRT
<213>  artifical

<400>  12

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Phe Tyr Gly Lys Thr Pro Leu His Leu Ala Ala Thr Ile Gly

35                  40                  45

His Leu Lys Ile Val Glu Val Leu Leu Lys Tyr Gly Ala Asp Val Asn
    50                  55                  60

Ala Thr Asp Trp Gly Asn Thr Pro Leu His Leu Ala Ala Ile Asn Gly
65                  70                  75                  80

His Leu Glu Ile Val Glu Val Leu Leu Lys His Gly Ala Asp Val Asn
                85                  90                  95

Ala Gln Asp Lys Phe Gly Lys Thr Ala Phe Asp Ile Ser Ile Asp Asn
            100                 105                 110

Gly Asn Glu Asp Leu Ala Glu Ile Leu Gln Lys Leu Asn
        115                 120                 125

<210>  13
<211>  159
<212>  PRT
<213>  artifical

<400>  13

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe His Gly Leu Thr Pro Leu His Leu Ala Ala Gly Met Gly
        35                  40                  45

His Leu Glu Ile Val Glu Val Leu Leu Lys Asn Gly Ala Asp Val Asn
    50                  55                  60

Ala Val Asp Thr Asp Gly Ile Thr Leu Leu His Leu Ala Ala Tyr Tyr
65                  70                  75                  80

Gly His Leu Glu Ile Val Glu Val Leu Leu Lys His Gly Ala Asp Val
                85                  90                  95

Asn Ala His Asp Tyr Ala Gly Ser Thr Pro Leu His Leu Ala Ala Asn
            100                 105                 110

Thr Gly His Leu Glu Ile Val Glu Val Leu Leu Lys Asn Gly Ala Asp
        115                 120                 125

Val Asn Ala Gln Asp Lys Phe Gly Lys Thr Ala Phe Asp Ile Ser Ile
    130                 135                 140

Asp Asn Gly Asn Glu Asp Leu Ala Glu Ile Leu Gln Lys Leu Asn
145                 150                 155

<210>  14
<211>  159
<212>  PRT
<213>  artifical

<400>  14

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Phe Tyr Gly Ile Thr Pro Leu His Leu Ala Ala Ala Tyr Gly
        35                  40                  45

His Leu Glu Ile Val Glu Val Leu Leu Lys His Gly Ala Asp Val Asn
    50                  55                  60

Ala His Asp Trp Asn Gly Trp Thr Pro Leu His Leu Ala Ala Lys Tyr
65                  70                  75                  80

Gly His Leu Glu Ile Val Glu Val Leu Leu Lys His Gly Ala Asp Val
                85                  90                  95

Asn Ala Ile Asp Asn Ala Gly Lys Thr Pro Leu His Leu Ala Ala Ala
            100                 105                 110

His Gly His Leu Glu Ile Val Glu Val Leu Leu Lys Tyr Gly Ala Asp
        115                 120                 125

Val Asn Ala Gln Asp Lys Phe Gly Lys Thr Ala Phe Asp Ile Ser Ile
    130                 135                 140

Asp Asn Gly Asn Glu Asp Leu Ala Glu Ile Leu Gln Lys Leu Asn
145                 150                 155

<210>  15
<211>  159
<212>  PRT
<213>  artifical

<400>  15
   Gly Ser Asp Leu Gly Lys Lys Leu Leu Glu Ala Ala Arg Ala Gly Gln
   1               5                   10                  15
   Asp Asp Glu Val Arg Ile Leu Met Ala Asn Gly Ala Asp Val Asn Ala
               20                  25                  30
   Lys Asp Phe Tyr Gly Ile Thr Pro Leu His Leu Ala Ala Ala Tyr Gly
           35                  40                  45
   His Leu Glu Ile Val Glu Val Leu Leu Lys His Gly Ala Asp Val Asn
       50                  55                  60
   Ala His Asp Trp Asn Gly Trp Thr Pro Leu His Leu Ala Ala Lys Tyr
   65                  70                  75                  80
   Gly His Leu Glu Ile Val Glu Val Leu Leu Lys His Gly Ala Asp Val
                   85                  90                  95
   Asn Ala Ile Asp Asn Ala Gly Lys Thr Pro Leu His Leu Ala Ala Ala
               100                 105                 110
   His Gly His Leu Glu Ile Val Glu Val Leu Leu Lys Tyr Gly Ala Asp
           115                 120                 125
   Val Asn Ala Gln Asp Lys Phe Gly Lys Thr Pro Phe Asp Leu Ala Ile
       130                 135                 140
   Asp Asn Gly Asn Glu Asp Ile Ala Glu Val Leu Gln Lys Ala Ala
   145                 150                 155

<210>  16
<211>  159
<212>  PRT
<213>  artifical

<400>  16

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Phe Tyr Gly Ile Thr Pro Leu His Leu Ala Ala Ala Tyr Gly
        35                  40                  45

His Leu Glu Ile Val Glu Val Leu Leu Lys His Gly Ala Asp Val Asn
    50                  55                  60

Ala His Asp Trp Asn Gly Trp Thr Pro Leu His Leu Ala Ala Lys Tyr
65                  70                  75                  80

Gly His Leu Glu Ile Val Glu Val Leu Leu Lys His Gly Ala Asp Val
                85                  90                  95

Asn Ala Ile Asp Asn Ala Gly Lys Thr Pro Leu His Leu Ala Ala Ala
            100                 105                 110

His Gly His Leu Glu Ile Val Glu Val Leu Leu Lys Tyr Gly Ala Asp
        115                 120                 125

Val Asn Ala Gln Asp Lys Phe Gly Lys Thr Ala Glu Asp Leu Ala Lys
    130                 135                 140

Asp Asn Gly Asn Gln Asp Ile Ala Asp Leu Leu Glu Lys Ala Leu
145                 150                 155

<210>  17
<211>  159
<212>  PRT
<213>  artifical

<400>  17

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Phe Tyr Gly Ile Thr Pro Leu His Leu Ala Ala Ala Tyr Gly
        35                  40                  45

His Leu Glu Ile Val Glu Val Leu Leu Lys His Gly Ala Asp Val Asn
    50                  55                  60

Ala His Asp Trp Asn Gly Trp Thr Pro Leu His Leu Ala Ala Lys Tyr
65                  70                  75                  80

Gly His Leu Glu Ile Val Glu Val Leu Leu Lys His Gly Ala Asp Val
                85                  90                  95

Asn Ala Ile Asp Asn Ala Gly Lys Thr Pro Leu His Leu Ala Ala Ala
            100                 105                 110

His Gly His Leu Glu Ile Val Glu Val Leu Leu Lys Tyr Gly Ala Asp
        115                 120                 125

Val Asn Ala Gln Asp Lys Phe Gly Lys Thr Ala Met Asp Leu Ala Arg
    130                 135                 140

Asp Asn Gly Asn Glu Asp Ile Tyr Lys Leu Leu Ala Lys Ala Leu
145                 150                 155

<210>  18
<211>  158

<212>  PRT
<213>  artifical

<400>  18

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Ile Thr Pro Leu His Leu Ala Ala Asn Phe Gly
        35                  40                  45

His Leu Glu Ile Val Glu Val Leu Leu Lys His Gly Ala Asp Val Asn
    50                  55                  60

Ala Phe Asp Tyr Asp Asn Thr Pro Leu His Leu Ala Ala Asp Ala Gly
65                  70                  75                  80

His Leu Glu Ile Val Glu Val Leu Leu Lys Tyr Gly Ala Asp Val Asn
                85                  90                  95

Ala Ser Asp Arg Asp Gly His Thr Pro Leu His Leu Ala Ala Arg Glu
            100                 105                 110

Gly His Leu Glu Ile Val Glu Val Leu Leu Lys Asn Gly Ala Asp Val
        115                 120                 125

Asn Ala Gln Asp Lys Phe Gly Lys Thr Ala Phe Asp Ile Ser Ile Asp
    130                 135                 140

Asn Gly Asn Glu Asp Leu Ala Glu Ile Leu Gln Lys Leu Asn
145                 150                 155

<210>  19
<211>  158
<212>  PRT
<213>  artifical

<400>  19

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Ile Thr Pro Leu His Leu Ala Ala Asn Phe Gly
        35                  40                  45

His Leu Glu Ile Val Glu Val Leu Leu Lys His Gly Ala Asp Val Asn

50                  55                  60

Ala Phe Asp Tyr Asp Asn Thr Pro Leu His Leu Ala Ala Asp Ala Gly
65                  70                  75                  80

His Leu Glu Ile Val Glu Val Leu Leu Lys Tyr Gly Ala Asp Val Asn
                85                  90                  95

Ala Ser Asp Arg Asp Gly His Thr Pro Leu His Leu Ala Ala Arg Glu
            100                 105                 110

Gly His Leu Glu Ile Val Glu Val Leu Leu Lys Asn Gly Ala Asp Val
        115                 120                 125

Asn Ala Gln Asp Lys Phe Gly Lys Thr Pro Phe Asp Leu Ala Ile Asp
    130                 135                 140

Asn Gly Asn Glu Asp Ile Ala Glu Val Leu Gln Lys Ala Ala
145                 150                 155

<210>  20
<211>  158
<212>  PRT
<213>  artifical

<400>  20

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Ile Thr Pro Leu His Leu Ala Ala Asn Phe Gly
        35                  40                  45

His Leu Glu Ile Val Glu Val Leu Leu Lys His Gly Ala Asp Val Asn
    50                  55                  60

Ala Phe Asp Tyr Asp Asn Thr Pro Leu His Leu Ala Ala Asp Ala Gly
65                  70                  75                  80

His Leu Glu Ile Val Glu Val Leu Leu Lys Tyr Gly Ala Asp Val Asn
                85                  90                  95

Ala Ser Asp Arg Asp Gly His Thr Pro Leu His Leu Ala Ala Arg Glu
            100                 105                 110

Gly His Leu Glu Ile Val Glu Val Leu Leu Lys Asn Gly Ala Asp Val
        115                 120                 125

Asn Ala Gln Asp Lys Phe Gly Lys Thr Ala Glu Asp Leu Ala Lys Asp
    130                 135                 140

Asn Gly Asn Gln Asp Ile Ala Asp Leu Leu Glu Lys Ala Leu
145                 150                 155

<210>  21
<211>  158
<212>  PRT
<213>  artifical

<400>  21

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Ile Thr Pro Leu His Leu Ala Ala Asn Phe Gly
        35                  40                  45

His Leu Glu Ile Val Glu Val Leu Leu Lys His Gly Ala Asp Val Asn
    50                  55                  60

Ala Phe Asp Tyr Asp Asn Thr Pro Leu His Leu Ala Ala Asp Ala Gly
65                  70                  75                  80

His Leu Glu Ile Val Glu Val Leu Leu Lys Tyr Gly Ala Asp Val Asn
                85                  90                  95

Ala Ser Asp Arg Asp Gly His Thr Pro Leu His Leu Ala Ala Arg Glu
            100                 105                 110

Gly His Leu Glu Ile Val Glu Val Leu Leu Lys Asn Gly Ala Asp Val
        115                 120                 125

Asn Ala Gln Asp Lys Phe Gly Lys Thr Ala Met Asp Leu Ala Arg Asp
    130                 135                 140

Asn Gly Asn Glu Asp Ile Tyr Lys Leu Leu Ala Lys Ala Leu
145                 150                 155

<210>  22
<211>  159
<212>  PRT
<213>  artifical

<400>  22

Gly Ser Asp Leu Gly Lys Lys Leu Leu Glu Thr Ala Arg Ala Gly Gln
1               5                   10                  15

Asp Asp Glu Val Arg Ile Leu Met Ala Asn Gly Ala Asp Val Asn Ala
            20                  25                  30

Thr Asp Phe Tyr Gly Leu Thr Pro Leu His Leu Ala Ala Tyr Tyr Gly
        35                  40                  45

His Leu Glu Ile Val Glu Val Leu Leu Lys Asn Gly Ala Asp Val Asn
    50                  55                  60

Ala Ser Asp Trp Asn Gly Tyr Thr Pro Leu Arg Leu Ala Ala Asp Ala
65                  70                  75                  80

Gly His Leu Glu Ile Val Glu Val Leu Leu Lys Asn Gly Ala Asp Val
                85                  90                  95

Asn Ala Phe Asp Gln Phe Gly Ser Thr Pro Leu His Leu Ala Ala Ala
            100                 105                 110

Thr Gly His Leu Glu Ile Val Glu Val Leu Leu Lys Tyr Gly Ala Asp
        115                 120                 125

Val Asn Ala Gln Asp Lys Phe Gly Lys Thr Ala Phe Asp Ile Ser Ile
    130                 135                 140

Asp Asn Gly Asn Glu Asp Leu Ala Glu Ile Leu Gln Lys Leu Asn
145                 150                 155

<210> 23
<211> 158
<212> PRT
<213> artifical

<400> 23

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Lys Thr Pro Leu His Leu Ala Ala Ala Ile Gly
        35                  40                  45

His Leu Glu Ile Val Glu Val Leu Leu Lys Asn Gly Ala Asp Val Asn
    50                  55                  60

Ala Thr Asp Tyr Gly Leu Thr Pro Leu His Leu Ala Ala Asp Asn Gly
65                  70                  75                  80

His Leu Glu Ile Val Glu Val Leu Leu Lys Asn Gly Ala Asp Val Asn
                85                  90                  95

Ala Phe Asp Phe Thr Gly Arg Thr Pro Leu His Leu Ala Ala Ser Gln
            100                 105                 110

Gly His Leu Glu Ile Val Glu Val Leu Leu Lys Asn Gly Ala Asp Val
        115                 120                 125

Asn Ala Gln Asp Lys Phe Gly Lys Thr Ala Phe Asp Ile Ser Ile Asp
    130                 135                 140

Asn Gly Asn Glu Asp Leu Ala Glu Ile Leu Gln Lys Leu Asn
145                 150                 155

<210>  24
<211>  160
<212>  PRT
<213>  artifical

<400>  24

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Trp His Gly Ile Thr Pro Leu His Leu Ala Ala Phe Tyr Gly
        35                  40                  45

His Leu Glu Ile Val Glu Val Leu Leu Lys Asn Gly Ala Asp Val Asn
    50                  55                  60

Ala Phe Asp Asp Tyr Asp Gly Ser Thr Pro Leu His Leu Ala Ala Trp
65                  70                  75                  80

Met Gly His Leu Glu Ile Val Glu Val Leu Leu Lys His Gly Ala Asp
                85                  90                  95

Val Asn Ala Thr Asp His Phe Gly Asn Thr Pro Leu His Leu Ala Ala
            100                 105                 110

Ala Met Gly His Leu Glu Ile Val Glu Val Leu Leu Lys His Gly Ala
        115                 120                 125

Asp Val Asn Ala Gln Asp Lys Phe Gly Lys Thr Ala Phe Asp Ile Ser
    130                 135                 140

Ile Asp Asn Gly Asn Glu Asp Leu Ala Glu Ile Leu Gln Lys Leu Asn
145                 150                 155                 160

<210>  25
<211>  126
<212>  PRT
<213>  artifical

<400>  25

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Glu Tyr Gly Leu Thr Pro Leu Tyr Leu Ala Thr Ala His Gly
        35                  40                  45

His Leu Glu Ile Val Glu Val Leu Leu Lys Asn Gly Ala Asp Val Asn
    50                  55                  60

Ala Val Asp Ala Ile Gly Phe Thr Pro Leu His Leu Ala Ala Phe Ile
65                  70                  75                  80

Gly His Leu Glu Ile Ala Glu Val Leu Leu Lys His Gly Ala Asp Val
                85                  90                  95

Asn Ala Gln Asp Lys Phe Gly Lys Thr Ala Phe Asp Ile Ser Ile Gly
            100                 105                 110

Asn Gly Asn Glu Asp Leu Ala Glu Ile Leu Gln Lys Leu Asn
        115                 120                 125


<210>  26
<211>  159
<212>  PRT
<213>  artifical

<400>  26

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Thr Asp Ile His Gly His Thr Pro Leu His Leu Ala Ala Ala Met Gly
        35                  40                  45

His Leu Glu Ile Val Glu Val Leu Leu Lys Asn Gly Ala Asp Val Asn
    50                  55                  60

Ala Asn Asp Trp Arg Gly Phe Thr Pro Leu His Leu Ala Ala Leu Asn

65                  70                  75                  80

Gly His Leu Glu Ile Val Glu Val Leu Leu Lys Asn Gly Ala Asp Val
                85                  90                  95

Asn Ala Thr Asp Thr Ala Gly Asn Thr Pro Leu His Leu Ala Ala Trp
            100                 105                 110

Phe Gly His Leu Glu Ile Val Glu Val Leu Leu Lys Asn Gly Ala Asp
        115                 120                 125

Val Asn Ala Gln Asp Lys Phe Gly Lys Thr Ala Phe Asp Ile Ser Ile
    130                 135                 140

Asp Asn Gly Asn Glu Asp Leu Ala Glu Ile Leu Gln Lys Leu Asn
145                 150                 155

<210>  27
<211>  159
<212>  PRT
<213>  artifical

<400>  27

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Thr Asp Ile His Gly His Thr Pro Leu His Leu Ala Ala Ala Met Gly
        35                  40                  45

His Leu Glu Ile Val Glu Val Leu Leu Lys Asn Gly Ala Asp Val Asn
    50                  55                  60

Ala Asn Asp Trp Arg Gly Phe Thr Pro Leu His Leu Ala Ala Leu Asn
65                  70                  75                  80

Gly His Leu Glu Ile Val Glu Val Leu Leu Lys Asn Gly Ala Asp Val
                85                  90                  95

Asn Ala Thr Asp Thr Ala Gly Asn Thr Pro Leu His Leu Ala Ala Trp
            100                 105                 110

Phe Gly His Leu Glu Ile Val Glu Val Leu Leu Lys Asn Gly Ala Asp
        115                 120                 125

Val Asn Ala Gln Asp Lys Phe Gly Lys Thr Pro Phe Asp Leu Ala Ile
    130                 135                 140

Asp Asn Gly Asn Glu Asp Ile Ala Glu Val Leu Gln Lys Ala Ala
145                 150                 155

<210>  28
<211>  159
<212>  PRT
<213>  artifical

<400>  28

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Thr Asp Ile His Gly His Thr Pro Leu His Leu Ala Ala Ala Met Gly
        35                  40                  45

His Leu Glu Ile Val Glu Val Leu Leu Lys Asn Gly Ala Asp Val Asn
    50                  55                  60

Ala Asn Asp Trp Arg Gly Phe Thr Pro Leu His Leu Ala Ala Leu Asn
65                  70                  75                  80

Gly His Leu Glu Ile Val Glu Val Leu Leu Lys Asn Gly Ala Asp Val
                85                  90                  95

Asn Ala Thr Asp Thr Ala Gly Asn Thr Pro Leu His Leu Ala Ala Trp
            100                 105                 110

Phe Gly His Leu Glu Ile Val Glu Val Leu Leu Lys Asn Gly Ala Asp
        115                 120                 125

Val Asn Ala Gln Asp Lys Phe Gly Lys Thr Ala Glu Asp Leu Ala Lys
    130                 135                 140

Asp Asn Gly Asn Gln Asp Ile Ala Asp Leu Leu Glu Lys Ala Leu
145                 150                 155

<210>  29
<211>  159
<212>  PRT
<213>  artifical

<400>  29
   Gly Ser Asp Leu Gly Lys Lys Leu Leu Glu Ala Ala Arg Ala Gly Gln
   1               5                   10                  15
   Asp Asp Glu Val Arg Ile Leu Met Ala Asn Gly Ala Asp Val Asn Ala
               20                  25                  30
   Thr Asp Ile His Gly His Thr Pro Leu His Leu Ala Ala Ala Met Gly
           35                  40                  45
   His Leu Glu Ile Val Glu Val Leu Leu Lys Asn Gly Ala Asp Val Asn
       50                  55                  60
   Ala Asn Asp Trp Arg Gly Phe Thr Pro Leu His Leu Ala Ala Leu Asn
   65                  70                  75                  80
   Gly His Leu Glu Ile Val Glu Val Leu Leu Lys Asn Gly Ala Asp Val
                   85                  90                  95
   Asn Ala Thr Asp Thr Ala Gly Asn Thr Pro Leu His Leu Ala Ala Trp
               100                 105                 110
   Phe Gly His Leu Glu Ile Val Glu Val Leu Leu Lys Asn Gly Ala Asp
           115                 120                 125
   Val Asn Ala Gln Asp Lys Phe Gly Lys Thr Ala Met Asp Leu Ala Arg
       130                 135                 140
   Asp Asn Gly Asn Glu Asp Ile Tyr Lys Leu Leu Ala Lys Ala Leu
   145                 150                 155

<210>  30
<211>  158
<212>  PRT
<213>  artifical

<400>  30

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Tyr Tyr Gly Ile Thr Pro Leu His Leu Ala Ala His Thr Gly
        35                  40                  45

His Leu Glu Ile Val Glu Val Leu Leu Lys His Gly Ala Asp Val Asn
    50                  55                  60

Ala Arg Asn Trp Gly Trp Thr Pro Leu His Leu Ala Ala Met Thr Gly
65                  70                  75                  80

His Leu Glu Ile Val Glu Val Leu Leu Lys Tyr Gly Ala Asp Val Asn
                85                  90                  95

Ala Val Asp Glu Asp Gly Asp Thr Pro Leu His Leu Ala Ala Thr His
            100                 105                 110

Gly His Leu Glu Ile Val Glu Val Leu Leu Lys Tyr Gly Ala Asp Val
        115                 120                 125

Asn Ala Gln Asp Lys Phe Gly Lys Thr Pro Phe Asp Leu Ala Ile Asp
    130                 135                 140

Asn Gly Asn Glu Asp Ile Ala Glu Val Leu Gln Lys Ala Ala
145                 150                 155

<210> 31
<211> 158
<212> PRT
<213> artifical

<400> 31

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Tyr Tyr Gly Ile Thr Pro Leu His Leu Ala Ala His Thr Gly
        35                  40                  45

His Leu Glu Ile Val Glu Val Leu Leu Lys His Gly Ala Asp Val Asn
    50                  55                  60

Ala Arg Asn Trp Gly Trp Thr Pro Leu His Leu Ala Ala Met Thr Gly
65                  70                  75                  80

His Leu Glu Ile Val Glu Val Leu Leu Lys Tyr Gly Ala Asp Val Asn
                85                  90                  95

Ala Val Asp Glu Asp Gly Asp Thr Pro Leu His Leu Ala Ala Thr His
            100                 105                 110

Gly His Leu Glu Ile Val Glu Val Leu Leu Lys Tyr Gly Ala Asp Val
        115                 120                 125

Asn Ala Gln Asp Lys Phe Gly Lys Thr Ala Glu Asp Leu Ala Lys Asp
    130                 135                 140

Asn Gly Asn Gln Asp Ile Ala Asp Leu Leu Glu Lys Ala Leu
145                 150                 155

<210> 32
<211> 158

<212>  PRT
<213>  artifical

<400>  32

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Tyr Tyr Gly Ile Thr Pro Leu His Leu Ala Ala His Thr Gly
        35                  40                  45

His Leu Glu Ile Val Glu Val Leu Leu Lys His Gly Ala Asp Val Asn
    50                  55                  60

Ala Arg Asn Trp Gly Trp Thr Pro Leu His Leu Ala Ala Met Thr Gly
65                  70                  75                  80

His Leu Glu Ile Val Glu Val Leu Leu Lys Tyr Gly Ala Asp Val Asn
                85                  90                  95

Ala Val Asp Glu Asp Gly Asp Thr Pro Leu His Leu Ala Ala Thr His
            100                 105                 110

Gly His Leu Glu Ile Val Glu Val Leu Leu Lys Tyr Gly Ala Asp Val
        115                 120                 125

Asn Ala Gln Asp Lys Phe Gly Lys Thr Ala Met Asp Leu Ala Arg Asp
    130                 135                 140

Asn Gly Asn Glu Asp Ile Tyr Lys Leu Leu Ala Lys Ala Leu
145                 150                 155

<210>  33
<211>  160
<212>  PRT
<213>  artifical

<400>  33

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Trp Tyr Gly Ile Thr Pro Leu His Leu Ala Ala Asp Thr Gly
        35                  40                  45

His Leu Glu Ile Val Glu Val Leu Leu Lys His Gly Ala Asp Val Asn

50                  55                  60

Ala Phe Asp Ser Tyr Thr Gly His Thr Pro Leu His Leu Ala Ala Gln
65                  70                  75                  80

Lys Gly Gln Leu Glu Ile Val Glu Val Leu Leu Lys Tyr Gly Ala Asp
                85                  90                  95

Val Asn Ala Ile Asp Arg His Gly Lys Thr Pro Leu His Leu Ala Ala
            100                 105                 110

Leu Met Gly His Leu Glu Ile Val Glu Val Leu Leu Lys Tyr Gly Ala
        115                 120                 125

Asp Val Asn Ala Gln Asp Lys Phe Gly Lys Thr Pro Phe Asp Leu Ala
    130                 135                 140

Ile Asp Asn Gly Asn Glu Asp Ile Ala Glu Val Leu Gln Lys Ala Ala
145                 150                 155                 160

<210>  34
<211>  160
<212>  PRT
<213>  artifical

<400>  34

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Trp Tyr Gly Ile Thr Pro Leu His Leu Ala Ala Asp Thr Gly
        35                  40                  45

His Leu Glu Ile Val Glu Val Leu Leu Lys His Gly Ala Asp Val Asn
    50                  55                  60

Ala Phe Asp Ser Tyr Thr Gly His Thr Pro Leu His Leu Ala Ala Gln
65                  70                  75                  80

Lys Gly Gln Leu Glu Ile Val Glu Val Leu Leu Lys Tyr Gly Ala Asp
                85                  90                  95

Val Asn Ala Ile Asp Arg His Gly Lys Thr Pro Leu His Leu Ala Ala
            100                 105                 110

Leu Met Gly His Leu Glu Ile Val Glu Val Leu Leu Lys Tyr Gly Ala
        115                 120                 125

Asp Val Asn Ala Gln Asp Lys Phe Gly Lys Thr Ala Glu Asp Leu Ala
    130                 135                 140

Lys Asp Asn Gly Asn Gln Asp Ile Ala Asp Leu Leu Glu Lys Ala Leu
145                 150                 155                 160

<210>  35
<211>  160
<212>  PRT
<213>  artifical

<400>  35

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Lys Asp Trp Tyr Gly Ile Thr Pro Leu His Leu Ala Ala Asp Thr Gly
        35                  40                  45

His Leu Glu Ile Val Glu Val Leu Leu Lys His Gly Ala Asp Val Asn
    50                  55                  60

Ala Phe Asp Ser Tyr Thr Gly His Thr Pro Leu His Leu Ala Ala Gln
65                  70                  75                  80

Lys Gly Gln Leu Glu Ile Val Glu Val Leu Leu Lys Tyr Gly Ala Asp
                85                  90                  95

Val Asn Ala Ile Asp Arg His Gly Lys Thr Pro Leu His Leu Ala Ala
            100                 105                 110

Leu Met Gly His Leu Glu Ile Val Glu Val Leu Leu Lys Tyr Gly Ala
        115                 120                 125

Asp Val Asn Ala Gln Asp Lys Phe Gly Lys Thr Ala Met Asp Leu Ala
    130                 135                 140

Arg Asp Asn Gly Asn Glu Asp Ile Tyr Lys Leu Leu Ala Lys Ala Leu
145                 150                 155                 160

<210>  36
<211>  125
<212>  PRT
<213>  artifical

<400>  36

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Phe Tyr Gly Lys Thr Pro Leu His Leu Ala Ala Thr Ile Gly
        35                  40                  45

His Leu Lys Ile Val Glu Val Leu Leu Lys Tyr Gly Ala Asp Val Asn
    50                  55                  60

Ala Thr Asp Trp Gly Asn Thr Pro Leu His Leu Ala Ala Ile Asn Gly
65                  70                  75                  80

His Leu Glu Ile Val Glu Val Leu Leu Lys His Gly Ala Asp Val Asn
                85                  90                  95

Ala Gln Asp Lys Phe Gly Lys Thr Pro Phe Asp Leu Ala Ile Asp Asn
            100                 105                 110

Gly Asn Glu Asp Ile Ala Glu Val Leu Gln Lys Ala Ala
        115                 120                 125

<210>  37
<211>  125
<212>  PRT
<213>  artifical

<400>  37

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Phe Tyr Gly Lys Thr Pro Leu His Leu Ala Ala Thr Ile Gly
        35                  40                  45

His Leu Lys Ile Val Glu Val Leu Leu Lys Tyr Gly Ala Asp Val Asn
    50                  55                  60

Ala Thr Asp Trp Gly Asn Thr Pro Leu His Leu Ala Ala Ile Asn Gly
65                  70                  75                  80

His Leu Glu Ile Val Glu Val Leu Leu Lys His Gly Ala Asp Val Asn
                85                  90                  95

Ala Gln Asp Lys Phe Gly Lys Thr Ala Glu Asp Leu Ala Lys Asp Asn
            100                 105                 110

Gly Asn Gln Asp Ile Ala Asp Leu Leu Glu Lys Ala Leu
        115                 120                 125

<210>  38
<211>  125
<212>  PRT
<213>  artifical

<400>  38

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Ser Asp Phe Tyr Gly Lys Thr Pro Leu His Leu Ala Ala Thr Ile Gly
        35                  40                  45

His Leu Lys Ile Val Glu Val Leu Leu Lys Tyr Gly Ala Asp Val Asn
    50                  55                  60

Ala Thr Asp Trp Gly Asn Thr Pro Leu His Leu Ala Ala Ile Asn Gly
65                  70                  75                  80

His Leu Glu Ile Val Glu Val Leu Leu Lys His Gly Ala Asp Val Asn
                85                  90                  95

Ala Gln Asp Lys Phe Gly Lys Thr Ala Met Asp Leu Ala Arg Asp Asn
            100                 105                 110

Gly Asn Glu Asp Ile Tyr Lys Leu Leu Ala Lys Ala Leu
        115                 120                 125

<210>  39
<211>  159
<212>  PRT
<213>  artifical

<400>  39

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe His Gly Leu Thr Pro Leu His Leu Ala Ala Gly Met Gly
        35                  40                  45

His Leu Glu Ile Val Glu Val Leu Leu Lys Asn Gly Ala Asp Val Asn
    50                  55                  60

Ala Val Asp Thr Asp Gly Ile Thr Leu Leu His Leu Ala Ala Tyr Tyr
65                  70                  75                  80

Gly His Leu Glu Ile Val Glu Val Leu Leu Lys His Gly Ala Asp Val
                85                  90                  95

Asn Ala His Asp Tyr Ala Gly Ser Thr Pro Leu His Leu Ala Ala Asn
            100                 105                 110

Thr Gly His Leu Glu Ile Val Glu Val Leu Leu Lys Asn Gly Ala Asp
        115                 120                 125

Val Asn Ala Gln Asp Lys Phe Gly Lys Thr Pro Phe Asp Leu Ala Ile
    130                 135                 140

Asp Asn Gly Asn Glu Asp Ile Ala Glu Val Leu Gln Lys Ala Ala
145                 150                 155

<210>  40
<211>  159
<212>  PRT
<213>  artifical

<400>  40

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe His Gly Leu Thr Pro Leu His Leu Ala Ala Gly Met Gly
        35                  40                  45

His Leu Glu Ile Val Glu Val Leu Leu Lys Asn Gly Ala Asp Val Asn
    50                  55                  60

Ala Val Asp Thr Asp Gly Ile Thr Leu Leu His Leu Ala Ala Tyr Tyr
65                  70                  75                  80

Gly His Leu Glu Ile Val Glu Val Leu Leu Lys His Gly Ala Asp Val
                85                  90                  95

Asn Ala His Asp Tyr Ala Gly Ser Thr Pro Leu His Leu Ala Ala Asn
            100                 105                 110

Thr Gly His Leu Glu Ile Val Glu Val Leu Leu Lys Asn Gly Ala Asp
        115                 120                 125

Val Asn Ala Gln Asp Lys Phe Gly Lys Thr Ala Glu Asp Leu Ala Lys

130                 135                 140

Asp Asn Gly Asn Gln Asp Ile Ala Asp Leu Leu Glu Lys Ala Leu
145                 150                 155

<210>  41
<211>  159
<212>  PRT
<213>  artifical

<400>  41

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe His Gly Leu Thr Pro Leu His Leu Ala Ala Gly Met Gly
        35                  40                  45

His Leu Glu Ile Val Glu Val Leu Leu Lys Asn Gly Ala Asp Val Asn
    50                  55                  60

Ala Val Asp Thr Asp Gly Ile Thr Leu Leu His Leu Ala Ala Tyr Tyr
65                  70                  75                  80

Gly His Leu Glu Ile Val Glu Val Leu Leu Lys His Gly Ala Asp Val
                85                  90                  95

Asn Ala His Asp Tyr Ala Gly Ser Thr Pro Leu His Leu Ala Ala Asn
            100                 105                 110

Thr Gly His Leu Glu Ile Val Glu Val Leu Leu Lys Asn Gly Ala Asp
        115                 120                 125

Val Asn Ala Gln Asp Lys Phe Gly Lys Thr Ala Met Asp Leu Ala Arg
    130                 135                 140

Asp Asn Gly Asn Glu Asp Ile Tyr Lys Leu Leu Ala Lys Ala Leu
145                 150                 155

<210>  42
<211>  159
<212>  PRT
<213>  artifical

<400>  42

Gly Ser Asp Leu Gly Lys Lys Leu Leu Glu Thr Ala Arg Ala Gly Gln
1               5                   10                  15

Asp Asp Glu Val Arg Ile Leu Met Ala Asn Gly Ala Asp Val Asn Ala
            20                  25                  30

Thr Asp Phe Tyr Gly Leu Thr Pro Leu His Leu Ala Ala Tyr Tyr Gly
        35                  40                  45

His Leu Glu Ile Val Glu Val Leu Leu Lys Asn Gly Ala Asp Val Asn
    50                  55                  60

Ala Ser Asp Trp Asn Gly Tyr Thr Pro Leu Arg Leu Ala Ala Asp Ala
65                  70                  75                  80

Gly His Leu Glu Ile Val Glu Val Leu Leu Lys Asn Gly Ala Asp Val
                85                  90                  95

Asn Ala Phe Asp Gln Phe Gly Ser Thr Pro Leu His Leu Ala Ala Ala
            100                 105                 110

Thr Gly His Leu Glu Ile Val Glu Val Leu Leu Lys Tyr Gly Ala Asp
        115                 120                 125

Val Asn Ala Gln Asp Lys Phe Gly Lys Thr Pro Phe Asp Leu Ala Ile
    130                 135                 140

Asp Asn Gly Asn Glu Asp Ile Ala Glu Val Leu Gln Lys Ala Ala
145                 150                 155

<210>  43
<211>  159
<212>  PRT
<213>  artifical

<400>  43

Gly Ser Asp Leu Gly Lys Lys Leu Leu Glu Thr Ala Arg Ala Gly Gln
1               5                   10                  15

Asp Asp Glu Val Arg Ile Leu Met Ala Asn Gly Ala Asp Val Asn Ala
            20                  25                  30

Thr Asp Phe Tyr Gly Leu Thr Pro Leu His Leu Ala Ala Tyr Tyr Gly
        35                  40                  45

His Leu Glu Ile Val Glu Val Leu Leu Lys Asn Gly Ala Asp Val Asn
    50                  55                  60

Ala Ser Asp Trp Asn Gly Tyr Thr Pro Leu Arg Leu Ala Ala Asp Ala
65                  70                  75                  80

Gly His Leu Glu Ile Val Glu Val Leu Leu Lys Asn Gly Ala Asp Val
                85                  90                  95

Asn Ala Phe Asp Gln Phe Gly Ser Thr Pro Leu His Leu Ala Ala Ala
            100                 105                 110

Thr Gly His Leu Glu Ile Val Glu Val Leu Leu Lys Tyr Gly Ala Asp
        115                 120                 125

Val Asn Ala Gln Asp Lys Phe Gly Lys Thr Ala Glu Asp Leu Ala Lys
    130                 135                 140

Asp Asn Gly Asn Gln Asp Ile Ala Asp Leu Leu Glu Lys Ala Leu
145                 150                 155

<210>  44
<211>  159
<212>  PRT
<213>  artifical

<400>  44

Gly Ser Asp Leu Gly Lys Lys Leu Leu Glu Thr Ala Arg Ala Gly Gln
1               5                   10                  15

Asp Asp Glu Val Arg Ile Leu Met Ala Asn Gly Ala Asp Val Asn Ala
            20                  25                  30

Thr Asp Phe Tyr Gly Leu Thr Pro Leu His Leu Ala Ala Tyr Tyr Gly
        35                  40                  45

His Leu Glu Ile Val Glu Val Leu Leu Lys Asn Gly Ala Asp Val Asn
    50                  55                  60

Ala Ser Asp Trp Asn Gly Tyr Thr Pro Leu Arg Leu Ala Ala Asp Ala
65                  70                  75                  80

Gly His Leu Glu Ile Val Glu Val Leu Leu Lys Asn Gly Ala Asp Val
                85                  90                  95

Asn Ala Phe Asp Gln Phe Gly Ser Thr Pro Leu His Leu Ala Ala Ala
            100                 105                 110

Thr Gly His Leu Glu Ile Val Glu Val Leu Leu Lys Tyr Gly Ala Asp
        115                 120                 125

Val Asn Ala Gln Asp Lys Phe Gly Lys Thr Ala Met Asp Leu Ala Arg
    130                 135                 140

Asp Asn Gly Asn Glu Asp Ile Tyr Lys Leu Leu Ala Lys Ala Leu
145                 150                 155

<210>  45
<211>  158
<212>  PRT
<213>  artifical

<400>  45

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Lys Thr Pro Leu His Leu Ala Ala Ala Ile Gly
        35                  40                  45

His Leu Glu Ile Val Glu Val Leu Leu Lys Asn Gly Ala Asp Val Asn
    50                  55                  60

Ala Thr Asp Tyr Gly Leu Thr Pro Leu His Leu Ala Ala Asp Asn Gly
65                  70                  75                  80

His Leu Glu Ile Val Glu Val Leu Leu Lys Asn Gly Ala Asp Val Asn
                85                  90                  95

Ala Phe Asp Phe Thr Gly Arg Thr Pro Leu His Leu Ala Ala Ser Gln
            100                 105                 110

Gly His Leu Glu Ile Val Glu Val Leu Leu Lys Asn Gly Ala Asp Val
        115                 120                 125

Asn Ala Gln Asp Lys Phe Gly Lys Thr Pro Phe Asp Leu Ala Ile Asp
    130                 135                 140

Asn Gly Asn Glu Asp Ile Ala Glu Val Leu Gln Lys Ala Ala
145                 150                 155


<210>  46
<211>  158
<212>  PRT
<213>  artifical

<400>  46

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Lys Thr Pro Leu His Leu Ala Ala Ala Ile Gly
        35                  40                  45

His Leu Glu Ile Val Glu Val Leu Leu Lys Asn Gly Ala Asp Val Asn
    50                  55                  60

Ala Thr Asp Tyr Gly Leu Thr Pro Leu His Leu Ala Ala Asp Asn Gly
65                  70                  75                  80

His Leu Glu Ile Val Glu Val Leu Leu Lys Asn Gly Ala Asp Val Asn
                85                  90                  95

Ala Phe Asp Phe Thr Gly Arg Thr Pro Leu His Leu Ala Ala Ser Gln
            100                 105                 110

Gly His Leu Glu Ile Val Glu Val Leu Leu Lys Asn Gly Ala Asp Val
        115                 120                 125

Asn Ala Gln Asp Lys Phe Gly Glu Thr Lys Glu Asp Leu Ala Lys Asp
    130                 135                 140

Asn Gly Asn Gln Asp Ile Ala Asp Leu Leu Glu Lys Ala Leu
145                 150                 155

<210>  47
<211>  158
<212>  PRT
<213>  artifical

<400>  47

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Phe Tyr Gly Lys Thr Pro Leu His Leu Ala Ala Ala Ile Gly
        35                  40                  45

His Leu Glu Ile Val Glu Val Leu Leu Lys Asn Gly Ala Asp Val Asn
    50                  55                  60

Ala Thr Asp Tyr Gly Leu Thr Pro Leu His Leu Ala Ala Asp Asn Gly
65                  70                  75                  80

His Leu Glu Ile Val Glu Val Leu Leu Lys Asn Gly Ala Asp Val Asn
                85                  90                  95

Ala Phe Asp Phe Thr Gly Arg Thr Pro Leu His Leu Ala Ala Ser Gln
            100                 105                 110

Gly His Leu Glu Ile Val Glu Val Leu Leu Lys Asn Gly Ala Asp Val

115                 120                 125

Asn Ala Gln Asp Lys Phe Gly Glu Thr Lys Met Asp Leu Ala Arg Asp
    130                 135                 140

Asn Gly Asn Glu Asp Ile Tyr Lys Leu Leu Ala Lys Ala Leu
145                 150                 155

<210>  48
<211>  160
<212>  PRT
<213>  artifical

<400>  48

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Trp His Gly Ile Thr Pro Leu His Leu Ala Ala Phe Tyr Gly
        35                  40                  45

His Leu Glu Ile Val Glu Val Leu Leu Lys Asn Gly Ala Asp Val Asn
    50                  55                  60

Ala Phe Asp Asp Tyr Asp Gly Ser Thr Pro Leu His Leu Ala Ala Trp
65                  70                  75                  80

Met Gly His Leu Glu Ile Val Glu Val Leu Leu Lys His Gly Ala Asp
                85                  90                  95

Val Asn Ala Thr Asp His Phe Gly Asn Thr Pro Leu His Leu Ala Ala
            100                 105                 110

Ala Met Gly His Leu Glu Ile Val Glu Val Leu Leu Lys His Gly Ala
        115                 120                 125

Asp Val Asn Ala Gln Asp Lys Phe Gly Lys Thr Pro Phe Asp Leu Ala
    130                 135                 140

Ile Asp Asn Gly Asn Glu Asp Ile Ala Glu Val Leu Gln Lys Ala Ala
145                 150                 155                 160

<210>  49
<211>  160
<212>  PRT
<213>  artifical

<400>  49

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Trp His Gly Ile Thr Pro Leu His Leu Ala Ala Phe Tyr Gly
        35                  40                  45

His Leu Glu Ile Val Glu Val Leu Leu Lys Asn Gly Ala Asp Val Asn
    50                  55                  60

Ala Phe Asp Asp Tyr Asp Gly Ser Thr Pro Leu His Leu Ala Ala Trp
65                  70                  75                  80

Met Gly His Leu Glu Ile Val Glu Val Leu Leu Lys His Gly Ala Asp
                85                  90                  95

Val Asn Ala Thr Asp His Phe Gly Asn Thr Pro Leu His Leu Ala Ala
            100                 105                 110

Ala Met Gly His Leu Glu Ile Val Glu Val Leu Leu Lys His Gly Ala
        115                 120                 125

Asp Val Asn Ala Gln Asp Lys Phe Gly Lys Thr Ala Glu Asp Leu Ala
    130                 135                 140

Lys Asp Asn Gly Asn Gln Asp Ile Ala Asp Leu Leu Glu Lys Ala Leu
145                 150                 155                 160

<210>  50
<211>  160
<212>  PRT
<213>  artifical

<400>  50

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

His Asp Trp His Gly Ile Thr Pro Leu His Leu Ala Ala Phe Tyr Gly
        35                  40                  45

His Leu Glu Ile Val Glu Val Leu Leu Lys Asn Gly Ala Asp Val Asn
    50                  55                  60

Ala Phe Asp Asp Tyr Asp Gly Ser Thr Pro Leu His Leu Ala Ala Trp
65                  70                  75                  80

Met Gly His Leu Glu Ile Val Glu Val Leu Leu Lys His Gly Ala Asp
                85                  90                  95

Val Asn Ala Thr Asp His Phe Gly Asn Thr Pro Leu His Leu Ala Ala
            100                 105                 110

Ala Met Gly His Leu Glu Ile Val Glu Val Leu Leu Lys His Gly Ala
        115                 120                 125

Asp Val Asn Ala Gln Asp Lys Phe Gly Lys Thr Ala Met Asp Leu Ala
    130                 135                 140

Arg Asp Asn Gly Asn Glu Asp Ile Tyr Lys Leu Leu Ala Lys Ala Leu
145                 150                 155                 160

<210>  51
<211>  5
<212>  PRT
<213>  artifical

<400>  51

Gly Gly Gly Gly Ser
1               5

<210>  52
<211>  10
<212>  PRT
<213>  artifical

<400>  52

Gly Gly Gly Gly Ser Gly Gly Gly Gly Ser
1               5                   10

<210>  53
<211>  15
<212>  PRT
<213>  artifical

<400>  53

Gly Gly Gly Gly Ser Gly Gly Gly Gly Ser Gly Gly Gly Gly Ser
1               5                   10                  15

<210>  54
<211>  20
<212>  PRT
<213>  artifical

<400>  54

Gly Gly Gly Gly Ser Gly Gly Gly Gly Ser Gly Gly Gly Gly Ser Gly
1               5                   10                  15

Gly Gly Gly Ser
            20

<210>  55
<211>  77
<212>  PRT
<213>  Homo sapiens

<400>  55

Glu Asp Asn Tyr Ala Leu Ala Val Leu Asp Asn Gly Asp Pro Leu Asn
1               5                   10                  15

Asn Thr Thr Pro Val Thr Gly Ala Ser Pro Gly Gly Leu Arg Glu Leu
            20                  25                  30

Gln Leu Arg Ser Leu Thr Glu Ile Leu Lys Gly Gly Val Leu Ile Gln
        35                  40                  45

Arg Asn Pro Gln Leu Cys Tyr Gln Asp Thr Ile Leu Trp Lys Asp Ile
    50                  55                  60

Phe His Lys Asn Asn Gln Leu Ala Leu Thr Leu Ile Asp
65                  70                  75

<210>  56
<211>  27
<212>  PRT
<213>  Homo sapiens

<400>  56

Phe Leu Arg Gly Gln Glu Cys Val Glu Glu Cys Arg Val Leu Gln Gly
1               5                   10                  15

Leu Pro Arg Glu Tyr Val Asn Ala Arg His Cys
            20                  25

<210>  57
<211>  65
<212>  PRT
<213>  Homo sapiens

<400>  57

Leu Pro Cys His Pro Glu Cys Gln Pro Gln Asn Gly Ser Val Thr Cys
1               5                   10                  15

Phe Gly Pro Glu Ala Asp Gln Cys Val Ala Cys Ala His Tyr Lys Asp
            20                  25                  30

Pro Pro Phe Cys Val Ala Arg Cys Pro Ser Gly Val Lys Pro Asp Leu
        35                  40                  45

Ser Tyr Met Pro Ile Trp Lys Phe Pro Asp Glu Glu Gly Ala Cys Gln
    50                  55                  60

Pro
65